# EUROPEAN PATENT SPECIFICATION

(11) **EP 1 056 725 B1**
(45) Date of publication and mention of the grant of the patent: **07.06.2006**
(21) Application number: 98963810.1
(22) Date of filing: 22.12.1998
(51) Int. Cl.: C07D 231/38, C07D 403/12, C07D 409/12, C07D 333/36, C07D 307/66, C07D 231/40, C07D 401/12, C07D 405/04, A61K 31/415

(54) **INHIBITION OF RAF KINASE USING ARYL AND HETEROARYL SUBSTITUTED HETEROCYCLIC UREAS**
HEMMUNG VON RAF-KINASE UNTER VERWENDUNG VON ARYL- UND HETEROARYLSUBSTITUIERTEN HETEROCYCLISCHEN HARNSTOFFEN
INHIBITION DE RAF KINASE A L'AIDE D'UREES HETEROCYCLIQUES ARYLE ET HETEROARYLE SUBSTITUEES

(30) Priority: 22.12.1997 US 996181
(43) Date of publication of application: 06.12.2000
(73) Proprietor: Bayer Pharmaceuticals Corp., West Haven, CT 06516 (US)
(72) Inventor: DUMAS, Jacques, Orange, CT 06477 (US); KHIRE, Uday, Hamden, CT 06518 (US); LOWINGER, Timothy, Bruno, Hyogo 662-0046 (JP); RIEDL, Bernd, 42329 Wuppertal (DE); SCOTT, William, J., Guilford, CT 06437 (US); SMITH, Roger, A., Madison, CT 06443 (US); WOOD, Jill, E., Hamden, CT 06517 (US); HATOUM-MOKDAD, Holia, Hamden, CT 06514 (US); JOHNSON, Jeffrey, Branford, CT 06405 (US); REDMAN, Aniko, Derby, CT 06418 (US); SIBLEY, Robert, North Haven, CT 06473 (US)
(74) Representative: Weiss, Wolfgang
(86) International application number: PCT/US1998/026082
(87) International publication number: WO 1999/032455

(56) References cited:
- WO-A-96/40673
- WO-A-98/22103
- WO-A-98/52559
- WO-A-99/23091
- WO-A-99/32110
- US-A- 5 162 360

## Description

### Field of the Invention

This invention relates to the use of a group of aryl ureas in treating raf mediated diseases, and pharmaceutical compositions for use in such therapy.

### Background of the Invention

The p21^{ras} oncogene is a major contributor to the development and progression of human solid cancers and is mutated in 30% of all human cancers (Bolton et al. *Ann. Rep. Med. Chem.* **1994,** *29,* 165-74; Bos. *Cancer Res.* **1989,** *49,* 4682-9). In its normal, unmutated form, the ras protein is a key element of the signal transduction cascade directed by growth factor receptors in almost all tissues (Avruch et al. *Trends Biochem. Sci.* **1994,** *19*, 279-83). Biochemically, ras is a guanine nucleotide binding protein, and cycling between a GTP-bound activated and a GDP-bound resting form is strictly controlled by ras' endogenous GTPase activity and other regulatory proteins. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. *Semin. Cancer Biol.* **1994,** *5,* 247-53). It has been shown that inhibiting the effect of active ras by inhibiting the raf kinase signaling pathway by administration of deactivating antibodies to raf kinase or by co-expression of dominant negative raf kinase or dominant negative MEK, the substrate of raf kinase, leads to the reversion of transformed cells to the normal growth phenotype (see: Daum et al. *Trends Biochem. Sci.* **1994,** *19*, 474-80; Fridman et al. *J. Biol. Chem.* **1994**, *269,* 30105-8. Kolch et al. (*Nature* **1991,** *349*, 426-28) have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human tumor types (Monia et al., *Nat. Med.* **1996**, *2*, 668-75).

US 5,162,360 discloses N-substituted aryl-N prime-heterocyclic substituted ureas and thioureas of a structure related to those disclosed herein. However, the compounds of the prior art are described only as inhibitors of the enzyme acyl-coenzyme A: cholesterol acyltransferase (ACAT) and thus are intended only for treating hypercholesterolemia and atherosclerosis.

WO 96/40673 discloses urea and thiourea-type compounds of a formula related to the compounds disclosed herein, but this state of the art is concerned only with compounds which function as tyrosine kinase inhibitors and not with raf kinase inhibitors.

### Summary of the Invention

The present invention provides compounds which are inhibitors of the enzyme raf kinase. Since the enzyme is a downstream effector of p21^{ras}, the instant inhibitors are useful in pharmaceutical compositions for human or veterinary use where inhibition of the raf kinase pathway is indicated, e.g., in the treatment of tumors and/or cancerous cell growth mediated by raf kinase. In particular, the compounds are useful in the treatment of human or animal, e.g., murine cancer, since the progression of these cancers is dependent upon the ras protein signal transduction cascade and therefore susceptible to treatment by interruption of the cascade, i.e., by inhibiting raf kinase. Accordingly, the compounds of the invention are useful in treating solid cancers, such as, for example, carcinomas (e.g., of the lungs, pancreas, thyroid, bladder or colon, myeloid disorders (e.g., myeloid leukemia) or adenomas (e.g., villous colon adenoma).

The present invention, therefore, provides compounds generally described as aryl ureas, including both aryl and heteroaryl analogues, which inhibit the raf pathway. The invention also provides a method for treating a raf mediated disease state in humans or mammals. Thus, the invention is directed to compounds and methods for the treatment of cancerous cell growth mediated by raf kinase comprising administering a compound of formula I 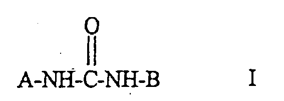 wherein B is generally an unsubstituted or substituted, up to tricyclic, aryl or heteroaryl moiety with up to 12 carbon atoms with at least one 5 or 6 member aromatic structure containing 0-4 members of the group consisting of nitrogen, oxygen and sulfur. A is a heteroaryl moiety discussed in more detail below.

The aryl and heteroaryl moiety of B may contain separate cyclic structures and can include a combination of aryl, heteroaryl and cycloalkyl structures. The substituents for these aryl and heteroaryl moieties can vary widely and include halogen, hydrogen, hydrosulfide, cyano, nitro, amines and various carbon-based moieties, including those which contain one or more of sulfur, nitrogen, oxygen and/or halogen and are discussed more particularly below.

Suitable aryl and heteroaryl moieties for B of formula I include, but are not limited to aromatic ring structures containing 4-12 carbon atoms and 1-3 rings, at least one of which is a 5-6 member aromatic ring. One or more of these rings may have 1-4 carbon atoms replaced by oxygen, nitrogen and/or sulfur atoms.

Examples of suitable aromatic ring structures include phenyl, pyridinyl, naphthyl, pyrimidinyl, benzothiozolyl, quinoline, isoquinoline, phthalimidinyl and combinations thereof, such as diphenyl ether (phenyloxyphenyl), diphenyl thioether (phenylthiophenyl), diphenyl amine (phenylaminophenyl), phenylpyridinyl ether (pyridinyloxyphenyl), pyridinylmethylphenyl, phenylpyyridinyl thioether (pyridinylthiophenyl), phenylbenzothiazolyl ether (benzothiazolyloxyphenyl), phenylbenzothiazolyl thioether (benzothiazolylthiophenyl), phenylpyrimidinyl ether, phenylquinoline thioether, phenylnaphthyl ether, pyridinylnapthyl ether, pyridinylnaphthyl thioether, and phthalimidyhnethylphenyl.

Examples of suitable heteroaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms.

For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or-5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-oxadiazol-3- or -5-yl, 1,3,4-thiadiazol-2- or -5-yl, 1,3,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofitryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc., throughout include methyl, ethyl, propyl, butyl, etc., including all straight-chain and branched isomers such as isopropyl, isobutyl, *sec*-butyl, *tert*-butyl, etc.

Suitable aryl groups include, for example, phenyl and 1- and 2-naphthyl.

Suitable cycloalkyl groups include cyclopropyl, cyclobutyl, cyclohexyl, etc. The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl" also includes saturated heterocycles.

Suitable halogens include F, Cl, Br, and/or I, from one to persubstitution (i.e., all H atoms on the group are replaced by halogen atom), being possible, mixed substitution of halogen atom types also being possible on a given moiety.

As indicated above, these ring systems can be unsubstituted or substituted by substituents such as halogen up to per-halosubstitution. Other suitable substituents for the moieties of B include alkyl, alkoxy, carboxy, cycloalkyl, aryl, heteroaryl, cyano, hydroxy and amine. These other substituents, generally referred to as X and X' herein, include -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂₋₁₀-alkenyl, substituted C₂₋₁₀-alkoxy, substituted C₃-C₁₀ cycloalkyl, substituted C₄-C₂₃ alkheteroaryl and -Y-Ar.

Where a substituent, X or X', is a substituted group, it is preferably substituted by one or more substituents independently selected from the group consisting of-CN, -CO₂R⁵, -C(O)R⁵, -C(O)NR⁵R^{5'}, -OR⁵, -SR⁵, -NR⁵R^{5'}, -NO_{2,} -NR⁵C(O)R^{5'}, -NR⁵C(O)OR^{5'} and halogen up to per-halo substitution.

The moieties R⁵ and R^{5'} are preferably independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀₋alkenyl , up to per-halosubstituted C₃-C₁₀ cycloalkyl, up to per-halosubstituted C₆-C₁₄ aryl and up to per-halosubstituted C₃-C₁₃ heteroaryl.

The bridging group Y is preferably -O-, -S-, -N(R⁵)-, -(CH₂)-ₘ, -C(O)-, -CH(OH)-, -NR⁵C(O)NR⁵R^{5'}-, -NR⁵C(O)-, -C(O)NR⁵-, -(CH₂)ₘO-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-, where m = 1-3, and X^{a} is halogen.

The moiety Ar is preferably a 5-10 member aromatic structure containing 0-2 members of the group consisting of nitrogen, oxygen and sulfur which is unsubstituted or substituted by halogen up to per-halosubstitution and optionally substituted by Zₙ₁, wherein n1 is 0 to 3.

Each Z substituent is preferably independently selected from the group consisting of -CN, -CO₂R⁵, -C(O)NR⁵R^{5'}, -C(O)- NR⁵, -NO₂, -OR⁵,- SR⁵,- NR⁵R^{5'}, -NR⁵C(O)OR^{5'}, -C(O)R⁵, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl. If Z is a substituted group, it is substituted by one or more substituents independently selected from the group consisting of -CN, -CO₂R⁵, -C(O)NR⁵R^{5'}, -OR⁵, -SR⁵, -NO₂, -NR⁵R^{5'}, -NR⁵C(O)R^{5'} and -NR⁵C(O)OR^{5'}.

The aryl and heteroaryl moieties of B of Formula I are preferably selected from the group consisting of 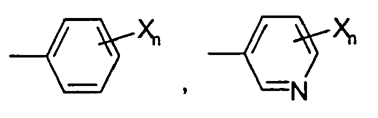 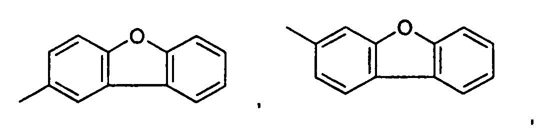 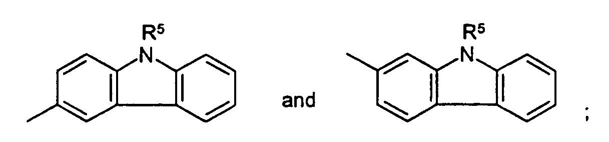 which are unsubstituted or substituted by halogen, up to per-halosubstitution. X is as defined above and n = 0-3.

The aryl and heteroaryl moieties of B are more preferably of the formula: 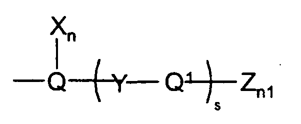 wherein Y is selected from the group consisting of-O-, -S-, -CH₂-, -SCH₂-, -CH₂S-, -CH(OH)-, -C(O)-, -CX^{a}₂, -CX^{a}H-, -CH₂O- and -OCH₂- and X^{a} is halogen.

Q is a six member aromatic structure containing 0-2 nitrogen, substituted or unsubstituted by halogen, up to per-halosubstitution and Q¹ is a mono- or bicyclic aromatic structure of 3 to 10 carbon atoms and 0-4 members of the group consisting of N, O and S, unsubstituted or unsubstituted by halogen up to per-halosubstitution. X, Z, n and n1 are as defined above, and s = 0 or 1.

In preferred embodiments, Q is phenyl or pyridinyl, substituted or unsubstituted by halogen, up to per-halosubstitution and Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, or Y-Q¹ is phthalimidinyl substituted or unsubstituted by halogen up to per-halo substitution. Z and X are preferably independently selected from the group consisting of -R⁶, -OR⁶ and -NHR⁷, wherein R⁶ is hydrogen, C₁-C₁₀-alkyl or C₃-C₁₀-cycloalkyl and R⁷ is preferably selected from the group consisting of hydrogen, C₃-C₁₀-alkyl, C₃₋C₆-cycloalkyl and C₆-C₁₀-aryl, wherein R⁶ and R⁷ can be substituted by halogen or up to per-halosubstitution.

The heteroaryl moiety A of formula I is preferably selected from the group consisting of 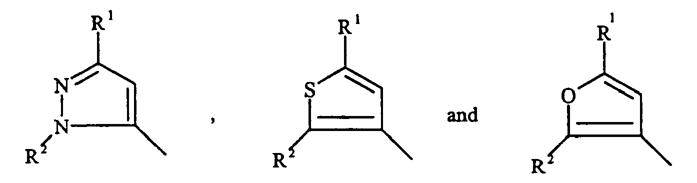 wherein R¹ is preferably selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloallcyl and R² is C₆-C₁₄ aryl, C₃-C₁₄ heteroaryl, substituted C₆-C₁₄ aryl or substituted C₃-C₁₄ heteroaryl.

Where R² is a substituted group, the substituents are preferably independently selected from the group consisting of halogen, up to per-halosubstitution, and Vₙ, where n = 0-3.

Each V is preferably independently selected from the group consisting of -CN, -OC(O)NR⁵R^{5'}, -CO₂R⁵, -C(O)NR⁵R^{5'}, -OR⁵, -SR⁵, -NR⁵R^{5'}, -C(O)R⁵, - NR⁵C(O)OR^{5'}, -SO₂R⁵, -SOR⁵, -NR⁵C(O)R^{5'}, -NO₂, C₁-C₁₀ alkyl, C₃-C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl.

If V is a substituted group, it is preferably substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} and -NO₂.

The substituents R⁵ and R^{5'} are preferably each independently selected form the group consisting of H, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl.

R² is preferably substituted or unsubstituted phenyl or pyridinyl, where the substituents for R² are selected from the group consisting of halogen, up to per-halosubstituition and Vₙ¹, wherein n = 0-3. Each V¹ is preferably independently selected from the group consisting of substituted and unsubstituted C₁-C₆ alkyl, C₃-C₁₀ cycloalkyl, C₆-C₁₀ aryl, -NO₂, -NH₂, -C(O)-C₁₋₆ alkyl, -C(O)N-(C₁₋₆ alkyl)₂, -C(O)NH-C₁₋₆ alkyl, -O-C₁₋₆ alkyl, -NHC(O)H, -NHC(O)OH, -N(C₁₋₆ alkyl)C(O)-C₁-₆ alkyl, -N-(C₁-₆ alkyl)C(O)-C₁-₆ alkyl, -NHC(O)-C₁-₆ alkyl, -OC(O)NH-C₆₋₁₄ aryl, -NHC(O)O-C₁-₆ alkyl, -S(O)-C₁-₆ alkyl and -SO₂-C₁-₆ alkyl. Where V' is a substituted group, it is preferably substituted by one or more halogen, up to per-halosubstitution.

Most preferably, R² is selected from substituted and unsubstituted phenyl or pyridinyl groups, where the substituents are halogen and Wₙ (n = 0-3).

W is preferably selected from the group consisting of -NO₂, -C₁-₃ alkyl, -NH(O)CH₃, -CF₃, -OCH₃, -F, -Cl, -NH₂, -OC(O)NH up to per-halosubstituted phenyl, -SO₂CH₃, pyridinyl, phenyl, up to per-halosubstituted phenyl and C₁-C₆ alkyl substituted phenyl.

The invention also relates the compounds within the scope of general formula I described above. These more particularly include pyrazolyl ureas of the formula 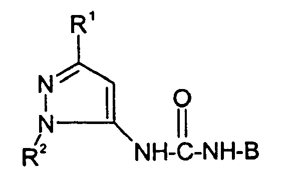 furyl ureas of the formula 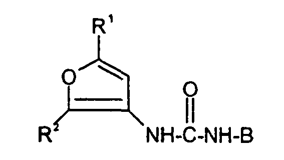 and thienyl ureas of the formula 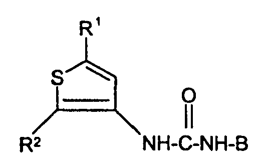 wherein R¹, R² and B are as defined above.

The present invention is also directed to pharmaceutically acceptable salts of formula I. Suitable-pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺², Ca⁺² or Ba⁺²), the ammonium cation, as well as acid salts of organic bases, including aliphatic and aromatic substituted ammonium, and quaternary ammonium cations such as those arising from protonation or peralkylation of triethylamine, *N,N*-diethylamine, *N,N*-dicyclohexylamine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art.

The present invention encompasses any isolated racemic or optically active form of compounds described in Formula I which possess Raf kinase inhibitors.

The compounds of Formula I may be prepared by use of known chemical reactions and procedures, some of which are commercially available. Nevertheless, the following general preparative methods are presented to aid one of skill in the art in synthesizing these compounds, with more detailed examples being presented in the experimental section describing the working examples.

### General Preparative Methods

Heterocyclic amines may be synthesized utilizing known methodology (Katritzky, et al. *Comprehensive Heterocyclic Chemistry;* Permagon Press: Oxford, UK (1984). March. *Advanced Organic Chemistry,* 3^{rd} Ed.; John Wiley: New York (1985)). For example, as shown in Scheme I, 5-aminopyrazoles substituted at the *N*-1 position with either aryl or heteroaryl moieties may be synthesized by the reaction of an α-cyanoketone **(2)** with the appropriate aryl- or heteroaryl hydrazine (**3**, R²=aryl or heteroaryl). Cyanoketone **2,** in turn, is available from the reaction of acetamidate ion with an appropriate acyl derivative, such as an ester, an acid halide, or an acid anhydride. In cases where the R² moiety offers suitable anion stabilization, 2-aryl- and 2-heteroarylfurans may be synthesized from a Mitsunobu reaction of cyanoketone **2** with alcohol **5,** followed by base catalyzed cyclization of enol ether **6** to give furylamine **7.**

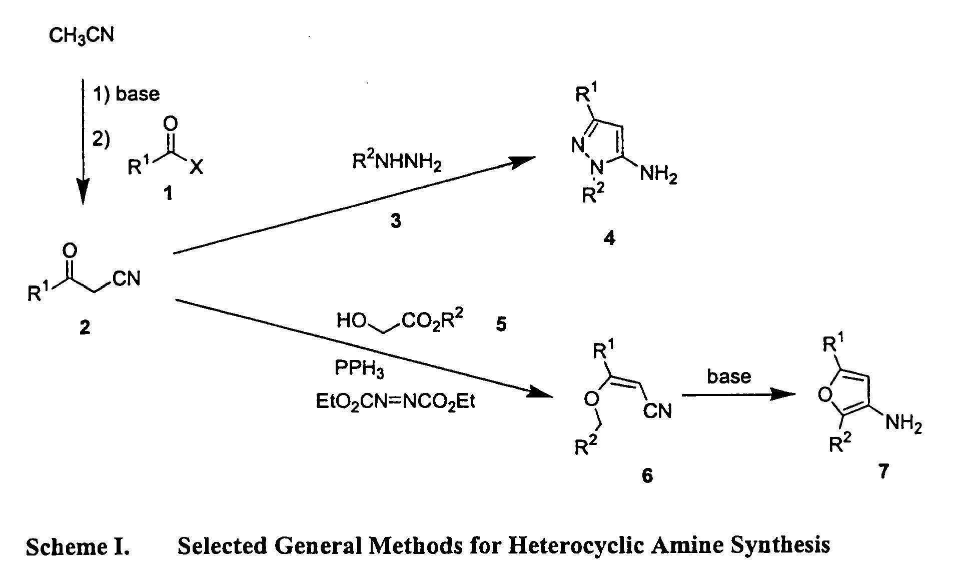

Substituted anilines may be generated using standard methods (March. *Advanced Organic Chemistry,* 3^{rd}Ed.; John Wiley: New York (1985). Larock. *Comprehensive Organic Transformations;* VCH Publishers: New York (1989)). As shown in Scheme II, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. *Hydrogenation Methods;* Academic Press: London, UK (1985)). Nitroaryls may also be directly reduced using a strong hydride source, such as LiAlH₄ (Seyden-Penne. *Reductions by the Alumino- and Borohydrides in Organic Synthesis;* VCH Publishers: New York (1991)), or using a zero valent metal, such as Fe, Sn or Ca, often in acidic media. Many methods exist for the synthesis of nitroaryls (March. *Advanced Organic Chemistry,* 3^{rd} Ed.; John Wiley: New York (1985). Larock. *Comprehensive Organic Transformations;* VCH Publishers: New York (1989)).

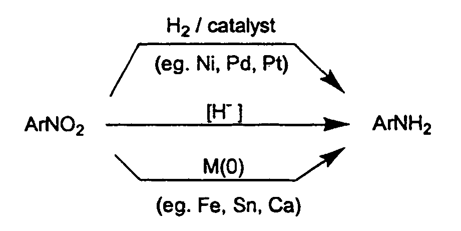

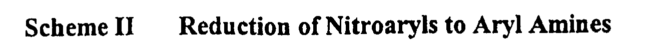

Nitroaryls are commonly formed by electrophilic aromatic nitration using HNO₃, or an alternative NO₂⁺ source. Nitro aryls may be further elaborated prior to reduction. Thus, nitroaryls substituted with 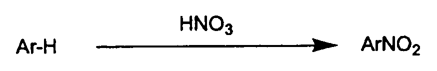 potential leaving groups (eg. F, Cl, Br, etc.) may undergo substitution reactions on treatment with nucleophiles, such as thiolate (exemplified in Scheme III) or phenoxide. Nitroaryls may also undergo Ullman-type coupling reactions (Scheme III).

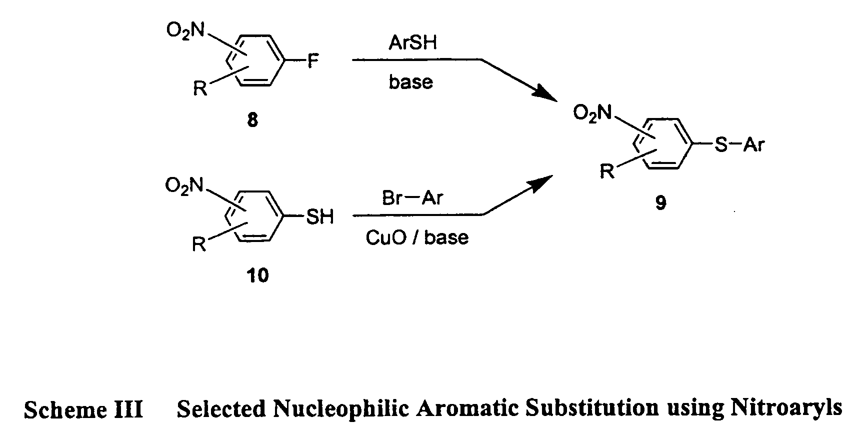

As shown in Scheme IV, urea formation may involve reaction of a heteroaryl isocyanate **(12)** with an aryl amine **(11).** The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N'*-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **16** with an azide source, followed by rearrangement affords the isocyanate. The corresponding carboxylic acid **(17)** may also be subjected to Curtius-type rearrangements using diphenylphosphoryl azide (DPPA) or a similar reagent. A urea may also be generated from the reaction of an aryl isocyanate **(15)** with a heterocyclic amine.

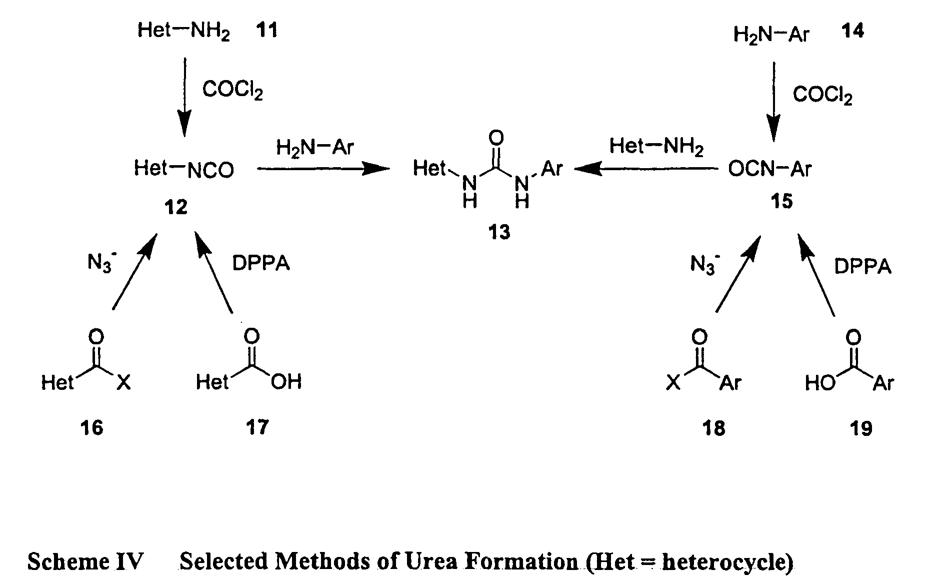

Finally, ureas may be further manipulated using methods familiar to those skilled in the art. For example, 2-aryl and 2-heteroarylthienyl ureas are available from the corresponding 2-halothienyl urea through transition metal mediated cross coupling reactions (exemplified with 2-bromothiophene **25,** Scheme V). Thus, reaction of nitrile **20** with an α-thioacetate ester gives 5-substituted-3-amino-2-thiophenecarboxylate **21** (Ishizaki et al. JP 6025221). Decarboxylation of ester **21** may be achieved by protection of the amine, for example as the tert-butoxy (BOC) carbamate **(22),** followed by saponification and treatment with acid. When BOC protection is used, decarboxylation may be accompanied by deprotection giving the substituted 3-thiopheneammonium salt **23.** Alternatively, ammonium salt **23** may be directly generated through saponification of ester **21** followed by treatment with acid. Following urea formation as described above, bromination affords penultimate halothiophene **25.** Palladium mediated cross coupling of thiophene **25** with an appropriate tributyl- or trimethyltin (R²= aryl or heteroaryl) then affords the desired 2-aryl- or 2-heteroarylthienyl urea.

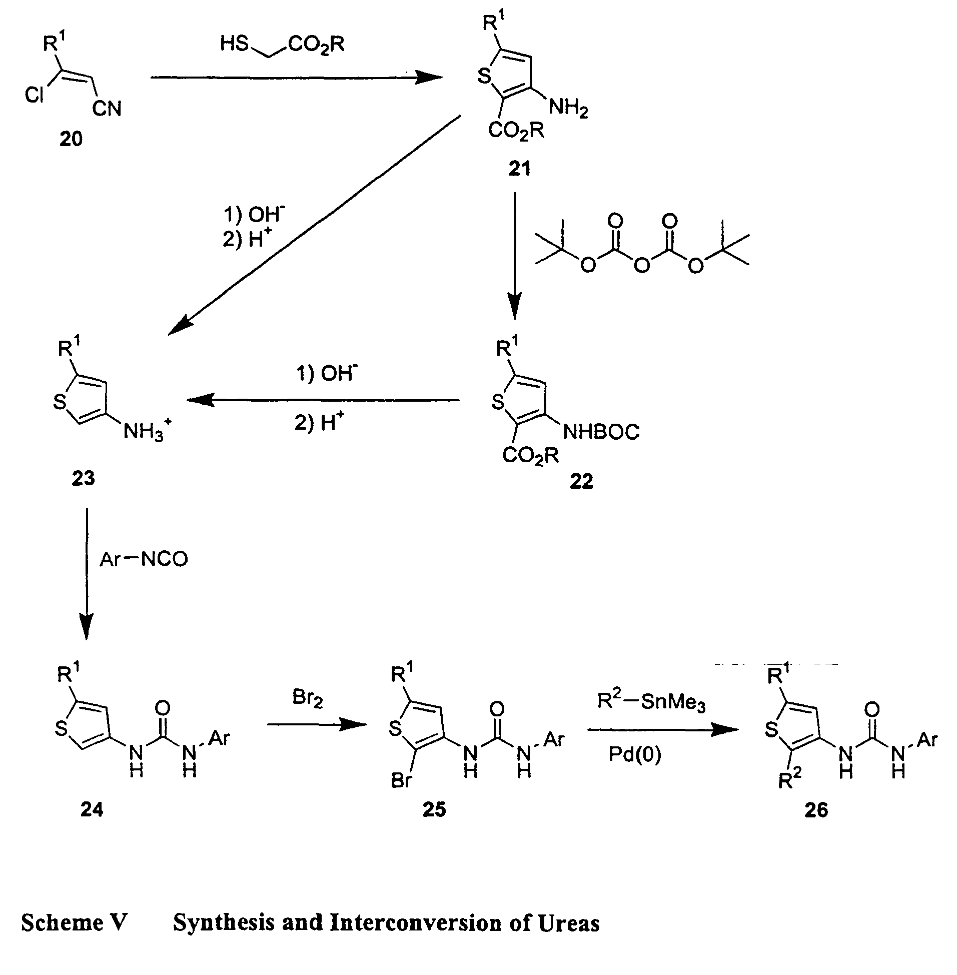

The invention also includes pharmaceutical compositions including a compound of Formula I, and a physiologically acceptable carrier.

The compounds may be administered orally, topically, parenterally, by inhalation or spray or vaginally, sublingually, or rectally in dosage unit formulations. The term 'administration by injection' includes intravenous, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally occurring phosphatide, for example, lecithin, or condensation products or an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; " Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl isobutyl tertbutyl or monoglycerin esters of acetic acid, capronic acid, lauric acid, myristinic-acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components.

Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily vaginal dosage regimen will preferably be from 0.01 to 200 mg/Kg of total body weight. The daily rectal dosage regime will preferably be from 0.01 to 200 mg/Kg of total body weight. The daily topical dosage regime will preferably be from 0.1 to 200 mg administered between one to four times daily. The transdermal concentration will preferably be that required to maintain a daily dose of from 0.01 to 200 mg/Kg. The daily inhalation dosage regime will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy.

It will be further appreciated by one skilled in the art that the optimal course of treatment, ie., the mode of treatment and the daily number of doses of a compound of Formula I or a pharmaceutically acceptable salt thereof given for a defined number of days, can be ascertained by those skilled in the art using conventional treatment tests.

It will be understood, however, that the specific dose level for any particular patient will depend upon a variety of factors, including the activity of the specific compound employed, the age, body weight, general health, sex, diet, time of administration, route of administration, and rate of excretion, drug combination and the severity of the condition undergoing therapy.

The entire disclosure of all applications, patents and publications cited above and below are hereby incorporated by reference, including provisional application [Attorney Docket Bayer 9V1], filed on December 22, 1997 as SN 08/996,181 and converted on December 22, 1998.

The compounds are producible from known compounds (or from starting materials which, in turn, are producible from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construde to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg.

All temperatures are reported uncorrected in degrees Celsius (°C). Unless otherwise indicated, all parts and percentages are by weight.

Commercial grade reagents and solvents were used without further purification. Thin-layer chromatography (TLC) was performed on Whatman® pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science® silica gel.

Melting points (mp) were determined using a Thomas-Hoover melting point apparatus or a Mettler FP66 automated melting point apparatus and are uncorrected. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3.30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an BP-1 methyl silicone column (0.33 mM coating; 25 m x 0.2 mm) and a Hewlett Packard 5971 Mass Selective Detector (ionization energy 70 eV).

Elemental analyses were conducted by Robertson Microlit Labs, Madison NJ. All compounds displayed NMR spectra, LRMS and either elemental analysis or HRMS consistant with assigned structures.

### List of Abbreviations and Acronyms:

- AcOH: acetic acid
- anh: anhydrous
- BOC: *tert*-butoxycarbonyl
- conc: concentrated
- dec: decomposition
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EtOAc: ethyl acetate
- EtOH: ethanol (100%)
- Et₂O: diethyl ether
- Et₃N: triethylamine
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Heterocyclic Amines

### A1 General Procedure for the Preparation of N¹-Aryl-5-aminopyrazoles

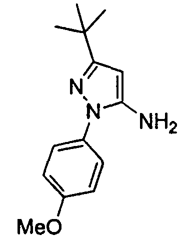

***N***^{***1***}**-(4-Methoxyphenyl)-5-amino-3-*tert*-butylpyrazole:** A mixture of 4-methoxyphenylhydrazine hydrochloride (3.5 g), 4,4-dimethyl-3-oxopentanenitrile (2.5 g), EtOH (30 mL), and AcOH (1 mL) was heated at the reflux temperature for 3 h, cooled to room temp., and poured into a mixture of Et₂O (100 mL) and a 10% Na₂CO₃ solution (100 mL). The organic layer was washed with a saturated NaCl solution, dried (MgSO₄) and concentrated under reduced pressure. The solid residue was washed with pentane to afford the desired pyrazole as a pale brown solid. (4.25g): ¹H-NMR (DMSO-d₆) δ 1.18 (s, 9H); 3.78 (s, 3H); 5.02 (br s, 2H); 5.34 (s, 1H); 6.99 (d, *J*=8 Hz, 2H); 7.42 (d, *J*=8 Hz, 2H).

### General Method for the Mitsunobu-Based Synthesis of 2-Aryl-3-aminofurans

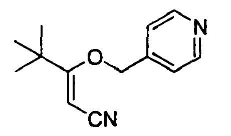

**Step 1. 4,4-Dimethyl-3-(4-pyridinylmethoxy)-2-pentenenitrile:** A solution of triphenylphosphine (2.93 g, 11.2 mmol) in anh THF (50 mL) was treated with diethyl azodicarboxylate (1.95 g, 11.2 mmol) and 4-pyridinylmethanol (1.22 g, 11.2 mmol), then stirred for 15 min. The resulting white slurry was treated with 4,4-dimethyl-3-oxopentanenitrile (1.00 g, 7.99 mmol), then stirred for 15 min. The reaction mixture was concentrated under reduced pressure. The residue was purified by column chromatography (30% EtOAc/70% hexane) to give the desired nitrile as a yellow solid (1.83 g, 76%): TLC (20% EtOAc/80% hexane) R_{*f*} 0.13; ¹H-NMR (CDCl₃) δ 1.13 (s, 9H), 4.60 (s, 1H), 5.51 (s, 2H), 7.27 (d, *J*=5.88 Hz, 2H), 8.60 (d, *J*=6.25 Hz, 2H); ¹³C-NMR (CDCl₃) δ 27.9 (3C), 38.2, 67.5, 70.8, 117.6, 121.2 (2C), 144.5, 149.9 (2C), 180.7; CI-MS *m*/*z* (rel abundance) 217 ((M+H)⁺, 100%).

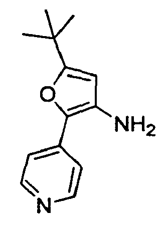

**Step 2. 3-Amino-2-(4-pyridinyl)-5-*tert*-butylfuran:** A solution of 4,4-dimethyl-3-(4-pyridinylmethoxy)-2-pentenenitrile (1.55 g, 7.14 mmol) in anh DMSO (75 mL) was treated with potassium *tert*-butoxide (0.88 g, 7.86 mmol) and stirred at room temp for 10 min. The resulting mixture was treated with EtOAc (300 mL), then sequentially washed with water (2 x 200 mL) and a saturated NaCl solution (100 mL). Combined aqueous phases were back-extracted with EtOAc (100 mL). The combined organic phases were dried (Na₂SO₄) and concentrated under reduced pressure. The residue was purified by column chromatography (gradient from 30% EtOAc/70% hexane to 100% EtOAc) to give the desired product as an orange oil (0.88 g, 57%): TLC (40% EtOAc/60% hexane) R_{*f*} 0.09; ¹H-NMR (CDCl₃) δ 1.28 (s, 9H), 3.65 (br s, 2H), 5.79 (s, 1H), 7.30 (d, *J*=6.25 Hz, 2H), 8.47 (d, *J*=6.25 Hz, 2H); EI-MS *m*/*z* (rel abundance) 216 (M⁺, 30%).

### Synthesis 3-Amino-5-alkylthiophenes from N-BOC 3-Amino-5-alkyl-2-thiophenecarboxylate esters

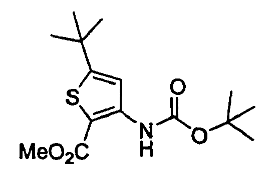

**Step 1. Methyl 3-(*tert*-Butoxycarbonylamino)-5-*tert*-butyl-2-thiophenecarboxylate:** To a solution of methyl 3-amino-5-*tert*-butyl-2-thiophenecarboxylate (150 g, 0.70 mol) in pyridine (2.8 L) at 5 °C was added di-*tert*-butyl dicarbonate (171.08 g, 0.78 mol, 1.1 equiv) and *N,N-*dimethylaminopyridine (86 g, 0.70 mol, 1.00 equiv) and the resulting mixture was stirred at room temp for 7 d. The resulting dark solution was concentrated under reduced pressure (approximately 0.4 mmHg) at approximately 20 °C. The resulting red solids were dissolved in CH₂Cl₂ (3 L) and sequentially washed with a 1 M H₃PO₄ solution (2 x 750 mL), a saturated NaHCO₃ solution (800 mL) and a saturated NaCl solution (2 x 800 mL), dried (Na₂SO₄) and concentrated under reduced pressure. The resulting orange solids were dissolved in abs. EtOH (2 L) by warming to 49 °C, then treated with water (500 mL) to afford the desired product as an off-white solid (163 g, 74%): ¹H-NMR (CDCl₃) δ 1.38 (s, 9H), 1.51 (s, 9H), 3.84 (s, 3H), 7.68 (s, 1H), 9.35 (br s, 1H); FAB-MS *m*/*z* (rel abundance) 314 ((M+H)⁺, 45%).

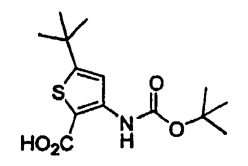

**Step 2. 3-(*tert*-Butoxycarbonylamino)-5-*tert*-butyl-2-thiophenecarboxylic Acid:** To a solution of methyl 3-(*tert*-butoxycarbonylamino)-5-*tert*-butyl-2-thiophenecarboxylate (90;0 g, 0.287 mol) in THF (630 mL) and MeOH (630 mL) was added a solution of NaOH (42.5 g, 1.06 mL) in water (630 mL). The resulting mixture was heated at 60 °C for 2 h, concentrated to approximately 700 mL under reduced pressure, and cooled to 0 °C. The pH was adjusted to approximately 7 with a 1.0 N HCl solution (approximately 1 L) while maintaining the internal temperature at approximately 0 °C. The resulting mixture was treated with EtOAc (4 L). The pH was adjusted to approximately 2 with a 1.0 N HCl solution (500 mL). The organic phase was washed with a saturated NaCI solution (4 x 1.5 L), dried (Na₂SO₄), and concentrated to approximately 200 mL under reduced pressure. The residue was treated with hexane (1 L) to form a light pink solid (41.6 g). Resubmission of the mother liquor to the concentration-precipitation protocol afforded additional product (38.4 g, 93% total yield): ¹H-NMR (CDCl₃) δ 1.94 (s, 9H), 1.54 (s, 9H), 7.73 (s, 1H), 9.19 (br s, 1H); FAB-MS *m*/*z* (rel abundance) 300 ((M+H)⁺, 50%).

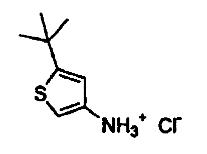

**Step 3. 5-*tert-*Butyl-3-thiopheneammonium Chloride:** A solution of 3-(*tert-*butoxycarbonylamino)-5-*tert*-butyl-2-thiophenecarboxylic acid (3.0 g, 0.010 mol) in dioxane (20 mL) was treated with an HCl solution (4.0 M in dioxane, 12.5 mL, 0.050 mol, 5.0 equiv), and the resulting mixture was heated at 80 °C for 2 h. The resulting cloudy solution was allowed to cool to room temp forming some precipitate. The slurry was diluted with EtOAc (50 mL) and cooled to -20 °C. The resulting solids were collected and dried overnight under reduced pressure to give the desired salt as an off-white solid (1.72 g, 90%): ¹H-NMR (DMSO-d₆) δ 1.31 (s, 9H), 6.84 (d, *J*=1.48 Hz, 1H), 7.31 (d, *J*=1.47 Hz, 1H), 10.27 (br s, 3H).

### B. General Methods for Synthesis of Substituted Anilines

### B1. General Method for Substituted Aniline Synthesis via Nucleophilic Aromatic Substitution using a Halopyridine

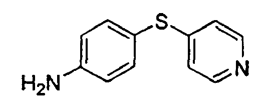

**3-(4-Pyridinylthio)aniline:** To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R_{*f*} 0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### C. General Methods of Urea Formation

### C1a. Reaction of a Heterocyclic Amine with an Aryl Isocyanate

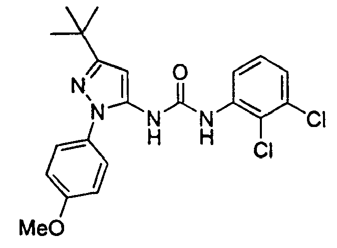

***N*-(1-(4-Methoxyphenyl)-3-*tert*-butyl-5-pyrazolyl)-*N'*-(2,3-dichlorophenyl)urea:** To a stirring solution of 1-(4-methoxyphenyl)-3-tert-butyl-S-aminopyrazole (0.342 g, 1.39 mmol) in anh toluene (9 mL) was added 2,3-dichlorophenyl isocyanate (0.276 mL, 2.09 mmol). The solution was sealed and stirred in the dark for 96 h at 60 °C. After this time, the reaction mixture was diluted with EtOAc (200 mL). The resulting mixture was sequentially washed with a 1 M HCl solution (2 x 125 mL) and a saturated NaCl solution (50 mL), dried (MgSO₄), and concentrated under reduced pressure. The residue was purified by column chromatography (20% EtOAc/80% hexane) to give the product as a white solid (0.335 g, 56%): TLC (20% EtOAc/80% hexane) R_{*f*} 0.22; ¹H NMR (DMSO-d₆) δ 1.24 (s, 9H), 3.79 (s, 3H), 6.33 (s, 1H), 7.05 (d, *J*=9 Hz, 2H), 7.28 (m, 2H), 7.38 (d, *J*=9 Hz, 2H), 8.05 (dd, *J*=3, 6 Hz, 1H), 8.75 (s, 1H), 9.12 (s, 1H); FAB-MS *m*/*z* 433 ((M+H)⁺).

### C1b. Reaction of a Heterocyclic Amine with an Aryl Isocyanate

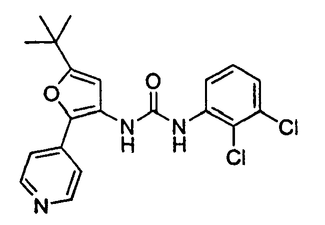

***N*-(2-(4-Pyridinyl)-5-*tert*-butyl-3-furyl)-*N'*-(2,3-dichlorophenyl)urea:** A solution of 3-amino-2-(4-pyridinyl)-5-*tert*-butylfuran (Method A2; 0.10 g, 0.46 mmol) and 2,3-dichlorophenyl isocyanate (0.13 g, 0.69 mmol) in CH₂Cl₂ was stirred at room temp. for 2 h, then was treated with 2-(dimethylamino)ethylamine (0.081 g, 0.92 mmol) and stirred for an additional 30 min. The resulting mixture was diluted with EtOAc (50 mL), then was sequentially washed with a 1 N HCl solution (50 mL), a saturated NaHCO₃ solution (50 mL) and a saturated NaCl solution (50 mL), dried (Na₂SO₄), and concentrated under reduced pressure. The residue was purified using column chromatography (gradient from 10% EtOAc/90% hexane to 40% EtOAc/60% hexane) to give the desired compound as a white solid (0.12 g, 63%): mp 195-198 °C; TLC (60% EtOAc/40% hexane) R_{*f*} 0.47; ¹H NMR (DMSO-d₆) δ 1.30 (s, 9H); 6.63 (s, 1H); 7.30-7.32 (m, 2H), 7.58 (dm, *J*=6.62 Hz, 2H), 8.16 (dd, *J*=2.57, 6.99 Hz, 1H), 8.60 (dm, *J*=6.25 Hz, 2H), 8.83 (s, 1H), 9.17 (s, 1H); ¹³C NMR (DMSO-d₆) δ 28.5 (3C), 32.5, 103.7, 117.3 (2C), 119.8, 120.4, 123.7, 125.6, 128.1, 131.6, 135.7, 136.5, 137.9, 150.0 (2C), 152.2, 163.5; CI-MS *m*/*z* (rel abundance) 404 ((M+H)⁺, 15%), 406 ((M+H+2)⁺, 8%).

### C1c. Reaction of a Heterocyclic Amine with an Isocyanate

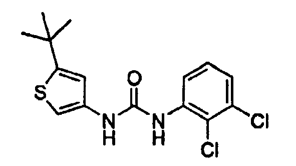

***N*-(5-*tert*-Butyl-3-thienyl)-*N*'-(2,3-dichlorophenyl)urea:** Pyridine (0.163 mL, 2.02 mmol) was added to a slurry of 5-*tert*-butylthiopheneammonium chloride (Method A4c; 0.30 g, 1.56 mmol) and 2,3-dichlorophenyl isocyanate (0.32 mL, 2.02 mmol) in CH₂Cl₂ (10 mL) to clarify the mixture and the resulting solution was stirred at room temp. overnight. The reaction mixture was then concentrated under reduced pressure and the residue was separated between EtOAc (15 mL) and water (15 mL). The organic layer was sequentially washed with a saturated NaHCO₃ solution (15 mL), a 1N HCl solution (15 mL) and a saturated NaCl solution (15 mL), dried (Na₂SO₄), and concentrated under reduced pressure. A portion of the residue was treated by preparative HPLC (C-18 column; 60% acetonitrile/40% water/0.05% TFA) to give the desired urea (0.180 g, 34%): mp 169-170 °C; TLC (20% EtOAc/80% hexane) R_{*f*} 0.57; ¹H-NMR (DMSO-d₆) δ 1.31 (s, 9H), 6.79 (s, 1H), 7.03 (s, 1H), 7.24-7.33 (m, 2H), 8.16 (dd, *J*=1.84, 7.72 Hz, 1H), 8.35 (s, 1H), 9.60 (s, 1H); ¹³C-NMR (DMSO-d₆) δ 31.9 (3C), 34.0, 103.4, 116.1, 119.3,120.0, 123.4, 128.1,131.6,135.6,138.1,151.7,155.2; FAB-MS *m*/*z* (rel abundance) 343 ((M+H)⁺, 83%), 345 ((M+H+2)⁺, 56%), 347 ((M+H+4)⁺, 12%).

### C2. Reaction of Substituted Aniline with N,N'-Carbonyldiimidazole Followed by Reaction with a Heterocyclic Amine

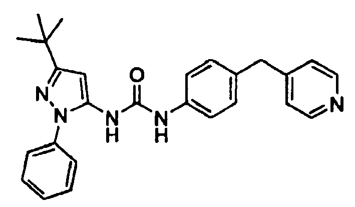

***N*-(1-Phenyl-3-*tert*-butyl-5-pyrazolyl)-*N'*-(4-(4-pyridinylmethyl)phenyl)urea:** A solution of 4-(4-pyridinylmethyl)aniline (0.25 g, 1.38 mmol) and *N,N'-*carbonyldiimidazole (0.23 g, 1.42 mmol) in CH₂Cl₂ (11 mL) at room temp. was stirred for 2 h, then treated with 5-amino-1-phenyl-3-*tert*-butyl-5-pyrazole (0.30 g, 1.38 mmol) and the resulting mixture was stirred at 50 °C overnight The reaction mixture was diluted with EtOAc (25 mL), then sequentially washed with water (30 mL) and a saturated NaCl solution (30 mL), dried (MgSO₄), and concentrated under reduced pressure. The residue was purified by column chromatography (gradient from 100% CH₂Cl₂ to 30% acetone/70% CH₂Cl₂) and the resulting material was recrystallized (EtOAc/Et₂O) to give the desired product complexed with 0.25 equiv H₂O (0.30 g): TLC (60% acetone/40% CH₂Cl₂) R_{*f*} 0.56; ¹H-NMR (DMSO-d₆) δ 1.25 (s, 9H); 3.86 (s, 2H), 6.34 (s, 1H), 7.11 (d, *J*=8.82 Hz, 2H), 7.19 (dm, *J*=6.25 Hz, 2H), 7.31 (d, *J*=1.84 Hz, 2H), 7.35-7.51 (m, 5 H), 8.34 (s, 1H), 8.42 (dm, *J*=5.98 Hz, 2H), 8.95 (s, 1H); FAB-MS *m*/*z* (rel abundance) 426 ((M+H)⁺, 100%).

### D. Interconverslon of Ureas

### D1. General Method for Electrophylic Halogenation of Aryl Ureas

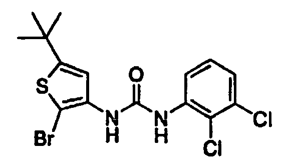

***N-*(2*-Bromo-5-tert*-butyl-3-thienyl)-*N'*-(2-3-dichlorophenyl)urea:** To a slurry of *N-*(5-*tert*-butyl-3-thienyl)-*N'*-(2,3-dichlorophenyl)urea (Method C1c; 3.00 g, 8.74 mmol) in CHCl₃ (200 mL) at room temp was slowly added a solution of Br₂ (0.46 mL, 1.7 mmol) in CHCl₃ (150 mL) via addition funnel over 2.5 h, causing the reaction mixture to become homogeneous. Stirring was continued 20 min after which TLC analysis indicated complete reaction. The reaction mixture was concentrated under reduced pressure, and the residue triturated (Et₂O/hexane) and the resulting solids were washed (hexane) to give the brominated product as a pink powder (3.45 g, 93%): mp 180-183 °C; TLC (10% EtOAc/90% hexane) R_{*f*} 0.68; ¹H NMR (DMSO-d₆) δ 1.28 (s, 9H), 7.27-7.31 (m, 2H), 7.33 (s, 1H), 8.11 (dd, *J*=3.3, 6.6 Hz, 1H), 8.95 (s, 1H), 9.12 (s, 1H); ¹³C NMR (DMSO-d₆) δ 31.5 (3C), 34.7, 91.1, 117.9, 120.1, 120.5, 123.8, 128.0, 131.6, 135.5, 137.9, 151.6, 155.3; FAB-MS *m*/*z* (rel abundance) 421 ((M+H)⁺, 7%), 423 (M+2+H)⁺, 10%).

### D2. General Method for Metal-Mediated Cross-Coupling Reactions with Halogen-Substituted Ureas

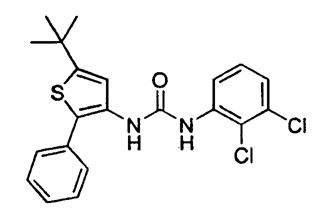

***N*-(2-Phenyl-5-*tert*-butyl-3-thienyl)-*N'*-(2,3-dichlorophenyl)urea:** To a solution of *N*-(3-(2-bromo-5-*tert*-butylthienyl)-*N'*-(2,3-dichlorophenyl)urea (0.50 g, 1.18 mmol) and phenyltrimethyltin (0.21 mL, 1.18 mmol) in DMF (15 mL) was added Pd(PPh₃)₂Cl₂ (0.082 g, 0.12 mmol), and the resulting suspension was heated at 80 °C overnight. The reaction mixture was diluted with EtOAc (50 mL) and water (50 mL), and the organic layer sequentially washed with water (3 x 50 mL) and a saturated NaCl solution (50 mL), then dried (Na₂SO₄) and concentrated under reduced pressure. The residue was purified by MPLC (Biotage® ; gradient from 100% hexane to 5% EtOAc/95% hexane) followed by preparative HPLC (C-18 column; 70% CH₃CN/30% water/0.05% TFA). The HPLC fractions were concentrated under reduced pressure and the resulting aqueous mixture was extracted with EtOAc (2 x 50 mL). The combined organic layers were dried (Na₂SO₄) and concentrated under reduced pressure to give a gummy semi-solid, which was triturated with hexane to afford the desired product as a white solid (0.050 g, 10%): mp 171-173 °C; TLC (5% EtOAc/95% hexane) R_{*f*} 0.25; ¹H NMR (CDCl₃) δ 1.42 (s, 9H), 6.48 (br s, 1H), 7.01 (s, 1H), 7.10-7.18 (m, 2H), 7.26-7.30 (m, 1H), 7.36 (app t, *J*=7.72 Hz, 2H), 7.39 (br s, 1H), 7.50 (dm, *J*=6.99 Hz, 2H), 7.16 (dd, *J*=2.20, 7.72 Hz, 1H); ¹³C NMR (CDCl₃) δ 32.1 (3C), 34.8, 118.4, 118.8, 120.7, 121.1, 124.2, 127.7, 127.9, 128.2 (2C), 128.5, 129.0 (2C), 132.4, 132.5, 136.9, 153.1, 156.3; FAB-MS *m*/*z* (rel abundance) 419 ((M+H)⁺, 6%), 421 ((M+H+2)⁺, 4%).

### D3. General Methods of Reduction of Nitro-Containing Aryl Ureas

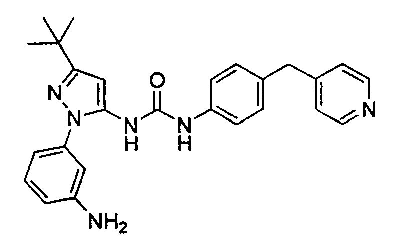

***N*-(1-(3-Aminophenyl)-3-*tert*-butyl-5-pyrazolyl)-*N'*-(4-(4-pyridinylthio)phenyl)urea:** A solution of *N*-(1-(3-nitrophenyl)-3-*tert*-butyl-5-pyrazolyl]-*N'*-(4-(4-pyridinylthio)phenyl)urea (Prepared in methods analogous to those described in A1 and C1a; 0.310 g, 0.635 mmol) in acetic acid (20 mL) was placed under an atmosphere of Ar using a vacuum-degassed and argon-purge protocol. To this was added water (0.2 mL) followed by iron powder (325 mesh; 0.354 g, 6.35 mmol). The reaction mixture was stirred vigorously under argon at room temp. for 18 h, at which time TLC indicated the absence of starting material. The reaction mixture was filtered and the solids were washed copiously with water (300 mL). The orange solution was then brought to pH 4.5 by addition of NaOH pellets (a white precipitate forms). The resulting suspension was extracted with Et₂O (3 x 250 mL), and the combined organic layers were washed with a saturated NaHCO₃ solution (2 x 300 mL) until foaming ceased. The resulting solution was dried (MgSO₄) and concentrated under reduced pressure. The resulting white solid was purified by column chromatography (gradient from 30% acetone/70% CH₂Cl₂ to 50% acetone/50% CH₂Cl₂) to give the product as a white solid (0.165 g, 57%): TLC (50% acetone/50% CH₂Cl₂) R_{*f*} 0.50; ¹H NMR (DMSO-d₆) δ 1.24 (s, 9H), 5.40 (br s, 2H), 6.34 (s, 1H), 6.57 (d, *J*=8 Hz, 2H), 6.67 (s, 1H), 6.94 (d, *J*=6 Hz, 2H), 7.12 (app t, *J*=8 Hz, 1H), 7.47 (d, *J*=9 Hz, 2H), 7.57 (d, *J*=9 Hz, 2H), 8.31 (d, *J*=6 Hz, 2H), 8.43 (s, 1H), 9.39 (s, 1H); FAB-MS *m*/*z* 459 ((M+H)⁺).

### D4. General Methods of Acylation of Amine-Containing Aryl Ureas

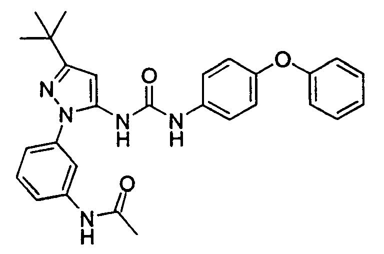

***N*-(1-(3-Acetamidophenyl)-3-*tert*-butyl-5-pyrazolyl)-*N'*-(4-phenoxyphenyl)urea:** To a solution of *N*-(1-(3-aminophenyl)-3-*tert*-butyl-5-pyrazolyl)-*N'*-(4-phenoxyphenyl)urea (prepared using methods analogous to those described in A1, C1a and D3; 0.154 g, 0.349 mmol) in CH₂Cl₂ (10 mL) was added pyridine (0.05 mL) followed by acetyl chloride (0.030 mL, 0.417 mmol). The reaction mixture was stirred under argon at room temp. for 3 h, at which time TLC analysis indicated the absence of starting material. The reaction mixture was diluted with CH₂Cl₂(20 mL), then the resulting solution was sequentially washed with water (30 mL) and a saturated NaCl solution (30 mL), dried (MgSO₄) and concentrated under reduced pressure. The resulting residue was purified by column chromatography (gradient from 5% EtOAc/95% hexane to 75% EtOAc/25% hexane) to give the product as a white solid (0.049 g, 30%): TLC (70% EtOAc/30% hexane) R_{*f*} 0.32; ¹H NMR (DMSO-d₆) δ 1.26 (s, 9H), 2.05 (s, 3H), 6.35 (s, 1H), 6.92-6.97 (m, 4H), 7.05-7.18 (m, 2H), 7.32-7.45 (m, 5H), 7.64-7.73 (m, 2H), 8.38 (s, 1H), 9.00 (s, 1H), 10.16 (s, 1H); FAB-MS *m*/*z* 484 ((M+H)⁺).

The following compounds have been synthesized according to the General Methods listed above:

**Table 1. 2-Substituted-5-tert-butylpyrazolyl Ureas**

| | | | | | | | |
|---|---|---|---|---|---|---|---|
| 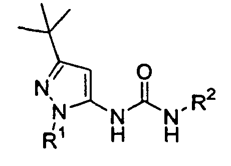 | | | | | | | |

| Entry | R¹ | R² | mp (°C) | TLC R_{*f*} | Solvent System | Mass Spec. [Source] | Synth. Method |
|---|---|---|---|---|---|---|---|
| 1 | 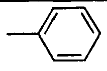 | 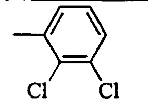 | | 0.42 | 20% EtOAc/ 80% hexane | 403 (M+H)+ [FAB] | A1, C1a |
| 2 | 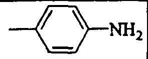 | 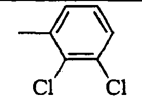 | | 0.50 | 67% EtOAc/ 33% hexane | 418 (M+H)+ [FAB] | A1, C1a, D3 |
| 3 | 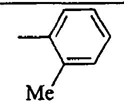 | 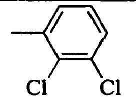 | | 0.27 | 20% EtOAc/ 80% hexane | 417 (M+H)+ [FAB] | A1, C1a |
| 4 | 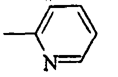 | 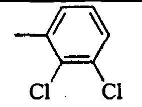 | | 0.47 | 20% EtOAc/ 80% hexane | 404 (M+H)+ [FAB] | A1, C1a |
| 5 | 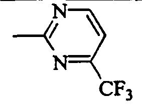 | 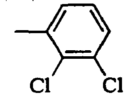 | | 0.30 | 33% EtOAc/ 67% hexane | 473 (M+H)+ [FAB] | A1, C1a |
| 6 | 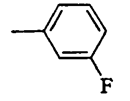 | 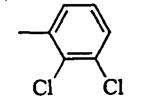 | | 0.27 | 100% EtOAc | 421 (M+H)+ [FAB] | A1, C1a |
| 7 | 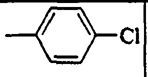 | 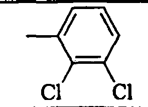 | | 0.50 | 20% EtOAc/ 80% hexane | 437 (M+H)+[FAB] | A1, C1a |
| 8 | 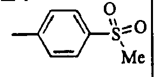 | 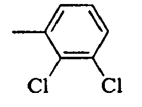 | | 0.60 | 50% EtOAc/ 50% hexane | 481 (M+H)+ [FAB] | A1, C1a |
| 9 | 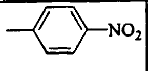 | 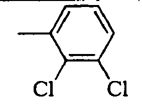 | | 0.37 | 20% EtOAc/ 80% hexane | 448 (M+H)+ [FAB.] | A1, C1a |
| 10 | 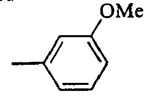 | 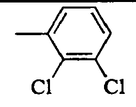 | | 0.35 | 20% EtOAc/ 80% hexane | 433 (M+H)+ [FAB] | A1, C1a |
| 11 | 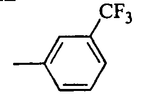 | 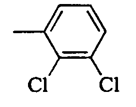 | | 0.40 | 20% EtOAc/ 80% hexane | 471 (M+H)+ [FAB] | A1, C1a |
| 12 | 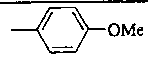 | 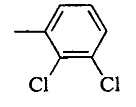 | | 0.22 | 20% EtOAc/ 80% hexane | 433 (M+H)+ [FAB] | A1, C1a |
| 13 | 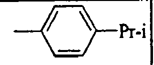 | 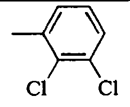 | | 0.51 | 20% EtOAc/ 80% hexane | 445 (M+H)+ [FAB] | A1, C1a |
| 14 | 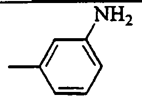 | 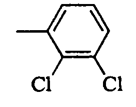 | | 0.39 | 50% EtOAc/ 50% hexane | 418 (M+H)+ [FAB] | A1, C1a, D3 |
| 15 | 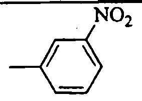 | 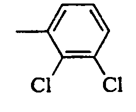 | | 0.31 | 30% EtOAc/ 70% hexane | 448 (M+H)+ [FAB] | A1, C1a |
| 16 | 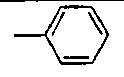 | 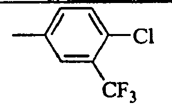 | 195 - 200 | | | 437 (M+H)+ [FAB] | A1, C1a |
| 17 | 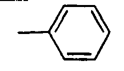 | 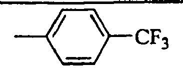 | 97- 100 | | | 403 (M+H)+ [FAB] | A1, C1a |
| 18 | 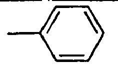 | 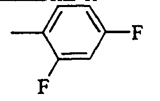 | 84- 85 | | | 371 (M+H)+ [FAB] | A1, C1a |
| 19 | 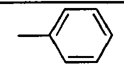 | 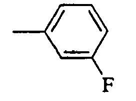 | 156 159 | | | 353 (M+H)+ [FAB] | A1, C1a |
| 20 | 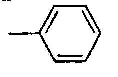 | 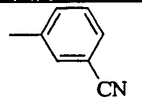 | 168 169 | | | 360 (M+H)+ [FAB] | A1, C1a |
| 21 | 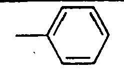 | 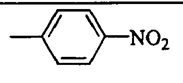 | 131 - 135 | | | 380 (M+H)+ [CI] | A1, C1a |
| 22 | 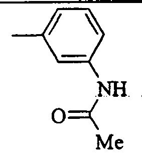 | 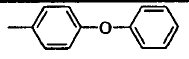 | | 0.31 | 70% EtOAc/ 30% hexane | 484 (M+H)+ [FAB] | A1, C1a, D3, D4 |
| 23 | 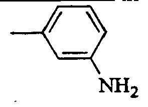 | 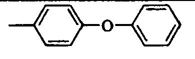 | | 0.14 | 50% EtOAc/ 50% hexane | 442 (M+H)+ [FAB] | A1, C1a, D3 |
| 24 | 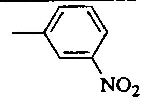 | 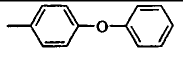 | | 0.19 | 30% EtOAc/ 70% hexane | 472 (M+H)+ [FAB] | A1, C1a |
| 25 | 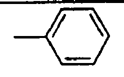 | 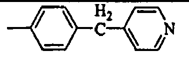 | | 0.56 | 60% acetone / 40% CH2Cl 2 | 426 (M+H)+ [FAB] | A1, C2 |
| 26 | 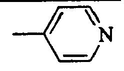 | 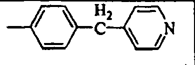 | | 0.34 | 10% MeOH/ 90% CH2Cl 2 | 427 (M+H)+ [FAB] | A1, C2 |
| 27 | 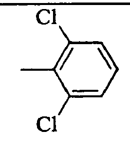 | 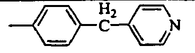 | | 0.44 | 40% acetone 60% CH2Cl 2 | 494 (M+H)+ [FAB] | A1, C2 |
| 28 | 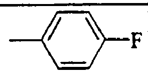 | 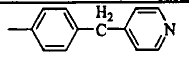 | | 0.44 | 40% acetone / 60% CH2Cl 2 | 444 (M+H)+ [FAB] | A1, C2 |
| 29 | 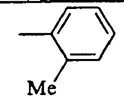 | 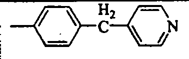 | | 0.46 | 40% acetone / 60% CH2Cl 2 | 440 (M+H)+ [FAB] | A1, C2 |
| 30 | 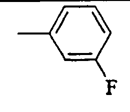 | 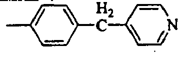 | | 0.48 | 40% acetone / 60% CH2Cl 2 | 444 (M+H)+ [FAB] | A1, C2 |
| 31 | 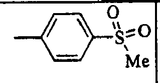 | 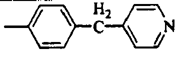 | | 0.34 | 40% acetone / 60% CH2Cl 2 | 504 (M+H)+ | A1, C2 |
| 32 | 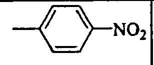 | 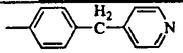 | | 0.47 | 40% acetone / 60% CH2Cl 2 | 471 (M+H)+ [FAB] | A1, C2 |
| 33 | 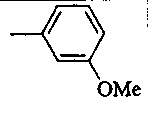 | 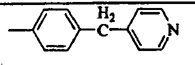 | | 0.51 | 60% acetone / 40% CH2Cl 2 | 456 (M+H)+ [FAB] | A1, C2 |
| 34 | 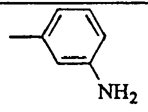 | 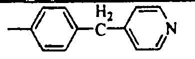 | | 0.50 | 50% acetone / 50% CH2Cl 2 | 441 (M+H)+ [FAB] | A1, C2, D3 |
| 35 | 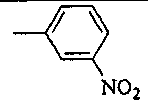 | 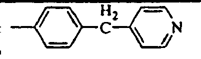 | | 0.43 | 30% acetone / 70% CH2Cl 2 | 471 (M+H)+ [FAB] | A1, C2 |
| 36 | 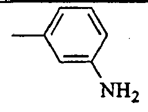 | 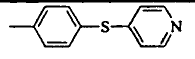 | | 0.50 | 50% acetone / 50% CH2Cl 2 | 459 (M+H)+ [FAB] | A1, C2, D3 |
| 37 | 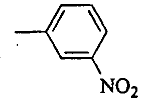 | 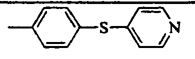 | | 0.47 | 30% acetone / 70% CH2Cl 2 | 489 (M+H)+ [FAB] | A1, C2 |
| 38 | 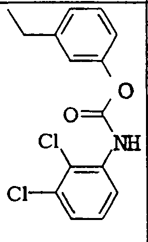 | 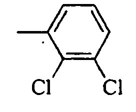 | | 0.47 | 50% EtOAc/ 50% hexane | 620 (M+H)+ [FAB] | A1, C2 |
| 39 | 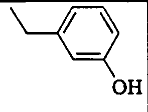 | 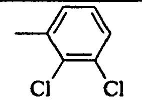 | | 0.34 | 50% EtOAc/ 50% hexane | 433 (M+H)+ [FAB] | A1, C2 |

**Table 2. Additional Ureas**

| Entry | R² | mp (°C) | TLC R_{*f*} | Solvent System | Mass Spec. | Synth. Method |
|---|---|---|---|---|---|---|
| 40 | 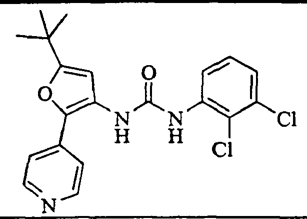 | 195- 198 | 0.47 | 60% EtOAc/ 40% hexane | 404 (M+H)+ [FAB] | A2, C1b |
| 41 | 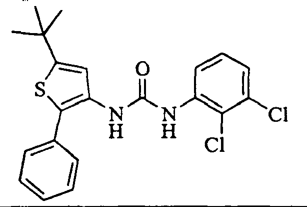 | 171- 173 | 0.25 | 5% EtOAc/ 95% hexane | 419 (M+H)+ [FAB] | A3, C1c, D1, D2 |

### BIOLOGICAL EXAMPLES

### In Vitro raf Kinase Assay:

In an in vitro kinase assay, raf is incubated with MEK in 20 mM Tris-HCI, pH 8.2 containing 2 mM 2-mercaptoethanol and 100 mM NaCl. This protein solution (20 µL) is mixed with water (5 µL) or with compounds diluted with distilled water from 10 mM stock solutions of compounds dissolved in DMSO. The kinase reaction is initiated by adding 25 µL [γ-³³P]ATP (1000-3000 dpm/pmol) in 80 mM Tris-HCl, pH 7.5, 120 mM NaCl, 1.6 mM DTT, 16 mM MgCl₂. The reaction mixtures are incubated at 32°C, usually for 22 min. Incorporation of ³³P into protein is assayed by harvesting the reaction onto phosphocellulose mats, washing away free counts with a 1% phosphoric acid solution and quantitating phosphorylation by liquid scintillation counting. For high throughput screening, 10 µM ATP and 0.4 µM MEK are used. In some experiments, the kinase reaction is stopped by adding an equal amount of Laemmli sample buffer. Samples are boiled 3 min and the proteins resolved by electrophoresis on 7.5% Laemmli gels. Gels are fixed, dried and exposed to an imaging plate (Fuji). Phosphorylation is analyzed using a Fujix Bio-Imaging Analyzer System.

All compounds exemplified displayed IC₅₀s of between 1 nM and 10 µM.

### Cellular Assay:

For in vitro growth assay, human tumor cell lines, including but not limited to HCT116 and DLD-1, containing mutated K-ras genes are used in standard proliferation assays for anchorage dependent growth on plastic or anchorage independent growth in soft agar. Human tumor cell lines were obtained from ATCC (Rockville MD) and maintained in RPMI with 10% heat inactivated fetal bovine serum and 200 mM glutamine. Cell culture media and additives are obtained from Gibco/BRL (Gaithersburg, MD) except for fetal bovine serum (JRH Biosciences, Lenexa, KS). In a standard proliferation assay for anchorage dependent growth, 3 X 10³ cells are seeded into 96-well tissue culture plates and allowed to attach overnight at 37 °C in a 5% CO₂ incubator. Compounds are titrated in media in dilution series and added to 96 well cell cultures. Cells are allowed to grow 5 days typically with a feeding of fresh compound containing media on day three. Proliferation is monitored by measuring metabolic activity with standard XTT colorimetric assay (Boehringer Mannheim) measured by standard ELISA plate reader at OD 490/560, or by measuring ³H-thymidine incorporation into DNA following an 8 h culture with 1 µCu ³H-thymidine, harvesting the cells onto glass fiber mats using a cell harvester and measuring ³H-thymidine incorporation by liquid scintillant counting.

For anchorage independent cell growth, cells are plated at 1 x 10³ to 3 x 10³ in 0.4% Seaplaque agarose in RPMI complete media, overlaying a bottom layer containing only 0.64% agar in RPMI complete media in 24-well tissue culture plates. Complete media plus dilution series of compounds are added to wells and incubated at 37 °C in a 5% CO₂ incubator for 10-14 days with repeated feedings of fresh media containing compound at 3-4 day intervals. Colony formation is monitored and total cell mass, average colony size and number of colonies are quantitated using image capture technology and image analysis software (Image Pro Plus, media Cybernetics).

These assays establish that the compounds of formula I are active to inhibit raf kinase activity and to inhibit oncogenic cell growth.

### In Vivo Assay:

An in vivo assay of the inhibitory effect of the compounds on tumors (e.g., solid cancers) mediated by raf kinase can be performed as follows:

CDI nu/nu mice (6-8 weeks old) are injected subcutaneously into the flank at 1 x 10⁶ cells with human colon adenocarcinoma cell line. The mice are dosed i.p., i.v. or p.o. at 10, 30, 100, or 300 mg/Kg beginning on approximately day 10, when tumor size is between 50-100 mg. Animals are dosed for 14 consecutive days once a day; tumor size was monitored with calipers twice a week.

The inhibitory effect of the compounds on raf kinase and therefore on tumors (e.g., solid cancers) mediated by raf kinase can further be demonstrated in vivo according to the technique of Monia et al. (*Nat. Med.* **1996**, *2*, 668-75).

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. A compound of formula I and pharmaceutically acceptable salts thereof 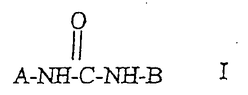 wherein A is a heteroaryl selected from the group consisting of 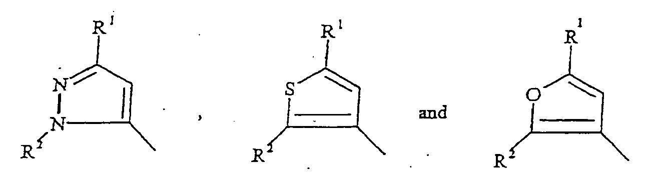 wherein R¹ is selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloalkyl;
B is an up to tricyclic, aryl or heteroaryl moiety of up to 12 carbon atoms with at least one 5- or 6-member aromatic structure containing 0-4 members of the group consisting of nitrogen, oxygen and sulfur, substituted by -Y-Ar
and optionally substituted by one or more substituents independently selected from the group consisting of halogen, up to per-halosubstitution, and Xₙ,
wherein n is 0-3 and each X is independently selected from the group consisting of-CN, -CO₂R⁵, -C(O)NR⁵R^{5'}, -C(O)R⁵, -NO₂, -OR⁵, -SR⁵, -NR⁵R^{5'}, -NR⁵C(O)OR⁵, -NR⁵C(O)R^{5'}, C₁-C₁₀ alkyl, C₂-C₁₀ alkenyl, C₁-C₁₀ alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂-C₁₀ alkenyl, substituted C₁-C₁₀ alkoxyl, substituted C₃-C₁₀ cycloalkyl, substituted C₄-C₂₃ alkheteroaryl;
where X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂-C₁₀ alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to perhalosubstituted C₂-C₁₀ alkenyl, up to per-halosubstituted C₃-C₁₀ cycloalkyl, up to per-halosubstituted C₆-C₁₄ aryl and up to per-halosubstituted C₃-C₁₃ heteroaryl,
wherein Y is -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵, -O(CH₂)ₘ-, -(CH₂)ₘS-, -(CH₂)ₘN(R⁵)-, -O(CH₂)ₘ-, -CHX^{a}-, -CX^{a}₂-, -S-(CH₂)ₘ- or -N(R⁵)(CH₂)ₘ- ,
m =1-3, and X^{a} is halogen; and
Ar is a 5-10 member aromatic structure containing 0-2 members of the group consisting of nitrogen, oxygen and sulfur atoms which is unsubstituted or substituted by halogen up to per-halosubstitution and optionally substituted by Zₙ₁, wherein n1 is 0 to 3 and each Z is independently selected from the group consisting of -CN, -CO₂R⁵ -C(O)NR⁵R^{5'}, -C(O)NR⁵, -NO₂,-OR⁵, -SR⁵ -NR⁵R^{5'}, -NR⁵C(O)OR^{5'}, -C(O)R⁵, NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl;
wherein if Z is a substituted group, it is substituted by the one or more substituents independently selected from the group consisting of-CN, -CO₂R⁵, -C(O)NR⁵R⁵, -OR⁵, -SR⁵, -NO₂, -NR⁵R^{5'}, -NR₅C(O)R^{5'} and -NR⁵C(O)OR^{5'}, and
wherein R² is C₆-C₁₄ aryl, C₃-C₁₄ heteroaryl, substituted C₆-C₁₄ aryl or substituted C₃-C₁₄ heteroaryl,
wherein if R² is a substituted group, it is substituted by one or more substituents independently selected from the group consisting of halogen, up to per-halosubstitution, and Vₙ,
wherein n = 0-3 and each V is independently selected from the group consisting of-CN, -CO₂R⁵, -C(O)NR⁵R^{5'}, -OR⁵, -SR⁵, -NR⁵R^{5'}, -C(O)R^{5'}, -OC(O)NR⁵R^{5'}, -NR⁵C(O)OR^{5'}, -SO₂R⁵, -SOR⁵, -NR⁵C(O)R^{5'}, -NO₂, C₁-C₁₀ alkyl, C₃₋C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where if V is a substituted group, it is substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} and -NO₂;
wherein R⁵ and R^{5'} are each independently as defined above.

2. A compound of claim 1, wherein R² is substituted or unsubstituted phenyl or pyridinyl, and the substituents for R² are selected from the group consisting of halogen, up to per-halosubstitution and Vₙ, wherein n = 0-3, and each V is independently selected from the group consisting of substituted and unsubstituted C₁₋C₆ alkyl, C₃-C₁₀ cycloalkyl, C₆-C₁₀ aryl, -NO₂, -NH₂, -C(O)-C₁-₆ alkyl, -C(O)N-(C₁₋₆ alkyl)₂, -C(O)NH-C₁₋₆ alkyl -O-C₁₋₆ alkyl, -NHC(O)H, -NHC(O)OH, -N(C₁-₆ alkyl)C(O)-C₁-₆ alkyl, -N-(C₁-₆ alkyl)C(O)-C₁-₆ alkyl, -NHC(O)-C₁-₆ alkyl, -OC(O)NH C₆₋₁₄ aryl , -NHC(O)O-C₁-₆ alkyl, -S(O)-C₁-₆ alkyl and -SO₂-C₁-₆ alkyl,
wherein if V is a substituted group, it is substituted by one or more halogen, up to per-halosubstitution.

3. A compound of claim 1, wherein B is 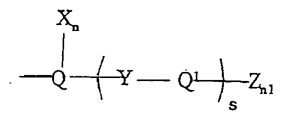 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen ;
Q is a six member aromatic structure containing 0-2 nitrogen, substituted or unsubstituted by halogen, up to per-halosubstitution;
Q¹ is a mono- or bicyclic aromatic structure of 3 to 10 carbon atoms and 0-4 members of the group consisting of N, O and S, unsubstituted or unsubstituted by halogen up to per-halosubstitution,
n is 0, Z and n1 are as defined in claim 1, and s = 1.

4. A compound of claim 3, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, and
Z is selected from the group consisting of R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, C₃-C₆-cycloalkyl and C₆-C₁₀-aryl, wherein R⁶ and R⁷ can be substituted by halogen or up to per-halosubstitution.

5. A compound of claim 1, wherein R¹ is t-butyl and R² is unsubstituted or substituted phenyl.

6. A compound of claim 4, wherein Q is phenyl, Q¹ is phenyl or pyridinyl, Y is -O-, -S- or-CH₂-, and X and Z are independently Cl, F, NO₂ or CF₃.

7. A compound of claim 6 wherein R¹ is t-butyl.

8. A compound of claim 1 of the formula 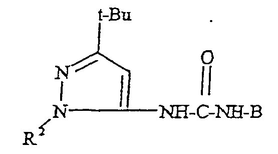 wherein B and R² are as defined in claim 1.

9. A compound of claim 8, wherein R² is selected from substituted and unsubstituted members of the group consisting of phenyl and pyridinyl, wherein if R² is a substituted group, it is substituted by one or more of the substituents selected from the group consisting of halogen and Wₙ, wherein n = 0-3, and W is selected from the group consisting of -NO₂, -C₁-₃, alkyl, -NH(O)CH₃, -CF₃, -OCH₃, -F, -Cl, -NH₂, -OC(O)NH- up to per-halosubstituted phenyl, -SO₂CH₃, pyridinyl, phenyl, up to per-halosubstituted phenyl and C₁-C₆ alkyl substituted phenyl.

10. A compound of claim 1 of the formula 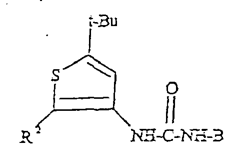 wherein B and R² are as defined in claim 1.

11. A compound of claim 10, wherein R² is selected from substituted and unsubstituted members of the group consisting of phenyl and pyridinyl, wherein if R² is a substituted group, it is substituted by one or more substituents selected from the group consisting of halogen and Wₙ, wherein n = 0-3, and W is selected from the group consisting of -NO₂, -C₁₋₃ alkyl, -NH(O)CH₃, -CF₃, -OCH₃, -F, -Cl, -NH₂, -SO₂CH₃, pyridinyl, phenyl, up to per-halosubstituted phenyl and C₁-C₆ alkyl substituted phenyl.

12. A compound of claim 1 of the formula 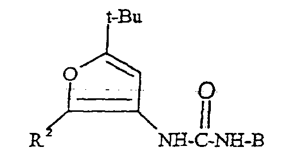 wherein B and R² are as defined in claim 1.

13. A compound of claim 13, wherein R² is selected from substituted and unsubstituted members of the group consisting of phenyl and pyridinyl, wherein if R² is a substituted group, it is substituted by one or more substituents selected from the group consisting of halogen and Wₙ, wherein n = 0-3, and W is selected from the group consisting of -NO₂, -C₁₋₃ alkyl, -NH(O)CH₃, -CF₃, -OCH₃, -F, -Cl, -NH₂, -SO₂CH₃, pyridinyl, phenyl, up to per-halosubstituted phenyl and C₁-C₆ alkyl substituted phenyl.

14. Use of a compound of formula I or a pharmaceutically acceptable salt thereof 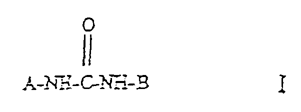 wherein A is a heteroaryl selected from the group consisting of 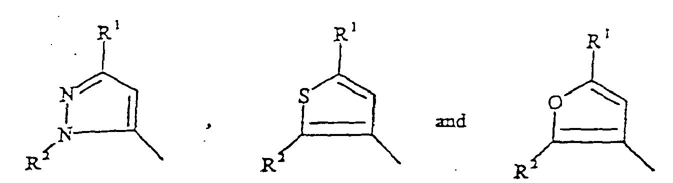 wherein R¹ is selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloalkyl;
B is a substituted or unsubstituted, up to tricyclic, aryl or heteroaryl moiety of up to 12 carbon atoms with at least one 5- or 6-member aromatic structure containing 0-4 members of the group consisting of nitrogen, oxygen and sulfur, wherein if B is a substituted group, it is substituted by one or more substituents independently selected from the group consisting of halogen, up to per-halosubstitution, and Xₙ,
wherein n is 0-3 and each X is independently selected from the group consisting of -CN, CO₂R⁵, -C(O)NR⁵R^{5'}, -C(O)R⁵, -NO₂, -OR⁵,- SR⁵, - NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl substituted C₂₋₁₀-alkenyl, substituted C₁₋₁₀-alkoxy subsituted C₃-C₁₀ cycloalkyl, substituted C₄-C₂₃ alkheteroaryl and -Y-Ar;
where X is a substituted group, it is substituted by one or more substituents independently selected from the group consisting of-CN, -CO₂R⁵, -C(O)R⁵, -C(O)NR⁵R⁵, -OR⁵, -SR⁵, -NR⁵R⁵, -NO₂, -NR⁵C(O)R⁵, -NR⁵C(O)OR⁵ and halogen up to per-halosubstitution,
wherein R⁵ and R^{5'} are independently selected from H, C₁-C₁₀ alkyl, C₂₋₁₀₋alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstitued C₂₋₁₀₋alkenyl up to per-halosubstituted C₃-C₁₀ cycloalkyl, up to per-halosubstituted C₆-C₁₄ aryl and up to per-halosubstituted C₃-C₁₃ heteroaryl, wherein Y is - O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- or -N(R⁵)(CH₂)ₘ-,
m = 1-3, and X¹ is halogen; and
Ar is a 5- or 6-member aromatic structure containing 0-2 members of the group consisting of nitrogen, oxygen and sulfur atoms which is unsubstituted or substituted by halogen up to per-halosubstitution and optionally substituted by Zₙ₁, wherein n1 is 0 to 3 and each Z is independently selected from the group consisting of -CN, -C(O)R⁵, -CO₂R³, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, C₁-C₁₀ alkyl, C₃-C₁₀ cycloalkyl, - C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl;
wherein if Z is a substituted group, it is substituted by the one or more substituents independently selected from the group consisting of -CN, -CO₂R⁵, -C(O)NR⁵R^{5'}, -OR⁵, -SR⁵, -NO₂, -NR⁵R^{5'}, -N-R⁵C(O)R^{5'} and -NR⁵C(O)OR^{5'}, and
wherein R² is C₆-C₁₄ aryl, C₃-C₁₄ heteroaryl, substituted C₆-C₁₄ aryl or substituted C₃-C₁₄ heteroaryl,
wherein if R² is a substituted group, it is substituted by one or more substituents independently selected from the group consisting of halogen, up to per-halosubstitution, and Vₙ,
wherein n = 0-3 and each V is independently selected from the group consisting of-CN, -CO₂R⁵, -C(O)NR⁵R^{5'}, -OR⁵, -SR⁵, -NR⁵R^{5'}, -OC(O)NR⁵R^{5'}, -NR⁵C(O)OR⁵, -NR⁵C(O)OR⁵, -SO₂R⁵, -SOR⁵, -NR⁵C(O)R^{5'}, -NO₂, C₁-C₁₀ alkyl, C₃₋C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where V is a substituted group, it is substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} and -NO₂,
wherein R⁵ and R^{5'} are each independently as defined above,
for the preparation of a medicament for the treatment of disease mediated by raf kinase.

15. A use as in claim 14, wherein R² is selected from substituted or unsubstituted members of the group consisting of phenyl and pyridinyl, and the substituents for R² are selected from the group consisting of halogen, up to per-halosubstituition and Vₙ, wherein n = 0-3, and each V is independently selected from
the group consisting of substituted and unsubstituted C₁-C₆ alkyl, C₃-C₁₀ cycloalkyl, C₆-C₁₀, aryl, -NO₂, -NH₂, -C(O)-C₁-₆ alkyl -C(O)N-(C₁-₆ alkyl)₂, -C(O)NH-C₁-₆ alkyl,-O-C₁₋₆ alkyl, -NHC(O)H, -NHC(O)OH, -N(C₁-₆ alkyl)C(O)-C₁₋₆ alkyl, -N-(C₁-₆ alkyl)C(O)-C₁₋₆ alkyl, -NHC(O)-C₁-₆alkyl, -NHC(O)O-C₁-₆ alkyl, -S(O)-C₁-₆ alkyl and -SO₂-C₁-₆alkyl,
wherein if V is a substituted group, it is substituted by one or more halogen, up to per-halosubstitution.

16. A use as in claim 14, wherein B is up to a tricyclic aromatic ring structure selected from the group consisting of 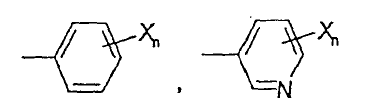 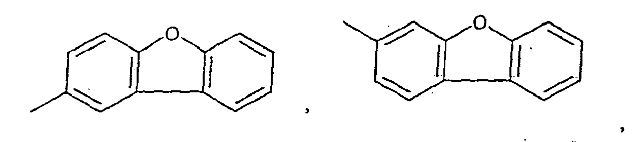 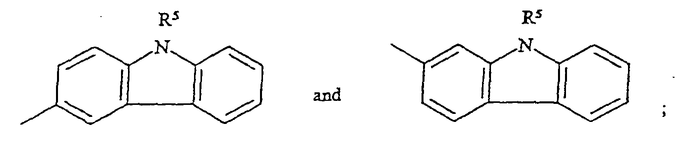 which is substituted or unsubstituted by halogen, up to per-halosubstitution, and wherein
n = 0-3 and
each X is independently selected from the group consisting of-CN, -CO₂R⁵, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁₋C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃₋C₁₃ heteroaryl, C₄-C₂₃ alkheteroaryl, and substituted C₁-C₁₀ alkyl, substituted C₂₋₁₀₋alkenyl, substituted C₁₋₁₀-alkoxy, substituted C₃-C₁₀ cycloalkyl, substituted C₄-C₂₃ alkheteroaryl and -Y-Ar;
wherein if X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-balosubstituted C₂₋₁₀-alkenyl, up to per-halosubstituted C₃-C₁₀ cycloalkyl, up to per-halosubstituted C₆-C₁₄ aryl and up to per-halosubstituted C₃-C₁₃ heteroaryl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, CHX^{a}-, -CX^{a}₂,-, -S-(CH₂)ₘ- and -N(R⁵)(CH₂)ₘ-,
m =1-3, and X^{a} is halogen; and
Ar is a 5-10 member aromatic structure containing 0-2 members of the group consisting of nitrogen, oxygen and sulfur which is unsubstituted or substituted by halogen up to per-halosubstitution and optionally substituted by Zₙ₁, wherein n1 is 0 to 3 and each Z is independently selected from the group consisting of -CN, -C(O)R⁵, -CO₂R⁵ -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein if Z is a substituted group, it is substituted by one or more substituents independently selected from the group consisting of -CN, -CO₂R⁵, -C(O)NR⁵R^{5'}, -OR⁵, -SR⁵, -NO₂, -NR⁵R^{5'}, -NR⁵C(O)R^{5'} and -NR⁵C(O)OR^{5'}.

17. A use of claim 14 wherein B is 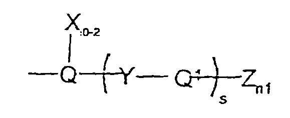 wherein
Y is selected from the group consisting of -O-, -S-, -CH₂-, -SCH₂-, -CH₂S-, -CH(OH)-, -C(O)- -CX^{a}₂, -CX^{a}H-, -CH₂O- and -OCH₂-,
X^{a} is halogen,
Q is a six member aromatic structure containing 0-2 nitrogen, substituted or unsubstituted by halogen, up to per-halosubstitution;
Q¹ is a mono- or bicyclic aromatic structure of 5-10 members with 3 to 10 carbon atoms and 0-2 members of the group consisting of N, O and S, unsubstituted or substituted by halogen up to per-halosubstitution,
X, Z and n1 are as defined in claim 15, and s = 0 or 1.

18. A use as in claim 17 , wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution,
Z and X are independently selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, C₃-C₆ cycloalkyl and C₆-C₁₀-aryl, wherein R⁶ and R⁷ can be substituted by halogen or up to per-halosubstitution.

19. A use as in claim 17, wherein Q is phenyl, Q¹ is phenyl or pyridinyl, Y is -O-, -S- or -CH₂-, and X and Z are independently Cl, F, NO₂ or CF₃.

20. A use as in claim 14, which comprises administering a compound of one of the formulae 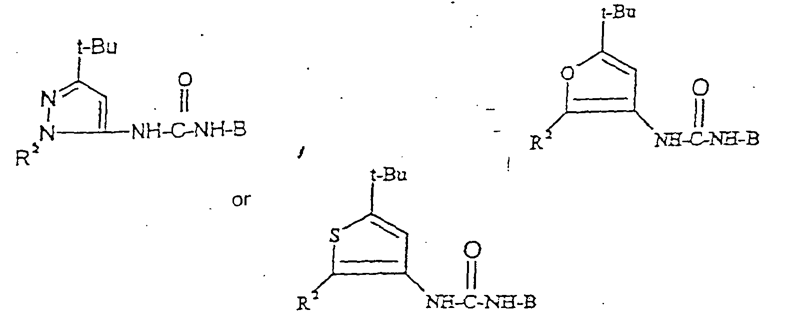 wherein B and R² are as defined in claim 15.

21. A use as in claim 20, wherein R² is selected from substituted and unsubstituted members of the group consisting of phenyl or pyridinyl, wherein if R² is a substituted group, it is substituted by one or more substituents selected from the group consisting of halogen and Wₙ, wherein n = 0-3, and W is selected from the group consisting of -NO₂, -C₁₋₃ alkyl, -NH(O)CH₃, -CF₃, -OCH₃, -F, -Cl, -NH₂, -OC(O)NH- up to per-halosubstituted phenyl, -SO₂CH₃, pyridinyl, phenyl, up to per-halosubstituted phenyl and C₁-C₆ alkyl substituted phenyl.

22. A use as in claim 14, comprising administering an amount of compound of formula I effective to inhibit raf kinase.

23. A pharmaceutical composition comprising a compound of claim 1 and a pharmaceutically acceptable carrier.

24. A pharmaceutical composition comprising a compound of claim 2 and a pharmaceutically acceptable carrier.

25. Use of a compound of formula I or a pharmaceutically acceptable salt thereof to a host in need thereof: 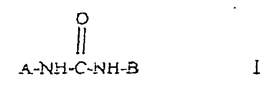 wherein A is 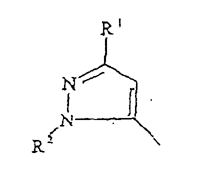 wherein R¹ is selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloalkyl;
B is a substituted or unsubstituted, up to tricyclic, aryl or heteroaryl moiety of up to 12 carbon atoms with at least one 5- or 6-member aromatic structure containing 0-4 members of the group consisting of nitrogen, oxygen and sulfur, wherein if B is a substituted group, it is substituted by one or more substituents independently selected from the group consisting of halogen, up to per-halosubstitution, and Xₙ,
wherein n is 0-3 and each X is independently selected from the group consisting of -CN, CO₂R⁵, -C(O)NR⁵R^{5'}, -C(O)R⁵, -NO₂, -OR⁵, SR⁵, - NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂₋₁₀-alkenyl, substituted C₁₋₁₀-alkoxy, substituted C₃-C₁₀ cycloalkyl, up to per-halosubstituted C₆-C₁₄ aryl, up to per-halosubstituted C₃₋C₁₃ heteroaryl substituted C₄-C₂₃ alkheteroaryl and -Y-Ar;
where X is 4 substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀₋alkenyl, C₃-C₁₀ cycloalkyl, C₆-C₁₄ aryl, C₃-C₁₃ heteroaryl, C₇-C₂₄ alkaryl, C₄-C₂₃ alkheteroaryl up to per-halosubstituted C₁-C₁₀ alkyl, up to per-halosubstituted C₂₋₁₀₋alkenyl up to per-halosubstituted C₃-C₁₀ cycloalkyl, up to per-halosubstituted C₆-C₁₄ aryl and up to per-halosubstituted C₃-C₁₃ heteroaryl,
wherein Y is - O-, -S-, -N(R⁵)-, -(CH₂)ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- or -N(R⁵)(CH₂)ₘ-,
m = 1-3, and X^{a} is halogen; and
Ar is a 5- or 6-member aromatic structure containing 0-2 members of the group consisting of nitrogen, oxygen and sulfur atoms which is unsubstituted or substituted by halogen up to per-halosubstitution and optionally substituted by Zₙ₁, wherein n1 is 0 to 3 and each Z is independently -CN, -C(O)R⁵, -CO₂R⁵, -C(O)NR⁵R^{5'}, -C(O)NR⁵,-NO₂, -OR⁵, -SR⁵, -NR⁵R^{5'}, -NR⁵C(O)OR^{5'}, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl or substituted C₄-C₂₃ alkheteroaryl,
wherein if Z is a substituted group, it is substituted by the one or more substituents independently selected from the group consisting of -CN, -CO₂R⁵, -C(O)NR⁵R^{5'}, -OR⁵, -SR⁵, -NO₂, -NR⁵R^{5'}, -NR⁵C(O)R^{5'} and -NR⁵C(O)OR^{5'}, and
wherein R² is C₆-C₁₄ aryl, C₃-C₁₄ heteroaryl, substituted C₆-C₁₄ aryl or substituted C₃-C₁₄ heteroaryl,
wherein if R² is a substituted group, it is substituted by one or more substituents independently selected from the group consisting of halogen, up to per-halosubstitution, and Vₙ,
wherein n = 0-3 and each V is independently selected from the group consisting of -CN. -CO₂R⁵, -C(O)NR⁵R^{5'}, -OR⁵, -SR⁵, -NR⁵R^{5'}, -OC(O)NR⁵R^{5'}, -C(O)R⁵, -NR⁵C(O)OR^{5'}, -SO₂R⁵, -SOR⁵, -NR⁵C(O)R^{5'}, -NO₂ C₁-C₁₀ alkyl, C₃₋C₁₀ cycloalkyl, C₆-C₁₄ aryl, C₃-C₁₃ heteroaryl, C₇-C₂₄ alkaryl, C₄-C₂₄ alkheteroaryl, substituted C₁₋C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where V is a substituted group, it is substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} and -NO₂,
wherein R⁵ and R^{5'} are each independently as defined above,
for the preparation of a medicament for the treatment of a solid cancer, myeloid disorders or adenoma.

26. A use as in claim 25, wherein the compound of formula I displays IC50s between 1nM and 10 µM as determined by an in-vitro raf kinase assay.

27. A. use according to claim 25, wherein the disease is a cancer dependent upon the ras protein signal transduction cascade and is treated by inhibiting raf kinase.

28. A. use according to claim 25, wherein the solid cancer is a carcinoma of the lungs, pancreas, rhyroid, bladder or colon.

29. A compound of formula 1 or 3 pharmaceutically acceptable salt 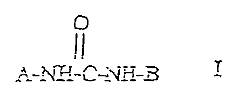 thereof
wherein A is 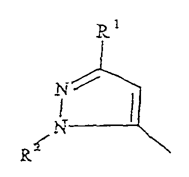 wherein R¹ is C₃-C₁₀ alkyl, C₃-C₁₀ cycloalkyl, up to per-halosubstituted C₁₋C₁₀ alkyl or up to per-halosubstituted C₃-C₁₀ cycloalkyl;
B is phenyl, 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 3-, 4-, 5-, 6-, 7- or 8-quinolinyl, or 1-, 3-, 4-, 5-, 6-, 7-, 8- isoquinolinyl,
substituted by -Y-Ar;
wherein Y is -O-, -S-, -N(R⁵)-, -(CH₂)-ₘ, -C(O)-, -CH(OH)- -(CH₂)ₘO-, NR⁵C(O)NR⁵R^{5'}-, -NR⁵C(O)-, -C(O)NR⁵, -O(CH₂)ₘ-, -(CH₂)ₘS-, -(CH₂)ₘN(R⁵)-, -O(CH₂)ₘ-, -CHX^{a}-, -CX^{a}₂-, -S-(CH₂)ₘ- or -N(R⁵)(CH₂)ₘ-,
m = 1-3, and X^{a} is halogen, and
Ar is phenyl or pyridinyl, optionally substituted by halogen up to per-halosubstitution and optionally substituted by Zₙ₁,
wherein n1 is 0 to 3 and each Z is independently -CN, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -C(O)R⁵, NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇₋C₂₄ alkaryl or substituted C₄-C₂₃ alkheteroaryl;
wherein if Z is a substituted group, it is substituted by the one or more substituents independently selected from the group consisting of-CN, -CO₂R⁵, -C(O)NR⁵R^{5'}, -OR⁵, -SR⁵, -NO², -NR⁵R^{5'}, -NR⁵C(O)R^{5'} and -NR⁵C(O)OR^{5'}, and
wherein R² is optionally substituted phenyl, pyridinyl, pyrimidinyl, pyrazinyl, pyridazinyl, naphthyl, quinolinyl, isoquinolinyl, phthalimidinyl, furyl, thienyl, pyrrolyl, imidazolyl, pyrazolyl, oxazolyl, isoxazolyl, thiazolyl, isothiazolyl, benzofuryl, benzothienyl, indolyl, benzopyrazolyl, benzoxazolyl, benzisoxazolyl, benzothiazolyl or benzisothiazolyl, wherein if R² is a substituted group, it is substituted by one or more substituents independently selected from the group consisting of halogen, up to per-halosubstitution, and Vₙ,
wherein n = 0-3 and each V is independently selected from the group consisting of -CN, -CO₂R⁵, -C(O)NR⁵R^{5'}, -OR⁵, -SR⁵, -NR⁵R^{5'}, -C(O)R⁵,-OC(O)NR⁵R^{5'}, -NR⁵C(O)OR^{5'}; -SO₂R⁵, -SOR⁵, -NR⁵C(O)R^{5'}, -NO₂, C₁-C₁₀ alkyl, C₃₋C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where if V is a substituted group, it is substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵,-NR⁵C(O)R^{5'}, -NR⁵C(O)OR^{5'} and -NO₂;
wherein R⁵ and R^{5'} are each independently as defined above.

30. A compound as in claim 29 wherein R² is phenyl, substituted phenyl, pyridinyl or substituted pyridinyl.

31. A compound as in claim 29 wherein Ar is phenyl or pyridinyl,

32. A compound as in claim 29 wherein Y is -O- or -S-.

33. A compound as in claim 29 wherein Z is Cl, F, CF₃, NO₂ or CN.

34. A compound as in claim 29 wherein B is optionally substituted diphenyl ether, diphenyl thioether, diphenyl amine, phenylpyridinyl ether, pyridinyhnethylphenyl or phenylpyridinylthioether,

35. A compound as in claim 1 wherein B is optionally substituted diphenyl ether, diphenyl thioether diphenyl amine, phenylpyridinyl ether, pyridinylmethylphenyl, phenylpyridinylthioether, phenylpyrimidinyl ether, phenylquinoline thioether, phenylnaphthyl ether, pyridinylnapthyl ether and pyridinylnaphthyl thioether.

36. A compound of formula I or a pharmaceutically acceptable salt thereof 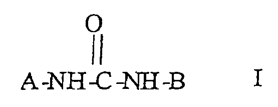 wherein
B is optionally substituted diphenyl ether, diphenyl thioether, phenylpyridinyl ether, pyridinylmethylphenyl, phenylquinoline thioether, phenylnaphthyl ether, pyridinylnapthyl ether, and pyridinyInaphthyl thioether; and
A is 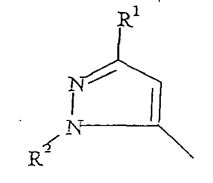 wherein
R¹ is C₃-C₁₀ alkyl, C₃-C₁₀ cycloalkyl, up to per-halosubstituted C₁-C₁₀ alkyl or up to per-halosubstituted C₃-C₁₀ cycloalkyl; and
R² is unsubstituted phenyl, unsubstituted pyridinyl, substituted phenyl or substituted pyridinyl; wherein if R² is a substituted group, it is substituted by one or more substituents independently selected from the group consisting of halogen, up to per-halosubstitution, and Vₙ,
wherein n = 0-3 and each V is independently selected from the group consisting of -CN, -CO₂R⁵, -C(O)NR⁵R^{5'}, -OR⁵, -SR⁵, -NR⁵R^{5'}, -C(O)R⁵, -OC(O) NR⁵R^{5'} -NR⁵C(O)OR^{5'}, -SO₂R⁵, -SOR⁵, -NR⁵C(O)R^{5'}, -NO₂, C₁-C₁₀ alkyl, C₃-C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where if V is a substituted group, it is substituted by one or more substituents independently selected from the group consisting of halogen, up to per-halosubstitution, -CN, -CO₂R⁵, -C(O)R⁵, -C(O)NR⁵R⁵, -NR⁵R^{5'}, -OR⁵, -SR⁵, -NR⁵C(O) R^{5'}, -NR⁵C(O)OR^{5'} and -NO₂;
wherein R⁵ and R^{5'} are each independently as defined above.

37. A compound of claim 1, wherein R² is phenyl or pyridinyl, optionally substituted by -NO₂, -OH, -NH₂, -OCH₃, -C₁-C₆ alkyl, up to perhalosubstituted C₁-C₆ alkyl, -NHC(O)-C₁-₆ alkyl, -SO₂-C₁-₆ alkyl or -O-C₁₋₆ alkyl, and optionally substituted once or twice by -F, -Cl or both.

38. A compound of claim 1 selected from
*N*-(1-phenyl-3-*tert*-butyl-5-pyrazolyl)-*N'*-(2,3-dichlorophenyl)urea;
*N*--(1-(4-aminophenyl)-3-*tert-*butyl-5-pyrazolyl)-*N'-*(2,3-dichlorophenyl)urea;
*N*--(1-(2-methylphenyl)-3-*tert*-butyl-5-pyrazolyl)-*N'*-(2,3-dichlorophenyl)urea;
*N*-(1-(2-pyridinyl)-3-*tert*-butyl-5-pyrazolyl)-*N'*-(2,3-dichlorophenyl)urea;
*N*-(1-(5-trifluoromethyl-2-pyrimidinyl)-3-*tert*-butyl-5-pyrazolyl)-*N'*-(2,3-dichlorophenyl)urea;
*N*-(1-(3-fluorophenyl)-3-*tert*-butyl-5-pyrazolyl)-*N'*-(2,3-dichlorophenyl)urea;
*N*-(1-(4-chlorophenyl)-3-*tert*-butyl-5-pyazolyl)-*N'*-(2,3-dichlorophenyl)urea;
*N*--(1-(4-methanesulfoxyphenyl)-3-*tert*-butyl-5-pyrazolyl)--*N'*-(2,3-dichlorophenyl) urea;
*N*--(1-(4-nitrophenyl)-3-*tert*-butyl-5-pyrazolyl)-*N'*-(2,3-dichlorophenyl)urea;
*N*--(1-(3-methoxyphenyl)-3-*tert*-butyl-5-pyrazolyl)-*N'*-(2,3-dichlorophenyl)urea;
*N*--(1-(3-trifluorophenyl)--3-*tert*-butyl-5-pyrazolyl)-*N'*-(2,3-dichlorophenyl)urea;
*N*--(1-(4-methoxhenyl)-3-*tert-*butyl-5-pyrazolyl)-*N'*-(2,3-dichlorophenyl)urea;
*N*--(1-(4-isopropylphenyl)-3-*tert*-butyl-5-pyrazolyl)-*N'*-(2,3-dichlorophenyl)urea;
*N*--(1-(3-aminophenyl)-3-*tert*-butyl-5-pyrazolyl)-*N'*-(2,3-dichlorophenyl)urea;
*N*--(1-(3-nitrophenyl)-3-*tert*-butyl-5-pyrazolyl)-*N'*-(2,3-dichlorophenyl)urea;
*N*-(1-phenyl-3-*tert*-butyl-5-pyrazolyl)-*N'*-(3-trifloro, 4-chlorophenyl)urea;
*N-*(1-phenyl-3-*tert*-butyl-5-pyrazolyl)-*N'*-(4-trifluorophenyl)urea;
*N*-(1-phenyl-3-tert-butyl-5-pyreolyl)-*N'*-(2,4-difluorphenyl)urea;
*N*-(1-phenyl-3-*tert*-butyl-5-pyrazolyl)-*N'*-(3-fluorophenyl)urea;
*N*-(1-phenyl-3-*tert*-butyl-5-pyrazolyl)-*N'*-(3-cyanohenyl)urea;
*N*-(1-Phenyl-3-*tert*-butyl-5-pyrazolyl)-*N'*-(4-nitrophenyl)urea;
*N*-(1-(3-acetamidophenyl)-3*-tert-*butyl-5-pyrazolyl)*-N'*-(4-phenoxyphenyl)urea;
*N*-(1-(3-aminophenyl)-3-*tert*-butyl-5-pyrazolyl)-*N'*-(4-phenoxyphenyl)urea;
*N*-(1-(3-nitrophenyl)-3-*tert*-butyl-5-pyrazolyl)-*N'*-(4-phenoxyphenyl)urea;
*N*-(1-phenyl-3-*tert*-butyl-5-pyrazolyl)-*N'*-(4-(4-pyridinylmethyl)phenyl)urea;
*N*-(1-(4-pyridinyl)-3-*tert*-butyl-5-pyrazolyl)*-N'-*(4-(4- pyridinylmethyl)phenyl) urea;
*N*-(1-(2,5-dichlorophenyl)-3-*tert*-butyl-5-pyrazolyl)-*N'*-(4-(4-pyridinylmethyl)phenyl)urea;
*N*-(1-(4-fluorophenyl)-3-*tert*-butyl-5-pyrazolyl)-*N'*-(4-(4-pyridinylmethyl)phenyl)urea;
*N*-(1-(2-methylphenyl)-3-*tert*-butyl-5-pyrazolyl)-*N'*-(4-(4-pyridinylmethyl)phenyl)urea;
*N*-(1-(3-fluorophenyl)-3-*tert*-butyl-5-pyrazolyl)-*N'*-(4-(4-pyridinylmethyl)phenyl)urea;
*N*-(1-(4-methanesulfoxyphenyl)-3-*tert*-butyl-5-pyrazolyl)-*N'*-(4-(4-pyridinylmethyl)phenyl)urea;
*N*-(1-(4-nitrophenyl)-3-*tert*-butyl-5-pyrazolyl)-*N'*-(4-(4-pyridiaylmethyl)phenyl)urea;
*N*-(1-(3-methoxyphenyl)-3-*tert*-butyl-5-pyrazolyl)-*N'*-(4-(4-pyridinylmethyl)phenyl)urea;
*N*-(1-(3-aminophenyl)-3-*tert*-butyl-5-pyrazolyl)*N'*-(4-(4-pyridinylmethyl)phenyl)urea;
*N*-(1-(3-nitrophenyl)-3-*tert*-butyl-5-pyrazolyl)-*N'*-(4-(4-pyridinylmethyl)phenyl)urea;
*N*-(1-(3-aminophenyl)-3-*tert*-butyl-5-pyrazolyl)-*N'*-(4-(4-pyridinylthio)phenyl)urea;
*N*-(1-(3-nitrophenyl)-3-*tert*-butyl-5 pyrazolyl)-*N'*-(4-(4- pyridinylthio)phenyl)urea;
*N*--(1-(3-(2,3-dichlorophenoxyacylamino)phenylmethyl)-3-*tert*-butyl-5-pyrazolyl)-*N*'-(2,3-dichlorophenyl)urea;
*N*-(1-(3-hydroxphenylmethyl)-3-*tert*-butyl-5-pyrazolyl)-*N'*-(2,3- dichlorophenyl) urea;
*N*-(2-phenyl-5-*tert*-butyl-3-thienyl)-*N'*-(2,3-dichlorophenyl)urea; and
*N*-(2-(4-pyridinyl)-5-*tert*-butyl-3-furyl)-*N'*-(2,3-dichlorophenyl)urea.

39. A compound of claim 1, wherein R¹ is t-butyl and R² is unsubstituted or substituted phenyl.

40. A compound of claim 3, wherein Q is phenyl, Q¹ is phenyl or pyridinyl, Y is -O-, -S- or -CH₂-, and Z is Cl, F, NO₂ or CF₃.

## Patentansprüche

1. Verbindung der Formel I und pharmazeutisch verträgliche Salze davon 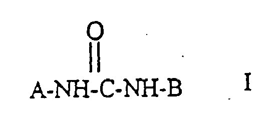 wobei A ein Heteroaryl ausgewählt aus der Gruppe bestehend aus 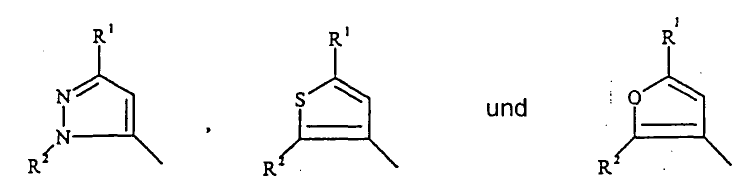 ist, wobei R¹ ausgewählt ist aus der Gruppe bestehend aus C₃-C₁₀-Alkyl, C₃-C₁₀₋Cycloalkyl, bis zu perhalosubstituiertem C₁-C₁₀-Alkyl und bis zu perhalosubstituiertem C₃-C₁₀-Cycloalkyl;
B ein bis zu tricyclischer Aryl- oder Heteroarylanteil von bis zu 12 Kohlenstoffatomen mit wenigstens einer 5- oder 6-gliedrigen aromatischen Struktur, die 0-4 Mitglieder aus der Gruppe bestehend aus Stickstoff, Sauerstoff und Schwefel enthält, ist, der durch -Y-Ar und gegebenenfalls durch einen oder mehrere Substituenten unabhängig ausgewählt aus der Gruppe bestehend aus Halogen, bis zur Perhalosubstitution und Xₙ substituiert ist,
wobei n 0-3 ist und jeder X unabhängig ausgewählt ist aus der Gruppe bestehend aus -CN, -CO₂R⁵, -C(O)NR⁵R^{5'}, -C(O)R⁵, -NO₂ -OR⁵, -SR⁵, -NR⁵R^{5'}, -NR⁵C(O)OR^{5'}, -NR⁵C(O)R^{5'},C₁-C₁₀-Alkyl, C₂-C₁₀-Alkenyl, C₁-C₁₀-Alkoxy, C₃-C₁₀₋Cycloalkyl, C₆-C₁₄-Aryl, C₇-C₂₄-Alkaryl, C₃-C₁₃-Heteroaryl, C₄-C₂₃-Alkheteroaryl, substituiertem C₁-C₁₀-Alkyl, substituiertem C₂-C₁₀-Alkenyl, substituiertem C₁-C₁₀₋Alkoxyl, substituiertem C₃-C₁₀-Cycloalkyl, substituiertem C₄-C₂₃-Alkheteroaryl;
wenn X eine substituierte Gruppe ist, sie durch einen oder mehrere Substituenten unabhängig ausgewählt sind aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen, bis zur Perhalosubstitution substituiert ist;
wobei R⁵ und R^{5'} unabhängig ausgewählt sind aus H, C₁-C₁₀-Alkyl, C₂-C₁₀₋Alkenyl, C₃-C₁₀-Cycloalkyl, C₆-C₁₄-Aryl, C₃-C₁₃-Heteroaryl, C₇-C₂₄-Alkaryl, C₄-C₂₃₋Alkheteroaryl, bis zu perhalosubstituiertem C₁-C₁₀-Alkyl, bis zu perhalosubstituiertem C₂-C₁₀-Alkenyl, bis zu perhalosubstituiertem C₃-C₁₀₋Cycloalkyl, bis zu perhalosubstituiertem C₆-C₁₄-Aryl und bis zu perhalosubstituiertem C₃-C₁₃-Heteroaryl,
wobei Y -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵, -O(CH₂)ₘ-, -(CH₂)ₘS-, -(CH₂)ₘN(R⁵)-, -O(CH₂)ₘ-, -CHX^{a}-, -CX^{a}₂-, -S-(CH₂)ₘ oder -N(R⁵)(CH₂)ₘ- ist,
m = 1-3 ist und X^{a} Halogen ist; und
Ar eine 5- bis 10-gliedrige aromatische Struktur ist, die 0-2 Mitglieder aus der Gruppe bestehend aus Stickstoff-, Sauerstoff- und Schwefelatomen enthält, die unsubstituiert oder substituiert ist durch Halogen, bis zur Perhalosubstitution und gegebenenfalls substituiert ist durch Zₙ₁, wobei n1 0 bis 3 ist und jeder Z unabhängig ausgewählt ist aus der Gruppe bestehend aus -CN, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -C(O)R⁵, NR⁵C(O)R^{5'}, C₁-C₁₀-Alkyl, C₃-C₁₀-Cycloalkyl, C₆-C₁₄-Aryl, C₃-C₁₃₋Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃-Alkheteroaryl;
wobei, wenn Z eine substituierte Gruppe ist, sie durch einen oder mehrere Substituenten unabhängig ausgewählt sind aus der Gruppe bestehend aus -CN, -CO₂R⁵, -C(O)NR⁵R^{5'}, -OR⁵, -SR⁵, -NO₂, -NR⁵R^{5'}, -NR⁵R^{5'}, -NR⁵C(O)R^{5'} und -NR⁵C(O)OR^{5'} substituiert ist, und
wobei R² C₆-C₁₄-Aryl, C₃-C₁₄-Heteroaryl, substituiertes C₆-C₁₄-Aryl oder substituiertes C₃-C₁₄-Heteroaryl ist,
wobei, wenn R² eine substituierte Gruppe ist, sie durch einen oder mehrere Substituenten unabhängig ausgewählt sind aus der Gruppe bestehend aus Halogen, bis zur Perhalosubstitution und Vₙ substituiert ist,
wobei n = 0-3 ist und jeder V unabhängig ausgewählt ist aus der Gruppe bestehend aus -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₄₋Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇₋C₂₄-Alkaryl und substituiertem C₄-C₂₄-Alkheteroaryl,
wobei, wenn V eine substituierte Gruppe ist, sie durch einen oder mehrere Substituenten unabhängig ausgewählt aus der Gruppe bestehend aus Halogen, bis zur Perhalosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} und -NO₂ substituiert ist;
wobei R⁵ und R^{5'} jeweils unabhängig wie oben definiert sind.

2. Verbindung nach Anspruch 1, wobei R² substituiertes oder unsubstituiertes Phenyl oder Pyridinyl ist, und die Substituenten für R² ausgewählt sind aus der Gruppe bestehend aus Halogen, bis zur Perhalosubstitution und Vₙ, wobei n = 0-3 ist, und jeder V unabhängig ausgewählt ist aus der Gruppe bestehend aus substituiertem oder unsubstituiertem C₁-C₆-Alkyl, C₃-C₁₀-Cycloalkyl, C₆-C₁₀-Aryl, -NO₂, -NH₂, -C(O)-C₁₋₆-Alkyl, -C(O)N-(C₁₋₆-Alkyl)₂, -C(O)NH-C₁₋₆-Alkyl, -O-C₁₋₆₋Alkyl, -NHC(O)H, -NHC(O)OH, -N(C₁₋₆-Alkyl)C(O)- C₁₋₆-Alkyl, -N-(C₁₋₆-Alkyl)C(O)-C₁₋₆-Alkyl, -NHC(O)-C₁₋₆-Alkyl, -OC(O)NH-C₆₋₁₄-Aryl, -NHC(O)O-C₁₋₆-Alkyl, -S(O)-C₁₋₆-Alkyl und -SO₂-C₁₋₆-Alkyl,
wobei, wenn V eine subsituierte Gruppe ist, sie durch einen oder mehrere Halogene, bis zur Perhalosubstitution substituiert ist.

3. Verbindung nach Anspruch 1, wobei B 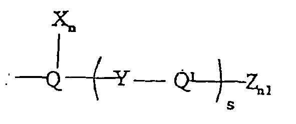 ist,
wobei
Y ausgewählt ist der Gruppe bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q eine sechsgliedrige aromatische Struktur ist, die 0-2 Stickstoffe enthält, die substituiert oder unsubstituiert durch Halogen, bis zur Perhalosubstitution ist;
Q¹ eine mono- oder bicyclische aromatische Struktur aus 3 bis 10 Kohlenstoffatomen und 0-4 Mitgliedern der Gruppe bestehend aus N, O und S ist, die unsubstituiert oder substituiert durch Halogen, bis zur Perhalosubstitution ist,
n 0 ist, Z und n1 wie in Anspruch 1 definiert sind und s = 1 ist.

4. Verbindung nach Anspruch 3, wobei
Q Phenyl oder Pyridinyl ist, das substituiert oder unsubstituiert durch Halogen, bis zur Perhalosubstitution ist,
Q¹ ausgewählt ist aus der Gruppe bestehend aus Phenyl, Pyridinyl, Naphthyl, Pyrimidinyl, Chinolin, Isochinolin, Imidazol und Benzothiazolyl, das substituiert oder unsubstituiert durch Halogen, bis zur Perhalosubstitution ist, und
Z ausgewählt ist aus der Gruppe bestehend aus -R⁶, -OR⁶ und -NHR⁷, wobei R⁶ Wasserstoff, C₁-C₁₀-Alkyl oder C₃-C₁₀-Cycloalkyl ist und R⁷ ausgewählt ist aus der Gruppe bestehend aus Wasserstoff, C₃-C₁₀-Alkyl, C₃-C₆-Cycloalkyl und C₆₋C₁₀-Aryl, wobei R⁶ und R⁷ durch Halogen oder bis zur Perhalosubstitution substituiert sein können.

5. Verbindung nach Anspruch 1, wobei R¹ t-Butyl ist und R² unsubstituiertes oder substituiertes Phenyl ist.

6. Verbindung nach Anspruch 4, wobei Q Phenyl ist, Q¹ Phenyl oder Pyridinyl ist, Y -O-, -S- oder -CH₂- ist und X und Z unabhängig Cl, F, NO₂ oder CF₃ sind.

7. Verbindung nach Anspruch 6, wobei R¹ t-Butyl ist.

8. Verbindung nach Anspruch 1, der Formel 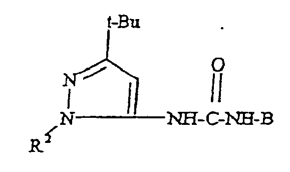 wobei B und R² wie in Anspruch 1 definiert sind.

9. Verbindung nach Anspruch 8, wobei R² ausgewählt ist aus substituierten oder unsubstituierten Mitgliedern der Gruppe bestehend aus Phenyl, Pyridinyl, wobei, wenn R² eine substituierte Gruppe ist, sie durch einen oder mehrere Substituenten ausgewählt aus der Gruppe bestehend aus Halogen und Wₙ substituiert ist, wobei n = 0-3 ist und W ausgewählt ist aus der Gruppe bestehend aus -NO₂, -C₁₋₃-Alkyl, -NH(O)CH₃, -CF₃, -OCH₃, -F, -Cl, -NH₂, -OC(O)NH-, bis zu perhalosubstituiertem Phenyl, -SO₂CH₃, Pyridinyl, Phenyl, bis zu perhalosubstituiertem Phenyl und C₁-C₆-Alkyl-substituiertem Phenyl.

10. Verbindung nach Anspruch 1, der Formel 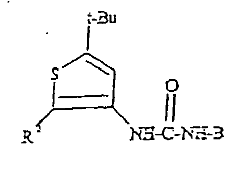 wobei B und R² wie in Anspruch 1 definiert sind.

11. Verbindung nach Anspruch 10, wobei R² ausgewählt ist aus substituierten und unsubstituierten Mitgliedern der Gruppe bestehend aus Phenyl und Pyridinyl, wobei, wenn R² eine substituierte Gruppe ist, sie durch einen oder mehrere Substituenten ausgewählt aus der Gruppe bestehend aus Halogen und Wₙ substituiert ist, wobei n = 0-3 ist und W ausgewählt ist aus der Gruppe bestehend aus -NO₂, -C₁₋₃-Alkyl, -NH(O)CH₃, -CF₃, -OCH₃, -F, -Cl, -NH₂, -SO₂CH₃, Pyridinyl, Phenyl, bis zu perhalosubstituiertem Phenyl und C₁-C₆-Alkyl-substituiertem Phenyl.

12. Verbindung nach Anspruch 1, der Formel 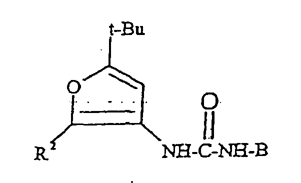 wobei B und R² wie in Anspruch 1 definiert sind.

13. Verbindung nach Anspruch 12, wobei R² ausgewählt ist aus substituierten und unsubstituierten Mitgliedern der Gruppe bestehend aus Phenyl und Pyridinyl, wobei, wenn R² eine substituierte Gruppe ist, sie durch einen oder mehrere Substituenten ausgewählt aus der Gruppe bestehend aus Halogen und Wₙ substituiert ist, wobei n = 0-3 ist und W ausgewählt ist aus der Gruppe bestehend aus -NO₂, -C₁₋₃-Alkyl, -NH(O)CH₃, -CF₃, -OCH₃, -F, -Cl, -NH₂, -SO₂CH₃, Pyridinyl, Phenyl, bis zu perhalosubstituiertem Phenyl und C₁-C₆-Alkyl-substituiertem Phenyl.

14. Verwendung einer Verbindung der Formel I oder eines pharmazeutisch verträglichen Salzes davon 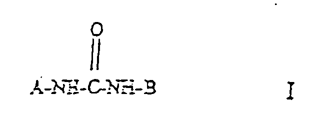 wobei A ein Heteroaryl ausgewählt aus der Gruppe bestehend aus 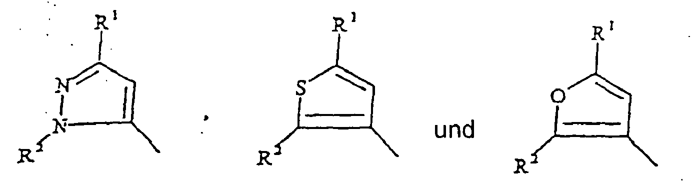 ist, wobei R¹ ausgewählt ist aus der Gruppe bestehend aus C₃-C₁₀-Alkyl, C₃-C₁₀₋Cycloalkyl, bis zu perhalosubstituiertem C₁-C₁₀-Alkyl und bis zu perhalosubstituiertem C₃-C₁₀-Cycloalkyl;
B ein substituierter oder unsubstituierter, bis zu tricyclischer Aryl- oder Heteroarylanteil von bis zu 12 Kohlenstoffatomen mit wenigstens einer 5- oder 6-gliedrigen aromatischen Struktur, enthaltend 0-4 Mitglieder der Gruppe bestehend aus Stickstoff, Sauerstoff und Schwefel, ist, wobei, wenn B eine substituierte Gruppe ist, sie durch einen oder mehrere Substituenten unabhängig ausgewählt aus der Gruppe bestehend aus Halogen, bis zur Perhalosubstitution und Xₙ substituiert ist,
wobei n 0-3 ist und jeder X unabhängig ausgewählt ist aus der Gruppe bestehend aus -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂₋₁₀-Alkenyl, C₁₋₁₀-Alkoxy, C₃-C₁₀₋Cycloalkyl, C₆-C₁₄-Aryl, C₇-C₂₄-Alkaryl, C₃-C₁₃-Heteroaryl, C₄-C₂₃-Alkheteroaryl, substituiertem C₁-C₁₀-Alkyl, substituiertem C₂₋₁₀-Alkenyl, substituiertem C₁₋₁₀₋Alkoxy, substituiertem C₃-C₁₀-Cycloalkyl, substituiertem C₄-C₂₃-Alkheteroaryl und
-Y-Ar;
wobei, wenn X eine substituierte Gruppe ist, sie durch einen oder mehrere Substituenten unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen, bis zur Perhalosubstitution substituiert ist,
wobei R⁵ und R^{5'} unabhängig ausgewählt sind aus H, C₁-C₁₀-Alkyl, C₂₋₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃₋Alkheteroaryl, bis zu perhalosubstituiertem C₁-C₁₀-Alkyl, bis zu perhalosubstituiertem C₂₋₁₀-Alkenyl, bis zu perhalosubstituiertem C₃-C₁₀₋Cycloalkyl, bis zu perhalosubstituiertem C₆-C₁₄-Aryl und bis zu perhalosubstituiertem C₃-C₁₃-Heteroaryl, wobei Y -O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- oder -N(R⁵)(CH₂)ₘ-,
m = 1-3 ist und X^{a} Halogen ist; und
Ar eine 5- oder 6-gliedrige aromatische Struktur, enthaltend 0-2 Mitglieder der Gruppe bestehend aus Stickstoff-, Sauerstoff- und Schwefelatomen, ist, die unsubstituiert oder substituiert durch Halogen, bis zur Perhalosubstitution und gegebenenfalls substituiert durch Zₙ₁ ist, wobei n1 0 bis 3 ist und jeder Z unabhängig ausgewählt ist aus der Gruppe bestehend aus -CN, -C(O)R⁵, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃₋Alkheteroaryl;
wobei, wenn Z eine substituierte Gruppe ist, sie durch einen oder mehrere Substituenten unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'} und -NR⁵C(O)OR^{5'} substituiert ist, und
wobei R² C₆-C₁₄-Aryl, C₃-C₁₄-Heteroaryl, substituiertes C₆-C₁₄-Aryl oder substituiertes C₃-C₁₄-Heteroaryl ist,
wobei, wenn R^{2'} eine substituierte Gruppe ist, sie durch einen oder mehrere Substituenten unabhängig ausgewählt aus der Gruppe bestehend aus Halogen, bis zur Perhalosubstitution und Vₙ substituiert ist,
wobei n = 0-3 ist und jeder V unabhängig ausgewählt ist aus der Gruppe bestehend aus -CN, -CO₂R⁵, -C(O)NR⁵R^{5'}, -OR⁵, -SR⁵, -NR⁵R^{5'}, -OC(O)NR⁵R^{5'}, -NR⁵C(O)OR^{5'}, -NR⁵C(O)OR^{5'}, -SO₂R⁵, -SOR⁵, -NR⁵C(O)R^{5'}, -NO₂, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₄₋Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇₋C₂₄-Alkaryl und substituiertem C₄-C₂₄-Alkheteroaryl,
wenn V eine substituierte Gruppe ist, sie durch einen oder mehrere Substituenten unabhängig ausgewählt aus der Gruppe bestehend aus Halogen, bis zur Perhalosubstitution, -CN, -CO₂R⁵_{,} -C(O)R⁵, -C(O)NR⁵R^{5'}, -NR⁵R^{5'}, -OR⁵, -SR⁵, -NR⁵C(O)R^{5'}, -NR⁵C(O)OR^{5'} und -NO₂ substituiert ist,
wobei R⁵ und R^{5'} jeweils unabhängig wie oben definiert sind,
zur Herstellung eines Medikaments zur Behandlung von Krankheiten, die durch Raf-Kinase vermittelt werden.

15. Verwendung nach Anspruch 14, wobei R² ausgewählt ist aus substituierten und unsubstituierten Mitgliedern der Gruppe bestehend aus Phenyl und Pyridinyl, und die Substituenten für R² ausgewählt sind aus der Gruppe bestehend aus Halogen, bis zur Perhalosubstitution und Vₙ, wobei n = 0-3 ist und jeder V unabhängig ausgewählt ist aus der Gruppe bestehend aus substituiertem und unsubstituiertem C₁-C₆-Alkyl, C₃-C₁₀-Cycloalkyl, C₆-C₁₀-Aryl, -NO₂, -NH₂, -C(O)-C₁₋₆-Alkyl, -C(O)N-(C₁₋₆-Alkyl)₂, -C(O)NH-C₁₋₆-Alkyl, -O-C₁₋₆-Alkyl, -NHC(O)H, -NHC(O)OH, -N(C₁₋₆-Alkyl)C(O)-C₁₋₆-Alkyl, -N-(C₁₋₆-Alkyl)C(O)-C₁₋₆-Alkyl, -NHC(O)-C₁₋₆-Alkyl, -NHC(O)O-C₁₋₆-Alkyl, -S(O)-C₁₋₆-Alkyl und -SO₂-C₁₋₆-Alkyl,
wobei, wenn V eine substituierte Gruppe ist, sie durch einen oder mehrere Halogene, bis zur Perhalosubstitution substituiert ist.

16. Verwendung nach Anspruch 14, wobei B eine bis zu tricyclische aromatische Ringstruktur ausgewählt aus der Gruppe bestehend aus 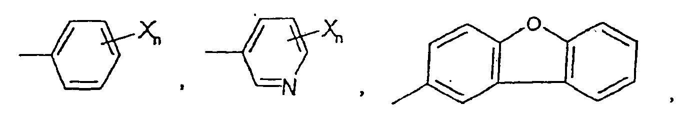 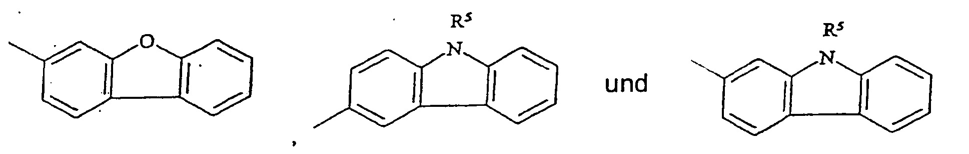 die substituiert oder unsubstituiert durch Halogen, bis zur Perhalosubstitution ist, ist, und
wobei
n = 0-3 ist und
jeder X unabhängig ausgewählt ist aus der Gruppe bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂₋₁₀-Alkenyl, C₁₋₁₀-Alkoxy, C₃-C₁₀-Cycloalkyl, C₆-C₁₄-Aryl, C₇-C₂₄₋Alkaryl, C₃-C₁₃-Heteroaryl, C₄-C₂₃-Alkheteroaryl und substituiertem C₁-C₁₀-Alkyl, substituiertem C₂-C₁₀-Alkenyl, substituiertem C₁₋₁₀-Alkoxy, substituiertem C₃-C₁₀₋Cycloalkyl, substituiertem C₄-C₂₃-Alkheteroaryl und -Y-Ar;
wobei, wenn X eine substituierte Gruppe ist, sie durch einen oder mehrere Substituenten unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen, bis zur Perhalosubstitution substituiert ist;
wobei R⁵ und R^{5'} unabhängig ausgewählt sind aus H, C₁-C₁₀-Alkyl, C₂-₁₀-Alkenyl, C₃-C₁₀-Cycloalkyl, C₄-C₁₄-Aryl, C₃-C₁₃-Heteroaryl, C₇-C₂₄-Alkaryl, C₄-C₂₃₋Alkheteroalkyl, bis zu perhalosubstituiertem C₁-C₁₀-Alkyl, bis zu perhalosubstituiertem C₂₋₁₀-Alkenyl, bis zu perhalosubstituiertem C₃-C₁₀₋Cycloalkyl, bis zu perhalosubstituiertem C₆-C₁₄-Aryl und bis zu perhalosubstituiertem C₃-C₁₃-Heteroaryl,
wobei Y -O-, -S-, -N(R⁵)-, -(CH₂)-ₘ, -C(O)-, -CH(OH)-, -(CH₂)ₘO-, -NR₅C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}-, -CX^{a}₂-, -S-(CH₂)ₘ- und -N(R⁵)(CH₂)ₘ- ist,
m = 1-3 ist und X^{a} Halogen ist; und
Ar eine 5- bis 10-gliedrige aromatische Struktur, enthaltend 0-2 Mitglieder der Gruppe bestehend aus Stickstoff, Sauerstoff und Schwefel, ist, die unsubstituiert oder substituiert durch Halogen, bis zur Perhalosubstitution und gegebenenfalls substituiert durch Zₙ₁ ist, wobei n1 0 bis 3 ist und jeder Z unabhängig ausgewählt ist aus der Gruppe bestehend aus -CN, -C(O)R⁵, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₃-C₁₀₋Cycloalkyl, C₆-C₁₄-Aryl, C₃-C₁₃-Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇₋C₂₄-Alkaryl und substituiertem C₄-C₂₃-Alkheteroaryl; wobei, wenn Z eine substituierte Gruppe ist, sie durch einen oder mehrere Substituenten unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'} und -NR⁵C(O)OR^{5'} substituiert ist.

17. Verwendung nach Anspruch 14, wobei B 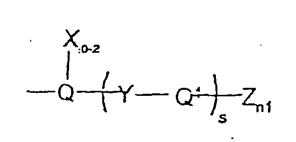 ist,
wobei
Y ausgewählt ist aus der Gruppe bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q eine sechsgliedrige aromatische Struktur, enthaltend 0-2 Stickstoffe, ist, die substituiert oder unsubstituiert durch Halogen, bis zur Perhalosubstitution ist;
Q¹ eine mono- oder bicyclische aromatische Struktur aus 5-10 Mitgliedern mit 3-10 Kohlenstoffatomen und 0-2 Mitgliedern der Gruppe bestehend aus N, O und S ist, die unsubstituiert oder substituiert durch Halogen, bis zur Perhalosubstitution ist,
X, Z und n1 wie in Anspruch 15 definiert sind und s = 0 oder 1 ist.

18. Verwendung nach Anspruch 17, wobei
Q Phenyl oder Pyridinyl ist, das substituiert oder unsubstituiert durch Halogen, bis zur Perhalosubstitution ist,
Q¹ ausgewählt ist aus der Gruppe bestehend aus Phenyl, Pyridinyl, Naphthyl, Pyrimidinyl, Chinolin, Isochinolin, Imidazol und Benzothiazolyl, das substituiert oder unsubstituiert durch Halogen, bis zur Perhalosubstitution ist,
Z und X unabhängig ausgewählt sind aus der Gruppe bestehend aus -R⁶; -OR⁶ und -NHR⁷, wobei R⁶ Wasserstoff, C₁-C₁₀-Alkyl oder C₃-C₁₀-Cycloalkyl ist und R⁷ ausgewählt ist aus der Gruppe bestehend aus Wasserstoff, C₃-C₁₀-Alkyl, C₃-C₆₋Cycloalkyl und C₆-C₁₀-Aryl, wobei R⁶ und R⁷ substituiert durch Halogen oder bis zur Perhalosubstitutiori substituiert sein können.

19. Verfahren nach Anspruch 17, wobei Q Phenyl ist, Q¹ Phenyl oder Pyridinyl ist, Y -O-, -S- oder -CH₂- ist und X und Z unabhängig Cl, F, NO₂ oder CF₃ sind.

20. Verfahren nach Anspruch 14, umfassend das Verabreichen einer Verbindung einer der Formeln 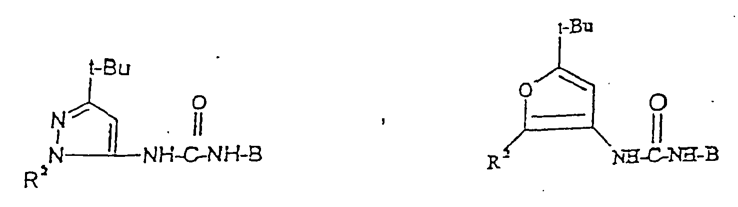 oder 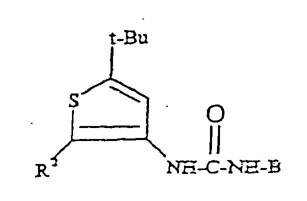 wobei B und R² wie in Anspruch 15 definiert sind.

21. Verwendung nach Anspruch 20, wobei R² ausgewählt ist aus substituierten und unsubstituierten Mitgliedern der Gruppe bestehend aus Phenyl oder Pyridinyl, wobei, wenn R² eine substituierte Gruppe ist, sie durch einen oder mehrere Substituenten ausgewählt aus der Gruppe bestehend aus Halogen und Wₙ substituiert ist, wobei n = 0-3 ist und W ausgewählt ist aus der Gruppe bestehend aus -NO₂, -C₁₋₃-Alkyl, -NH(O)CH₃, -CF₃, -OCH₃, -F, -Cl, -NH₂, -OC(O)NH-, bis zu perhalosubstituiertem Phenyl, -SO₂CH₃, Pyridinyl, Phenyl, bis zu perhalosubstituiertem Phenyl und C₁-C₆-Alkyl-substituiertem Phenyl.

22. Verwendung nach Anspruch 14, umfassend das Verabreichen einer Menge der Verbindung der Formel I, die wirksam ist, um Raf-Kinase zu inhibieren.

23. Pharmazeutische Zusammensetzung, umfassend eine Verbindung nach Anspruch 1 und einen pharmazeutisch verträglichen Träger.

24. Pharmazeutische Zusammensetzung, umfassend eine Verbindung nach Anspruch 2 und einen pharmazeutisch verträglichen Träger.

25. Verwendung einer Verbindung der Formel I oder eines pharmazeutisch verträglichen Salzes davon an einen Wirt, der diese benötigt 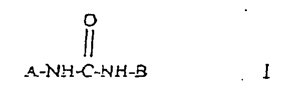 wobei A 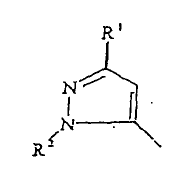 ist,
wobei R¹ ausgewählt ist aus der Gruppe bestehend aus C₃-C₁₀-Alkyl, C₃-C₁₀₋Cycloalkyl, bis zu perhalosubstituiertem C₁-C₁₀-Alkyl und bis zu perhalosubstituiertem C₃-C₁₀-Cycloalkyl;
B ein substituierter oder unsubstituierter, bis zu tricyclischer Aryl- oder Heteroarylanteil von bis zu 12 Kohlenstoffatomen mit wenigstens einer 5- oder 6-gliedrigen aromatischen Struktur, enthaltend 0-4 Mitglieder der Gruppe bestehend aus Stickstoff, Sauerstoff und Schwefel, ist, wobei, wenn B eine substituierte Gruppe ist, sie durch einen oder mehrere Substituenten unabhängig ausgewählt aus der Gruppe bestehend aus Halogen, bis zur Perhalosubstitution und Xₙ substituiert ist,
wobei n 0-3 ist und jeder X unabhängig ausgewählt ist aus der Gruppe bestehend aus -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂₋₁₀-Alkenyl, C₁₋₁₀-Alkoxy, C₃-C₁₀₋Cycloalkyl, C₆-C₁₄-Aryl, C₇-C₂₄-Alkaryl, C₃-C₁₃-Heteroaryl, C₄-C₂₃-Alkheteroaryl, substituiertem C₁-C₁₀-Alkyl, substituiertem C₂₋₁₀-Alkenyl, substituiertem C₁₋₁₀₋Alkoxy, substituiertem C₃-C₁₀-Cycloalkyl, bis zu perhalosubstituiertem C₆-C₁₄₋Aryl, bis zu perhalosubstituiertem C₃-C₁₃-Heteroaryl, substituiertem C₄-C₂₃₋Alkheteroaryl und -Y-Ar;
wobei, wenn X eine substituierte Gruppe ist, sie durch einen oder mehrere Substituenten unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen, bis zur Perhalosubstitution substituiert ist,
wobei R⁵ und R^{5'} unabhängig ausgewählt sind aus H, C₁-C₁₀-Alkenyl, C₂₋₁₀₋Alkenyl, C₃-C₁₀-Cycloalkyl, C₆-C₁₄-Aryl, C₃-C₁₃-Heteroaryl, C₇-C₂₄-Alkaryl, C₄-C₂₃₋Alkheteroaryl, bis zu perhalosubstituiertem C₁-C₁₀-Alkyl, bis zu perhalosubstituiertem C₂₋₁₀-Alkenyl, bis zu perhalosubstituiertem C₃-C₁₀₋Cycloalkyl, bis zu perhalosubstituiertem C₆-C₁₄-Aryl und bis zu perhalosubstituiertem C₃-C₁₃-Heteroaryl, wobei Y -O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- oder -N(R⁵)(CH₂)ₘ- ist,
m = 1-3 ist und X^{a} Halogen ist; und
Ar eine 5- oder 6-gliedrige aromatische Struktur enthaltend 0-2 Mitglieder der Gruppe bestehend aus Stickstoff-, Sauerstoff- und Schwefelatomen, ist, die unsubstituiert oder substituiert durch Halogen, bis zur Perhalosubstitution und gegebenenfalls substituiert durch Zₙ₁ ist, wobei n1 0 bis 3 ist und jeder Z unabhängig ausgewählt ist aus der Gruppe bestehend aus -CN, -C(O)R⁵, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₃-C₁₀-Cycloalkyl, C₆-C₁₄-Aryl, C₃-C₁₃-Heteroaryl, C₇₋C₂₄-Alkaryl, C₄-C₂₃-Alkheteroaryl, substituiertem C₁-C₁₀-Alkyl, substituiertem C₃₋C₁₀-Cycloalkyl, substituiertem C₇-C₂₄-Alkaryl oder substituiertem C₄-C₂₃₋Alkheteroaryl;
wobei, wenn Z eine substituierte Gruppe ist, sie durch einen oder mehrere Substituenten unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'} und -NR⁵C(O)OR^{5'} substituiert ist, und
wobei R² C₆-C₁₄-Aryl, C₃-C₁₄-Heteroaryl, substituiertes C₆-C₁₄-Aryl oder substituiertes C₃-C₁₄-Heteroaryl ist,
wobei, wenn R² eine substituierte Gruppe ist, sie durch einen oder mehrere Substituenten unabhängig ausgewählt aus der Gruppe bestehend aus Halogen, bis zur Perhalosubstitution und Vₙ substituiert ist,
wobei n = 0-3 ist und jeder V unabhängig ausgewählt ist aus der Gruppe bestehend aus -CN, -CO₂R⁵, -C(O)NR⁵R^{5'}, -OR⁵, -SR⁵, -NR⁵R^{5'}, -OC(O)NR⁵R^{5'}, -C(O)R⁵, -NR⁵C(O)OR^{5'}, -SO₂R⁵, -SOR⁵, -NR⁵C(O)R^{5'}, -NO₂, C₁-C₁₀-Alkyl, C₃₋C₁₀-Cycloalkyl, C₆-C₁₄-Aryl, C₃-C₁₃-Heteroaryl, C₇-C₂₄-Alkaryl, C₄-C₂₄₋Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇₋C₂₄-Alkaryl und substituiertem C₄-C₂₄-Alkheteroaryl,
wenn V eine substituierte Gruppe ist, sie durch einen oder mehrere Substituenten unabhängig ausgewählt aus der Gruppe bestehend aus Halogen, bis zur Perhalosubstitution, -CN, -CO₂R⁵, -C(O)R⁵, -C(O)NR⁵R^{5'}, -NR⁵R^{5'} -OR⁵, -SR⁵, -NR⁵C(O)R^{5'}, -NR⁵C(O)OR^{5'} und -NO₂ substituiert ist,
wobei R⁵ und R^{5'} jeweils unabhängig wie oben definiert sind,
zur Herstellung eines Medikaments zur Behandlung eines soliden Krebses, von myeloiden Erkrankungen oder eines Adenoma.

26. Verwendung nach Anspruch 25, wobei die Verbindung der Formel I IC50s zwischen 1 nM und 10 µM zeigt, wie bestimmt durch einen *in vitro* Raf-Kinase-Assay.

27. Verwendung nach Anspruch 25, wobei die Erkrankung ein von der Ras-Protein-Signaltransduktionskaskade abhängiger Krebs ist und durch eine Inhibierung der Raf-Kinase behandelt wird.

28. Verwendung nach Anspruch 25, wobei der solide Krebs ein Karzinom der Lungen, des Pankreas, der Schilddrüse, der Harnblase oder des Colons ist.

29. Verbindung der Formel I oder eines pharmazeutisch verträglichen Salzes davon 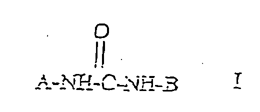 wobei A 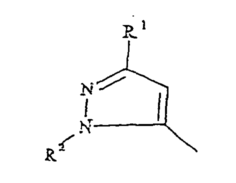 ist,
wobei R¹ ein C₃-C₁₀-Alkyl, C₃-C₁₀-Cycloalkyl, ein bis zu perhalosubstituiertes C₁₋C₁₀-Alkyl oder ein bis zu perhalosubstituiertes C₃-C₁₀-Cycloalkyl ist;
B Phenyl, 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 1-, 3-, 4-, 5-, 6- oder 7-Benzothiazolyl, 2-, 4-, 5-, 6- oder 7-Benzisothiazolyl, 2-, 3-, 4-, 5-, 6-, 7- oder 8-Chinolinyl oder 1-, 3-, 4-, 5-, 6-, 7-, 8-Isochinolinyl,
substituiert durch -Y-Ar, ist;
wobei Y -O-, -S-, -N(R⁵)-, -(CH₂)-ₘ, -C(O)-, -CH(OH)-, -(CH₂)ₘO-, -NR⁵C(O)NR⁵R^{5'} -NR⁵C(O)-, -C(O)NR⁵, -O(CH₂)m-, -(CH₂)ₘS-, -(CH₂)ₘN(R⁵)-, -O(CH₂)ₘ-, -CHX^{a}-, -CX^{a}₂-, -S-(CH₂)ₘ- oder -N(R⁵)(CH₂)ₘ- ist,
m = 1-3 ist und X^{a} Halogen ist; und
Ar Phenyl oder Pyridinyl, gegebenenfalls substituiert durch Halogen, bis zur Perhalosubstitution und gegebenenfalls substituiert durch Zₙ₁, ist,
wobei n1 0 bis 3 ist und jeder Z unabhängig -CN, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -C(O)R⁵, NR⁵C(O)R^{5'}, C₁₋C₁₀-Alkyl, C₃-C₁₀-Cyclalkyl, C₆-C₁₄-Aryl, C₃-C₁₃-Heteroaryl, C₇-C₂₄-Alkaryl, C₄₋C₂₃-Alkheteroaryl, substituiertes C₁-C₁₀-Alkyl, substituiertes C₃-C₁₀-Cycloalkyl, substituiertes C₇-C₂₄-Alkaryl oder substituiertes C₄-C₂₃-Alkheteroaryl ist;
wobei, wenn Z eine substituierte Gruppe ist, sie durch einen oder mehrere Substituenten unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'} und -NR⁵C(O)OR^{5'} substituiert ist, und
wobei R² gegebenenfalls substituiertes Phenyl, Pyridinyl, Pyrimidinyl, Pyrazinyl, Pyridazinyl, Naphthyl, Chinolinyl, Isochinolinyl, Phthalimidinyl, Furyl, Thienyl, Pyrrolyl, Imidazolyl, Pyrazolyl, Oxazolyl, Isoxazolyl, Thiazolyl, Isothiazolyl, Benzofuryl, Benzothienyl, Indolyl, Benzopyrazolyl, Benzoxazolyl, Benzisoxazolyl, Benzothiazolyl oder Benzisothiazolyl ist, wobei, wenn R² eine substituierte Gruppe ist, sie durch einen oder mehrere Substituenten unabhängig ausgewählt aus der Gruppe bestehend aus Halogen, bis zur Perhalosubstitution und Vₙ substituiert ist,
wobei n = 0-3 ist und jeder V unabhängig ausgewählt ist aus der Gruppe bestehend aus -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₄₋Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇₋C₂₄-Alkaryl und substituiertem C₄-C₂₄-Alkheteroaryl,
wobei, wenn V eine substituierte Gruppe ist, sie durch einen oder mehrere Substituenten unabhängig ausgewählt aus der Gruppe bestehend aus Halogen, bis zur Perhalosubstitution, -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'} und -NO₂ substituiert ist;
wobei R⁵ und R^{5'} jeweils unabhängig wie oben definiert sind.

30. Verbindung nach Anspruch 29, wobei R² Phenyl, substituiertes Phenyl, Pyridinyl oder substituiertes Pyridinyl ist.

31. Verbindung nach Anspruch 29, wobei Ar Phenyl oder Pyridinyl ist.

32. Verbindung nach Anspruch 29, wobei Y -O- oder -S- ist.

33. Verbindung nach Anspruch 29, wobei Z Cl, F, CF₃, NO₂ oder CN ist.

34. Verbindung nach Anspruch 29, wobei B Diphenylether, Diphenylthioether, Diphenylamin, Phenylpyridinylether, Pyridinylmethylphenyl oder Phenylpyridinylthioether, gegebenenfalls substituiert, ist.

35. Verbindung nach Anspruch 1, wobei B Diphenylether, Diphenylthioether, Diphenylamin, Phenylpyridinylether, Pyridinylmethylphenyl, Phenylpyridinylthioether, Phenylpyrimidinylether, Phenylchinolinthioether, Phenylnaphthylether, Pyridinyinaphthylether und Pyridinylnaphthylthioether, gegebenenfalls substituiert, ist.

36. Verbindung der Formel I oder eines pharmazeutisch verträglichen Salzes davon 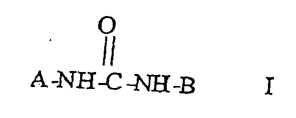 wobei
B gegebenenfalls substituierter Diphenylether, Diphenylthioether, Phenylpyridinylether, Pyridinylmethylphenyl, Phenylchinolinthioether, Phenylnaphthylether, Pyridinylnaphthylether und Pyridinylnaphthylthioether ist; und
A 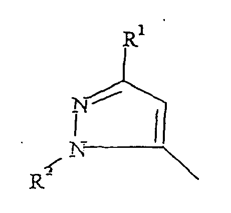 ist,
wobei
R¹ C₃-C₁₀-Alkyl, C₃-C₁₀-Cycloalkyl, bis zu perhalosubstituiertes C₁-C₁₀-Alkyl oder bis zu perhalosubstituiertes C₃-C₁₀-Cycloalkyl ist; und
R² unsubstituiertes Phenyl, unsubstituiertes Pyridinyl, substituiertes Phenyl oder substituiertes Pyridinyl ist; wobei, wenn R² eine substituierte Gruppe ist, sie durch einen oder mehrere Substituenten unabhängig ausgewählt aus der Gruppe bestehend aus Halogen, bis zur Perhalosubstitution und Vₙ substituiert ist,
wobei n = 0-3 ist und jeder V unabhängig ausgewählt ist aus der Gruppe bestehend aus -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'} -NO₂, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₄₋Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇₋C₂₄-Alkaryl und substituiertem C₄-C₂₄-Alkheteroaryl,
wobei, wenn V eine substituierte Gruppe ist, sie durch einen oder mehrere Substituenten unabhängig ausgewählt aus der Gruppe bestehend aus Halogen, bis zur Perhalosubstitution, -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'}und -NO₂ substituiert ist;
wobei R⁵ und R^{5'} jeweils unabhängig wie oben definiert sind.

37. Verbindung nach Anspruch 1, wobei R² Phenyl oder Pyridinyl, gegebenenfalls substituiert durch -NO₂, -OH, -NH₂, -OCH₃, -C₁-C₆-Alkyl, bis zu perhalosubstituiertes C₁-C₆-Alkyl, -NHC(O)-C₁₋₆-Alkyl, -SO₂-C₁₋₆-Alkyl oder -O-C_{1- 6}-Alkyl und gegebenenfalls einfach oder zweifach substituiert durch -F, -Cl oder beide, ist.

38. Verbindung nach Anspruch 1, ausgewählt aus
N-(1-Phenyl-3-tert-butyl-5-pyrazolyl)-N'-(2,3-dichlorphenyl)harnstoff;
N-(1-(4-Aminophenyl)-3-tert-butyl-5-pyrazolyl)-N'-(2,3-dichlorphenyl)harnstoff;
N-(1-(2-Methylphenyl)-3-tert-butyl-5-pyrazolyl)-N'-(2,3-dichlorphenyl)harnstoff;
N-(1-(2-Pyridinyl)-3-tert-butyl-5-pyrazolyl)-N'-(2,3-dichlorphenyl)harnstoff;
N-(1-(5-Trifluormethyl-2-pyrimidinyl)-3-tert-butyl-5-pyrazolyl)-N'-(2,3-dichlorphenyl)harnstoff;
N-(1-(3-Fluorphenyl)-3-tert-butyl-5-pyrazolyl)-N'-(2,3-dichlorphenyl)harnstoff;
N-(1-(4-Chlorphenyl)-3-tert-butyl-5-pyrazolyl)-N'-(2,3-dichlorphenyl)harnstoff;
N-(1-(4-Methansulfoxyphenyl)-3-tert-butyl-5-pyrazolyl)-N'-(2,3-dichlorphenyl)harnstoff:
N-(1-(4-Nitrophenyl)-3-tert-butyl-5-pyrazolyl)-N'-(2,3-dichlorphenyl)harnstoff;
N-(1-(3-Methoxyphenyl)-3-tert-butyl-5-pyrazolyl)-N'-(2,3-dichlorphenyl)harnstoff;
N-(1-(3-Trifluorphenyl)-3-tert-butyl-5-pyrazolyl)-N'-(2,3-dichlorphenyl)harnstoff;
N-(1-(4-Methoxyphenyl)-3-tert-butyl-5-pyrazolyl)-N'-(2,3-dichlorphenyl)harnstoff;
N-(1-(4-Isopropylphenyl)-3-tert-butyl-5-pyrazolyl)-N'-(2,3-dichlorphenyl)harnstoff;
N-(1-(3-Aminophenyl)-3-tert-butyl-5-pyrazolyl)-N'-(2,3-dichlorphenyl)harnstoff;
N-(1-(3-Nitrophenyl)-3-tert-butyl-5-pyrazolyl)-N'-(2,3-dichlorphenyl)harnstoff;
N-(1-Phenyl-3-tert-butyl-5-pyrazolyl)-N'-(3-trifluor-4-chlorphenyl)harnstoff;
N-(1-Phenyl-3-tert-butyl-5-pyrazolyl)-N'-(4-trifluorphenyl)harnstoff;
N-(1-Phenyl-3-tert-butyl-5-pyrazolyl)-N'-(2,4-difluorphenyl)harnstoff;
N-(1-Phenyl-3-tert-butyl-5-pyrazolyl)-N'-(3-fluorphenyl)harnstoff;
N-(1-Phenyl-3-tert-butyl-5-pyrazolyl)-N'-(3-cyanphenyl)harnstoff;
N-(1-Phenyl-3-tert-butyl-5-pyrazolyl)-N'-(4-nitrophenyl)harnstoff;
N-(1-(3-Acetamidophenyl)-3-tert-butyl-5-pyrazolyl)-N'-(4-phenoxyphenyl)harnstoff;
N-(1-(3-Aminophenyl)-3-tert-butyl-5-pyrazolyl)-N'-(4-phenoxyphenyl)harnstoff;
N-(1-(3-Nitrophenyl)-3-tert-butyl-5-pyrazolyl)-N'-(4-phenoxyphenyl)harnstoff;
N-(1-Phenyl-3-tert-butyl-5-pyrazolyl)-N'-(4-(4-pyridinylmethyl)phenyl)harnstoff;
N-(1-(4-Pyridinyl)-3-tert-butyl-5-pyrazolyl)-N'-(4-(4-pyridinylmethyl)phenyl)harnstoff;
N-(1-(2,5-Dichlorphenyl)-3-tert-butyl-5-pyrazolyl)-N'-(4-(4-pyridinylmethyl)phenyl)harnstoff;
N-(1-(4-Fluorphenyl)-3-tert-butyl-5-pyrazolyl)-N'-(4-(4-pyridinylmethyl)phenyl)harnstoff;
N-(1-(2-Methylphenyl)-3-tert-butyl-5-pyrazolyl)-N'-(4-(4-pyridinylmethyl)phenyl)harnstoff;
N-(1-(3-Fluorphenyl)-3-tert-butyl-5-pyrazolyl)-N'-(4-(4-pyridinylmethyl)phenyl)harnstoff;
N-(1-(4-Methansulfoxyphenyl)-3-tert-butyl-5-pyrazolyl)-N'-(4-(4-pyridinylmethyl)phenyl)harnstoff;
N-(1-(4-Nitrophenyl)-3-tert-butyl-5-pyrazolyl)-N'-(4-(4-pyridinylmethyl)phenyl)harnstoff;
N-(1-(3-Methoxyphenyl)-3-tert-butyl-5-pyrazolyl)-N'-(4-(4-pyridinylmethyl)phenyl)harnstoff;
N-(1-(3-Aminophenyl)-3-tert-butyl-5-pyrazolyl)-N'-(4-(4-pyridinylmethyl)phenyl)harnstoff;
N-(1-(3-Nitrophenyl)-3-tert-butyl-5-pyrazolyl)-N'-(4-(4-pyridinylmethyl)phenyl)harnstoff;
N-(1-(3-Aminophenyl)-3-tert-butyl-5-pyrazolyl)-N'-(4-(4-pyridinylthio)phenyl)harnstoff;
N-(1-(3-Nitrophenyl)-3-tert-butyl-5-pyrazolyl)-N'-(4-(4-pyridinylthio)phenyl)harnstoff;
N-(1-(3-(2,3-Dichlorphenoxyacylamino)phenylmethyl)-3-tert-butyl-5-pyrazolyl)-N'-(2,3-dichlorphenyl)harnstoff;
N-(1-(3-Hydroxyphenylmethyl)-3-tert-butyl-5-pyrazolyl)-N'-(2,3-dichlorphenyl)harnstoff;
N-(2-Phenyl-5-tert-butyl-3-thienyl)-N'-(2,3-dichlorphenyl)harnstoff;
und
N-(2-(4-Pyridinyl)-5-tert-butyl-3-furyl)-N'-(2,3-dichlorphenyl)harnstoff.

39. Verbindung nach Anspruch 1, wobei R¹ t-Butyl ist und R² unsubstituiertes oder substituiertes Phenyl ist.

40. Verbindung nach Anspruch 3, wobei Q Phenyl ist, Q¹ Phenyl oder Pyridinyl ist, Y -O-, -S- oder -CH₂- ist und Z Cl, F, NO₂ oder CF₃ ist.

## Revendications

1. Composé de formule 1 et sels pharmaceutiquement acceptables de celui-ci 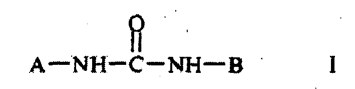 dans laquelle A est un radical hétéroaryle choisi dans le groupe constitué par 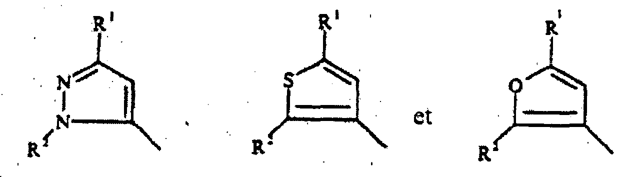 où R¹ est choisi dans le groupe constitué par les radicaux alkyle en C₃-C₁₀, cycloalkyle en C₃-C₁₀, alkyle en C₁-C₁₀ halogéné jusqu'à la perhalogénation, et cycloalkyle en C₃₋C₁₀ halogéné jusqu'à la perhalogénation,
B est un fragment aryle ou hétéroaryle d'au plus 12 atomes de carbone, contenant jusqu'à 3 cycles, avec au moins une structure aromatique de 5 ou 6 chaînons contenant 0 à 4 membres du groupe constitué par l'azote, l'oxygène et le soufre, substitué par -Y-Ar et éventuellement substitué par un ou plusieurs substituants choisis indépendamment dans le groupe constitué par les halogènes, jusqu'à la perhalogénation, et Xn,
où n = 0-3 et chaque X est choisi indépendamment dans le groupe constitué par les radicaux -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, alkaryle en C₇-C₂₄, hétéroaryle en C₃-C₁₃, alkylhétéroaryle en C₄-C₂₃, alkyle en C₁-C₁₀ substitué, alcényle en C₂-C₁₀ substitué, alcoxy en C₁-C₁₀ substitué, çycloalkyle en C₃-C₁₀ substitué, alkylhétéroaryle en C₄-C₂₃ substitué ;
lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'}, et halogène jusqu'à la perhalogénation ;
où R⁵ et R^{5'} sont choisis indépendamment parmi H et les radicaux alkyle en C₁-C₁₀, alcényle en C₂-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ halogéné jusqu'à la perhalogénation, alcényle en C₂-C₁₀ halogéné jusqu'à la perhalogénation, cycloalkyle en C₃-C₁₀ halogéné jusqu'à la perhalogénation, aryle en C₆-C₁₄ halogéné jusqu'à la perhalogénation et hétéroaryle en C₃-C₁₃ halogène jusqu'à la perhalogénation,
Y est -O-, -S-, -N(R⁵)-, -(CH₂)ₘ-, -C(O)-, -CH(OH)-, -(CH₂)ₘO-, -NR⁵C(O)NR⁵R^{5'}-, -NR⁵C(O)-, -C(O)NR⁵, -O(CH₂)ₘ-, -(CH₂)ₘS-, -(CH₂)ₘ,N(R⁵)-, O(CH₂)ₘ-, -CHX^{a}-,-CX^{a}₂-, -S(CH₂)ₘ- ou -N(R⁵)(CH₂)ₘ-,
m = 1-3 et X^{a} est un halogène ; et
Ar est une structure aromatique de 5 à 10 chaînons contenant 0 à 2 membres du groupe constitué par des atomes d'azote, d'oxygène et de soufre, non substituée ou halogénée jusqu'à la perhalogénation, et éventuellement substituée par Zₙ₁ où n1 est un nombre de 0 à 3 et chaque Z est choisi indépendamment dans le groupe constitué par les radicaux-CN, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -C(O)R⁵, -NR⁵C(O)R^{5'}, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ substitué, cycloalkyle en C₃-C₁₀ substitué, alkaryle en C₇-C₂₄ substitué et alkylhétéroaryle en C₄-C₂₃ substitué ;
lorsque Z est un groupe substitué, il est substitué par un ou plusieurs substituants choisis indépendamment dans le groupe constitué par -CN, -CO₂R⁵, -C(O)NR⁵R^{5'}, -OR⁵, -SR⁵, -NO₂, -NR⁵R^{5'}, -NR⁵C(O)R^{5'} et -NR⁵C(O)OR^{5'}, et
R² est un radical aryle en C₆-C₁₄, hétéroaryle en C₃-C₁₄, aryle en C₆-C₁₄ substitué ou hétéroaryle en C₃-C₁₄ substitué,
lorsque R² est un groupe substitué, il est substitué par un ou plusieurs substituants choisis indépendamment dans le groupe constitué par les halogènes, jusqu'à la perhalogénation, et Vₙ,
où n = 0 à 3 et chaque V est choisi indépendamment dans le groupe constitué par les radicaux -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, hétéroaryle en C₃₋C₁₃, alkaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkaryle en C₇-C₂₄ substitué et alkylhétéroaryle en C₄-C₂₄ substitué,
lorsque V est un groupe substitué, il est substitué par un ou plusieurs substituants indépendamment choisis dans le groupe constitué par les halogènes jusqu'à la perhalogénation,' -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'} et -NO₂;
où R⁵ et R^{5'} ont chacun indépendamment la définition donnée ci-dessus.

2. Composé selon la revendication 1, dans lequel R² est un radical phényle ou pyridinyle substitué ou non substitué, et les substituants de R² sont choisis dans le groupe constitué par les halogènes jusqu'à la perhalogénation et Vₙ, où n = 0 à 3, et chaque V est choisi indépendamment dans le groupe constitué par les radicaux substitués et non substitués alkyle en C₁-C₆, cycloalkyle en C₃-C₁₀, aryle en C₆-C₁₀, -NO₂, -NH₂, -C(O)-(alkyle en C₁-C₆), -C(O)N-(alkyle en C₁-C₆)₂, -C(O)NH-(alkyle en C₁-C₆), -O-alkyle en C₁-C₆, -NHC(O)H, -NHC(O)OH, -N(alkyl en C₁-C₆)C(O)-(alkyle en C₁-C₆), -N-(alkyl en C₁₋C₆)C(O)-(alkyle en C₁-C₆), -NHC(O)-(alkyle en C₁-C₆), -OC(O)NH-(aryle en C₆-C₁₄), -NHC(O)O-(alkyle en C₁-C₆), -S(O)-alkyle en C₁-C₆ et -SO₂-alkyle en C₁-C₆,
lorsque V est un groupe substitué, il est substitué par un ou plusieurs halogènes, jusqu'à la perhalogénation.

3. Composé selon la revendication 2, dans lequel B est 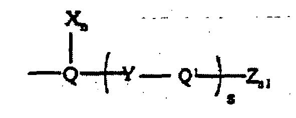 où
Y est choisi dans le groupe se composant de -O-, -S-, -CH₂-, -SCH₂-, -CH₂S-, -CH(OH)-, -C(O)-, -CX^{a}₂ -,-CX^{a}H-, -CH₂O- et -OCH₂-,
X^{a} est un halogène,
Q est une structure aromatique contenant 0 à 2 atomes d'azote, non substituée ou halogénée jusqu'à la perhalogénation,
Q¹ est une structure aromatique mono ou bicyclique constituée de 3 à 10 atomes de carbone et de 0 à 4 membres du groupe constitué par N, O et S, non substituée ou halogénée jusqu'à la perhalogénation,
n est 0, Z et n1 sont comme définis dans la revendication 1, et s = 1.

4. Composé selon la revendication 3, dans lequel :
Q est un radical phényle ou piridinyle, non substitué ou substitué par des halogènes jusqu'à la perhalogénation,
Q¹ est choisi dans le groupe constitué par les radicaux phényle, pyridinyle, naphtyle, pyrimidinyle, quinolie, isoquinoline, imidazole et benzothiazolyle, non substitués ou halogénés jusqu'à la perhalogénation, et
Z est choisi dans le groupe constitué par -R⁶, -OR⁶ et -NHR⁷, où R⁶ est un atome d'hydrogène, un radical alkyle en C₁-C₁₀, cycloalkyle en C₃-C₁₀, et R⁷ est choisi dans le groupe constitué par l'hydrogène, les radicaux alkyle en C₃-C₁₀, cycloalkyle en C₃-C₆ et aryle en C₆-C₁₀, et R⁶ et R⁷ peuvent être halogénés jusqu'à la perhalogénation.

5. Composé selon la revendication 1, où R¹ est le radical t-butyle et R² et un radical phényle non substitué ou substitué.

6. Composé selon la revendication 4 dans lequel Q est un radical phényle, Q¹ est un radical phényle ou pyridinyle, Y est -O-, -S- ou -CH₂-, et X et Z' sont indépendamment un radical Cl, F, NO₂ ou CF₃.

7. Composé selon la revendication 6 dans lequel R² est un radical t-butyle.

8. Composé selon la revendication 1 de formule 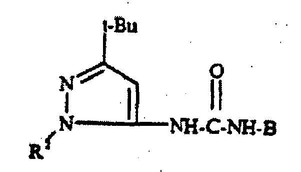 dans laquelle B et R² ont la définition donnée dans la revendication 1.

9. Composé selon la revendication 8, dans lequel R² est choisi parmi des membres substitués et non substitués du groupe constitué par les radicaux phényle et pyridinyle, et, lorsque R² est un groupe substitué, il est substitué par un ou plusieurs substituants choisis dans le groupe constitué par les halogènes et Wₙ, où n = 0 à 3 et W est choisi dans le groupe constitué par les radicaux -NO₂, alkyle en C₁-C₃, -NH(O)CH₃, -CF₃, -OCH₃, -F, -Cl, -NH₂, -OC(O)NH- phényle halogéné jusqu'à la perhalogénation, -SO₂CH₃, pyridinyle, phényle, phényle halogéné jusqu'à la perhalogénation et phényle substitué par alkyle en C₁-C₆.

10. Composé selon la revendication 1, de formule 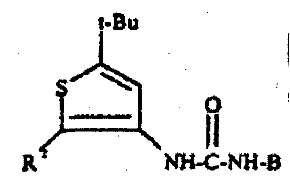 dans laquelle B et R² ont la définition donnée dans la revendication 1.

11. Composé selon la revendication 10, dans lequel R² est choisi parmi des membres substitués et non substitués du groupe constitué par les radicaux phényle et pyridinyle, et, lorsque R² est un groupe substitué, il est substitué par un ou plusieurs substituants choisis dans le groupe constitué par les halogènes et Wₙ, où n = 0 à 3 et W est choisi dans le groupe constitué par les radicaux -NO₂, alkyle en C₁-C₃, -NH(O)CH₃, -CF₃, -OCH₃, -F, -Cl, -NH₂, -SO₂CH₃, pyridinyle, phényle, phényle halogéné jusqu'à la perhalogénation, et phényle substitué par alkyle en C₁-C₆.

12. Composé selon la revendication 1, de formule 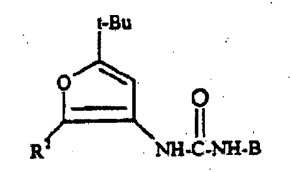 dans laquelle B et R² ont la définition donnée dans la revendication 1.

13. Composé selon la revendication 13, dans lequel R² est choisi parmi des membres substitués et non substitués du groupe constitué par les radicaux phényle et pyridinyle, et, lorsque R² est un groupe substitué, il est substitué par un ou plusieurs substituants choisis dans le groupe constitué par les halogènes et Wₙ, où n = 0 à 3 et W est choisi dans le groupe constitué par les radicaux -NO₂, alkyle en C₁-C₃, -NH(O)CH₃, -CF₃, -OCH₃, -F, -Cl, -NH₂, -SO₂CH₃, pyridinyle, phényle, phényle halogéné jusqu'à la perhalogénation, et phényle substitué par alkyle en C₁-C₆.

14. Utilisation d'un composé de formule 1 ou d'un sel pharmaceutiquement acceptable de celui-ci : 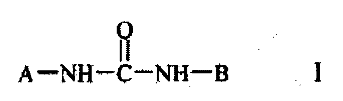 dans laquelle A est un radical hétéroaryle choisi dans le groupe constitué de 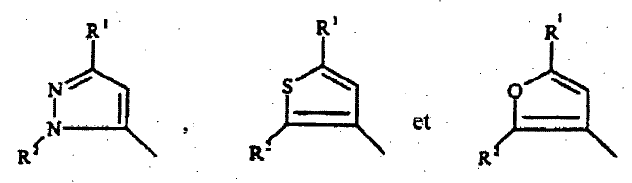 où R¹ est choisi dans le groupe constitué par les radicaux alkyle en C₃-C₁₀, cycloalkyle en C₃-C₁₀, alkyle en C₁-C₁₀ halogéné jusqu'à la perhalogénation, et cycloalkyle en C₃-C₁₀ halogéné jusqu'à la perhalogénation ;
B est un fragment aryle ou hétéroaryle d'au plus 12 atomes de carbone, contenant jusqu'à 3 cycles, substitué ou non substitué, avec au moins une structure aromatique de 5 ou 6 chaînons contenant 0 à 4 membres du groupe constitué par l'azote, l'oxygène et le soufre, et, si B est un groupe substitué, il est substitué par un ou plusieurs substituants choisis dans le groupe constitué par les halogènes, jusqu'à la perhalogénation, et Xₙ,
où n = 0-3 et chaque X est choisi indépendamment dans le groupe constitué par les radicaux -CN; -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, alkaryle en C₇-C₂₄, hétéroaryle en C₃-C₁₃, alkylhétéroaryle en C₄-C₂₃, alkyle en C₁-C₁₀ substitué, alcényle en C₂-C₁₀ substitué, alcoxy en C₁-C₁₀ substitué, cycloalkyle en C₃-C₁₀ substitué, alkylhétéroaryle en C₄-C₂₃ substitué et -Y -Ar ;
lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, OR⁵, -SR⁵, -NR⁵R^{5'}, -NO₂, -NR⁵C(O)R^{5'}, -NR⁵C(O)OR^{5'}, et halogène jusqu'à la perhalogénation ;
R⁵ et R^{5'} sont choisis indépendamment parmi H et les radicaux alkyle en C₁-C₁₀, alcényle en C₂-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ halogéné jusqu'à la perhalogénation, alcényle en C₂-C₁₀ halogéné jusqu'à la perhalogénation, cycloalkyle en C₃-C₁₀ halogéné jusqu'à la perhalogénation, aryle en C₆-C₁₄ halogéné jusqu'à la perhalogénation et hétéroaryle en C₃-C₁₃ halogéné jusqu'à la perhalogénation, Y est -O-, -S-, -N(R⁵)-, -(CH₂)ₘ-, -C(O)-, -CH(OH)-, -(CH₂)ₘO-, -(CH₂)ₘS-, -(CH₂)ₘN(R⁵)-, -O(CH₂)ₘ-, -CHX^{a}-, -CHX^{a}₂-, -S-(CH₂)ₘ- ou N(R⁵)(CH₂)ₘ -,
m = 1-3 et X^{a} est un halogène ; et
Ar est une structure aromatique de 5 ou 6 chaînons contenant 0 à 2 membres du groupe constitué par des atomes d'azote, d'oxygène et de soufre, non substituée ou halogénée jusqu'à la perhalogénation, et éventuellement substituée par Zₙ₁ où n1 est un nombre de 0 à 3 et chaque Z est choisi indépendamment dans le groupe constitué par les radicaux -CN, -C(O)R⁵, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ substitué, cycloalkyle en C₃-C₁₀ substitué, alkaryle en C₇-C₂₄ substitué et alkylhétéroaryle en C₄-C₂₃ substitué ;
lorsque Z est un groupe substitué, il est substitué par un ou plusieurs substituants choisis indépendamment dans le groupe constitué par -CN, CO₂R⁵, -C(O)NR⁵R^{5'}, -OR⁵, -SR⁵,-NO₂, -NR⁵R^{5'}, -NR⁵C(O)R^{5'} et -NR⁵C(O)OR^{5'}, et
R² est un radical aryle en C₆-C₁₄, hétéroaryle en C₃-C₁₄, aryle en C₆-C₁₄ substitué ou hétéroaryle en C₃-C₁₄ substitué,
lorsque R² est un groupe substitué, il est substitué par un ou plusieurs substituants choisis indépendamment dans le groupe constitué par les halogènes, jusqu'à la perhalogénation, et Vₙ,
où n = 0 à 3 et chaque V est choisi indépendamment dans le groupe constitué par les radicaux -CN, CO₂R⁵, -C(O)NR⁵R^{5'}, -OR⁵, -SR⁵, -NR⁵R^{5'}, -OC(O)NR⁵R^{5'}, -NR⁵C(O)OR^{5'}, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkaryle en C₇-C₂₄ substitué et alkylhétéroaryle en C₄-C₂₄ substitué,
lorsque V est un groupe substitué, il est substitué par un ou plusieurs substituants choisis dans le groupe constitué par les halogènes jusqu'à la perhalogénation, -CN, CO₂R⁵, -C(O)R⁵, -C(O)NR⁵R^{5'}, -NR⁵R^{5'}, -OR⁵, -SR⁵, -NR⁵C(O)R^{5'}, NR⁵C(O)OR^{5'} et NO₂,
où R⁵ et R^{5'} ont chacun indépendamment la définition donnée ci-dessus pour la préparation d'un médicament destiné au traitement de maladies dues à la raf kinase.

15. Utilisation selon la revendication 14, dans laquelle R² est choisi parmi des membres substitués et non substitués du groupe constitué par les radicaux phényle et pyridinyle, et les substituants de R² sont choisis dans le groupe constitué par les halogènes, jusqu'à la perhalogénation, et Vₙ, où n = 0 à 3 et chaque V est choisi indépendamment dans le groupe constitué par les radicaux substitués ou non substitués alkyle en C₁-C₆, cycloalkyle en C₃-C₁₀, aryle en C₆-C₁₀, -NO₂, -NH₂, -C(O)-(alkyle en C₁-C₆), -C(O)N-(alkyle en C₁-C₆)₂, -C(O)NH-(alkyle en C₁-C₆), -O-alkyle en C₁-C₆, -NHC(O)H, -NHC(O)OH, -N(alkyle en C₁-C₆)C(O)-(alkyle en C₁-C₆), -N-(alkyl en C₁-C₆)C(O)-(alkyle en C₁-C₆), -NHC(O)-(alkyle en C₁-C₆), -NHC(O)O-(alkyle en C₁-C₆), -S(O)-alkyle en C₁-C₆ et -SO₂-alkyle en C₁-C₆, et
lorsque V est un groupe substitué, il est substitué par un ou plusieurs halogènes, jusqu'à la perhalogénation.

16. Utilisation selon la revendication 14, dans laquelle B est une structure cyclique aromatique contenant jusqu'à 3 cycles, choisie dans le groupe constitué de 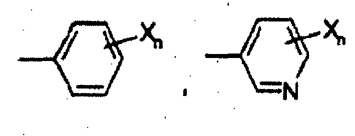 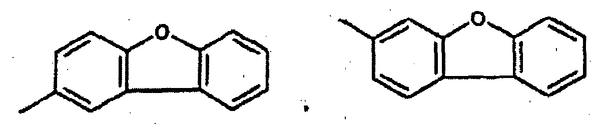 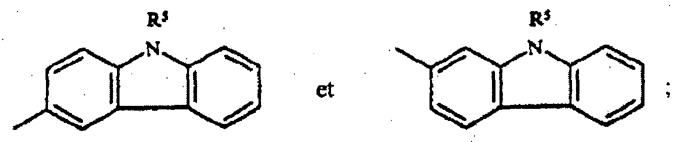 non substituée ou halogénée jusqu'à la perhalogénation, et dans laquelle
n= 0-3 et
chaque X est choisi indépendamment dans le groupe constitué par des radicaux -CN, -CO₂R⁵, -C(O)NR⁵R^{5'}, C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, alkaryle en C₇-C₂₄, hétéroaryle en C₃-C₁₃, alkylhétéroaryle en C₄-C₂₃, et alkyle en C₁-C₁₀ substitué, alcényle en C₂-C₁₀ substitué, alcoxy en C₁-C₁₀ substitué, cycloalkyle en C₃-C₁₀ substitué, alkylhétéroaryle en C₄-C₂₃ substitué et -Y-Ar ; lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'}, et halogène jusqu'à la perhalogénation ;
R⁵ et R^{5'} sont choisis indépendamment parmi H et les radicaux alkyle en C₁-C₁₀, alcényle en C₂-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ halogéné jusqu'à la perhalogénation, alcényle en C₂-C₁₀ halogéné jusqu'à la perhalogénation, cycloalkyle en C₃-C₁₀ halogéné jusqu'à la perhalogénation, aryle en C₆-C₁₄ halogéné jusqu'à la perhalogénation et hétéroaryle en C₃-C₁₃ halogéné jusqu'à la perhalogénation,
Y est -O-, -S-, -N(R⁵)-, -(CH₂)ₘ-, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)m-, - -CHX^{a}-, -CX^{a}₂-, -S(CH₂)ₘ₋ et -N(R⁵)(CH₂)ₘ-,
m = 1-3 et X^{a} est un halogène ; et
Ar est une structure aromatique de 5 à 10 chaînons contenant 0 à 2 membres du groupe constitué par l'azote, l'oxygène et le soufre, non substituée ou halogénée jusqu'à la perhalogénation, et éventuellement substitué par Zₙ₁ où n1 est un nombre de 0 à 3 et chaque Z est choisi indépendamment dans le groupe constitué par les radicaux -CN, -C(O)R⁵, -CO₂R⁵, -C(O)NR⁵R^{5'}, -NO₂, -OR⁵, -SR⁵, -NR⁵R^{5'}, -NR⁵C(O)OR^{5'}, -NR⁵C(O)R^{5'}, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ substitué, cycloalkyle en C₃-C₁₀ substitué, alkaryle en C₇-C₂₄ substitué et alkylhétéroaryle en C₄-C₂₃ substitué ; lorsque Z est un groupe substitué, il est substitué par un ou plusieurs substituants choisis indépendamment dans le groupe constitué par -CN, -CO₂R⁵, -C(O)NR⁵R^{5'}, -OR⁵, -SR⁵, -NO₂, -NR⁵R^{5'}, -NR⁵C(O)R^{5'} et -NR⁵C(O)OR^{5'}.

17. Utilisation selon la revendication 14, dans laquelle B est un radical de formule 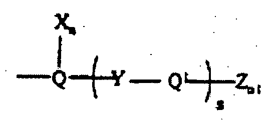 dans laquelle
Y est choisi dans le groupe constitué par -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est un halogène,
Q est une structure aromatique de 6 chaînons contenant 0 à 2 atomes d'azote, non substituée ou halogénée jusqu'à la perhalogénation,
Q¹ est une structure aromatique mono- ou bicyclique de 5 à 10 chaînons contenant de 3 à 10 atomes de carbone et de 0 à 2 membres du groupe constitué par N, O et S, non substituée ou halogénée jusqu'à la perhalogénation,
X, Z, et n1 ont la définition donnée dans la revendication 15, et s = 0 ou 1.

18. Utilisation selon la revendication 17, dans laquelle
Q est un radical phényle ou pyridinyle, substitué ou non substitué par halogène, jusqu'à la perhalogénation,
Q¹ est choisi dans le groupe constitué par les radicaux phényle, pyridinyle, naphtyle, pyrimidinyle, quinoline, isoquinoline, imidazole et benzothiazolyle, non substitués ou halogénés jusqu'à la perhalogénation,
Z et X sont choisis indépendamment dans le groupe constitué par -R⁶, -OR⁶ et -NHR⁷, où R⁶ est un atome d'hydrogène ou un radical alkyle en C₁-C₁₀ ou cycloalkyle en C₃-C₁₀ et R⁷ est choisi dans le groupe constitué par l'hydrogène et les radicaux alkyle en C₃-C₁₀, cycloalkyle en C₃-C₆ et aryle en C₆-C₁₀, R⁶ et R⁷ pouvant être halogénés jusqu'à la perhalogénation.

19. Utilisation selon la revendication 17, dans laquelle Q est un radical phényle, Q¹ est un radical phényle ou pyridinyle, Y est -O-, -S- ou -CH₂-, et X et Z sont indépendamment un radical Cl, F, NO₂ ou CF₃.

20. Utilisation selon la revendication 14, comprenant l'administration d'un composé ayant l'une des formules 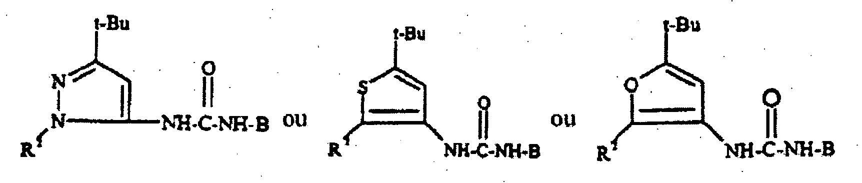 dans lesquelles B et R² ont la définition donnée dans la revendication 15.

21. Utilisation selon la revendication 20, dans laquelle R² est choisi parmi des membres substitués et non substitués du groupe constitué par les radicaux phényle et pyridinyle, et, lorsque R² est un groupe substitué, il est substitué par un ou plusieurs substituants choisis dans le groupe constitué par les halogènes et Wₙ où n = 0 à 3 et W est choisi dans le groupe constitué par les radicaux -NO₂, alkyle en C₁-C₃, -NH(O)CH₃, -CF₃, -OCH₃, -F, -Cl, -NH₂, -OC(O)NH-phényle halogéné jusqu'à la perhalogénation, -SO₂CH₃, pyridinyle, phényle, phényle halogéné jusqu'à la perhalogénation, et phényle substitué par alkyle en C₁-C₆.

22. Utilisation selon la revendication 14, comprenant l'administration d'une quantité de composé de formule 1 efficace pour inhiber la raf kinase.

23. Composition pharmaceutique comprenant un composé selon la revendication 1 et un support pharmaceutiquement acceptable.

24. Composition pharmaceutique comprenant un composé selon la revendication 2 et un support pharmaceutiquement acceptable.

25. Utilisation d'un composé de formule 1 ou d'un sel pharmaceutiquement acceptable de celui-ci sur un hôte en ayant besoin de celui-ci : 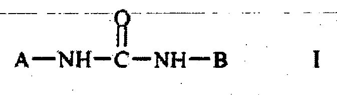 dans laquelle A est 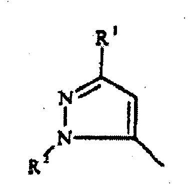 où R¹ est choisi dans le groupe constitué par les radicaux alkyle en C₃-C₁₀, cycloalkyle en C₃-C₁₀, alkyle en C₁-C₁₀ halogéné jusqu'à la perhalogénation, et cycloalkyle en C₃-C₁₀ halogéné jusqu'à la perhalogénation,
B est un fragment aryle ou hétéroaryle d'au plus 12 atomes de carbone, contenant jusqu'à 3 cycles, avec au moins une structure aromatique de 5 ou 6 chaînons contenant 0 à 4 membres du groupe constitué par l'azote, l'oxygène et le soufre, et si B est un groupe substitué, il est substitué par un ou plusieurs substituants choisis indépendamment dans le groupe constitué par, les halogènes, jusqu'à la perhalogénation, et Xn,
où n = 0-3 et chaque X est choisi indépendamment dans le groupe constitué par les radicaux -CN, -CO₂R⁵, -C(O)NR⁵R^{5'}, -C(O)R5, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, alkaryle en C₇-C₂₄, hétéroaryle en C₃-C₁₃, alkylhétéroaryle en C₄-C₂₃, alkyle en C₁-C₁₀ substitué, alcényle em C₂-C₁₀ substitué, alcoxy en C₁-C₁₀ substitué, cycloalkyle en C₃-C₁₀ substitué, aryle en C₆-C₁₄ halogéné jusqu'à la perhalogénation, hétéroaryle en C₃-C₁₃ halogéné jusqu'à la perhalogénation, alkythétéroaryle en C₄-C₂₃ substitué et -Y-Ar ;
lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'}, et halogène jusqu'à la perhalogénation ;
où R⁵ et R^{5'} sont choisis indépendamment parmi H et les radicaux alkyle en C₁-C₁₀, alcényle en C₂-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ halogéné jusqu'à la perhalogénation, alcényle en C₂-C₁₀ halogéné jusqu'à la perhalogénation, cycloalkyle en C₃-C₁₀ halogéné jusqu'à la perhalogénation, aryle en C₆-C₁₄ halogéné jusqu'à la perhalogénation et hétéroaryle en C₃-C₁₃ halogéné jusqu'à la perhalogénation,
où Y est -O-, -S-, -N(R⁵)-, -(CH₂)ₘ-, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-,-S(CH₂)ₘ- ou -N(R⁵)(CH₂)ₘ-,
m = 1-3 et X^{a} est un halogène ; et
Ar est une structure aromatique de 5 ou 6 chaînons contenant 0 à 2 membres du groupe constitué par des atomes d'azote, d'oxygène et de soufre, non substituée ou halogénée jusqu'à la perhalogénation, et éventuellement substituée par Zₙ₁ où n1 est un nombre de 0 à 3 et chaque Z est indépendamment un radical -CN, -C(O)R⁵, -CO₂R⁵, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ substitué, cycloalkyle en C₃-C₁₀ substitué, alkaryle en C₇-C₂₄ substitué ou alkylhétéroaryle en C₄-C₂₃ substitué ;
où si Z est un groupe substitué, il est substitué par un ou plusieurs substituants choisis indépendamment dans le groupe constitué par -CN, -CO₂R⁵, -C(O)NR⁵R^{5'}; -OR⁵, -SR⁵, -NO₂, -NR⁵R^{5'}, -NR⁵C(O)R^{5'} et -NR⁵C(O)OR^{5'}, et
où R² est un radical aryle en C₆-C₁₄, hétéroaryle en C₃-C₁₄, aryle en C₆-C₁₄ substitué ou hétéroaryle en C₃-C₁₄ substitué,
où si R² est un groupe substitué, il est substitué par un ou plusieurs substituants choisis indépendamment dans le groupe constitué par les halogènes, jusqu'à la perhalogénation, et Vₙ,
où n = 0 à 3 et chaque V est choisi indépendamment dans le groupe constitué par les radicaux -CN, -CO₂R⁵, -C(O)NR⁵R^{5'}, -OR⁵, -SR⁵, -NR⁵R^{5'}, -OC(O)NR⁵R^{5'}, C(O)R⁵, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkaryle en C₇-C₂₄ substitué et alkylhétéroaryle en C₄-C₂₄ substitué,
lorsque V est un groupe substitué, il est substitué par un ou plusieurs substituants indépendamment choisis dans le groupe constitué par les halogènes jusqu'à la perhalogénation, -CN, -CO₂R⁵, -C(O)R⁵, -C(O)NR⁵R^{5'}, -NR⁵R^{5'}, -OR⁵, -SR⁵, -NR⁵C(O)R^{5'} -NR⁵C(O)OR^{5'} et -NO₂ ;
où R⁵ et R^{5'} ont chacun indépendamment la définition donnée ci-dessus
pour la préparation d'un médicament destiné au traitement de cancers solides, de maladies myéloïdes ou d'adénomes.

26. Utilisation selon la revendication 25 dans laquelle le composé de formule 1 présente des CI50 de 1 nM à 10 µM comme déterminé par un essai raf kinase in vitro.

27. Utilisation selon la revendication 25 dans laquelle la maladie est un cancer dépendant de la cascade de la transduction du signal de la protéine ras et est traitée en inhibant la raf kinase.

28. Utilisation selon la revendication 25 dans laquelle le cancer solide est un carcinome du poumon, du pancréas, de la thyroïde, de la vessie ou du colon.

29. Composé de formule 1 ou d'un sel pharmaceutiquement acceptable de celui-ci 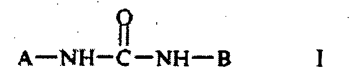 dans lequel A est 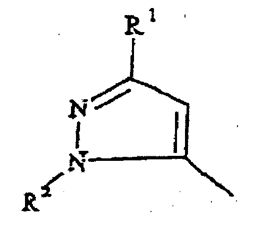 où R¹ est un radical alkyle en C₃-C₁₀, cycloalkyle en C₃-C₁₀, alkyle en C₁-C₁₀ halogéné jusqu'à la perhalogénation ou cycloalkyle en C₃-C₁₀ halogéné jusqu'à la perhalogénation, B est un phényle, un 2- ou un 3-furyle, un 2- ou un 3-thiényle, un 1-, un 2- ou un 3-pyrrolyle, un 1-, un 2-, un 4- ou un 5-imidazolyle, un 1-, un 3-, un 4- ou un 5-pyrazolyle, un 2-, un 4- ou un 5-oxazolyle, un 3-, un 4- ou un 5-isooxazolyle, un 2-, un 4- ou un 5-thiazolyle, un 3-, un 4- ou un 5- isothiazolyle, un 2-, un 3- ou un 4- pyridyle, un 2-, un 4-, un 5- ou un 6-pyrimidinyle, un 3- ou un 4-pyridazinyle, un pyrazinyle, un 2-, un 3-, un 4- un 5, un 6- ou un 7-benzofuryle, un 2-, un 3-, un 4- un 5, un 6- ou un 7-benzothiényle, un 1-, un 2-, un 3-, un 4- un 5, un 6- ou un 7-indolyle, un 1-, un 3-, un 4- un 5, un 6- ou un 7-benzopyrazolyle, un 2-, un 4- un 5, un 6- ou un 7- benzoxazolyle, un 3-, un 4- un 5, un 6- ou un 7-benzisoxazolyle, un 1-, un 3-, un 4- un 5, un 6- ou un 7-benzothiazolyle, un 2-, un 4- un 5, un 6- ou un 7-benzisothiazolyle, un 2-, un 3-, un 4- un 5, un 6-, un 7- ou un 8-quinolinyle ou un 1-, un 3-, un 4- un 5, un 6-, un 7- ou un 8-isoquinolinyle; substitué par -Y-Ar ;
dans lequel Y est -O-, -S-, -N(R⁵)-, -(CH₂)ₘ-, -C(O)-, -CH(OH)-, -(CH₂)ₘO-,-NR⁵C(O)NR⁵R^{5'}-, -NR⁵C(O)-, -C(O)NR⁵, -O(CH₂)ₘ , -(CH₂)ₘS-, -(CH₂)ₘN(R⁵)-, -O(CH₂)-ₘ-, CHX^{a}-, -CX^{a}₂-, -S(CH₂)ₘ ou -N(R⁵)(CH₂)ₘ-,
m = 1-3 et X^{a} est un halogène ; et
Ar est un phényle ou un pyridinyle éventuellement halogéné jusqu'à la perhalogénation et éventuellement substitué par Zₙ₁,
où n1 est un nombre de 0 à 3 et chaque Z est indépendamment -CN, -CO₂R⁵; -C(O)NR⁵R^{5'}, -C(O)NR⁵, -NO₂, -OR⁵, -SR⁵, -NR⁵R^{5'}, -NR⁵C(O)OR^{5'}, -C(O)R⁵, -NR⁵C(O)R^{5'}, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₃, alkyle en C₁-C₁₀ substitué, cycloalkyle en C₃-C₁₀ substitué, alkaryle en C₇-C₂₄ substitué et alkylhétéroaryle en C₄-C₂₃ substitué ;
où si Z est un groupe substitué, il est substitué par un ou plusieurs substituants choisis indépendamment dans le groupe constitué par -CN, -CO₂R⁵, -C(O)NR⁵R^{5'}, -OR⁵-, -SR⁵, -NO₂, -NR⁵R^{5'}, -NR⁵C(O)R^{5'} et -NR⁵C(O)OR^{5'}, et
où R² est éventuellement un phényle, un pyridinyle, un pyrimidinyle, un pyrazinyle, un pyridazinyle, un naphtyle, un quinolinyle, un isoquinolinyle, un phtalimidinyle, un furyle, un thyényle, un pyrrolyle, un imidazolyle, un pyrazolyle, un oxazolyle, un isoxazolyle, un thiazolyle, un isothiazolyle, un benzofuryle, un benzothiényle, un indolyle, un benzopyrazolyle, un benzoxazolyle, un benzoisoxazolyle, un benzothiazolyle ou un benzisothiazolyle, lorsque R² est un groupe substitué, il est substitué par un ou plusieurs substituants choisis indépendamment dans le groupe constitué par les halogènes, jusqu'à la perhalogénation, et Vₙ,
où n = 0 à 3 et chaque V est choisi indépendamment dans le groupe constitué par les radicaux -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, -hétéroaryle en C₃-C₁₃, alkaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkaryle en C₇-C₂₄ substitué et alkylhétéroaryle en C₄-C₂₄ substitué,
lorsque V est un groupe substitué, il est substitué par un ou plusieurs substituants indépendamment choisis dans le groupe constitué par les halogènes jusqu'à la perhalogénation, -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'} et -NO₂ ;
où R⁵ et R^{5'} ont chacun indépendamment la définition donnée ci-dessus.

30. Composé selon la revendication 29 dans lequel R² est un radical phényle, un radical phényle substitué, un radical pyridinyle ou un radical pyridinyle substitué.

31. Composé selon la revendication 29 dans lequel Ar est un radical phényle ou un radical pyridinyle.

32. Composé selon la revendication 29 dans lequel Y est -O- ou -S-.

33. Composé selon la revendication 29 dans lequel Z est un radical Cl, F, CF₃, NO₂ ou CN.

34. Composé selon la revendication 29 dans lequel B est un éther diphényle, un thioéther diphényle, un amino diphényle, un éther phénylpyridinyle, un méthylphényle pyridinyle ou un thioéther phénylpyridinyle, éventuellement substitués.

35. Composé selon la revendication 1 dans lequel B est un éther diphényle, un thioéther diphényle, un amino diphényle, un éther phénylpyridinyle, un méthylphényle pyridinyle, un thioéther phénylpyridinyle, un éther phénylpyrimidinyle, un thioéther phénylquinoline, un éther phénylnaphtyle, un éther pyridinylnaphtyle et un thioéther pyridinylnaphtyle, éventuellement substitués.

36. Composé de formule 1 ou d'un sel pharmaceutiquement acceptable de celui-ci 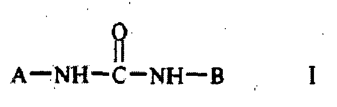 dans laquelle
B est un éther diphényle, un thioéther diphényle, un éther phénylpyridinyle, un méthylphényle pyridinyle, un thioéther phénylquinoline, un éther phénylnaphtyle, un éther pyridinylnaphtyle et un thioéther pyridinylnaphtyle, éventuellement substitués; et
A est un radical 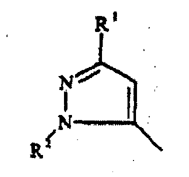 où
R¹ est un radical alkyle en C₃-C₁₀, un cycloalkyle en C₃-C₁₀, un alkyle en C₁-C₁₀ halogéné jusqu'à la perhalogénation, ou un cycloalkyle en C₃-C₁₀ halogéné jusqu'à la perhalogénation ; et
R² est un radical phényle non substitué, un radical pyridinyle non substitué, un radical phényle substitué ou un radical pyridinyle substitué ; lorsque R² est un groupe substitué, il est substitué par un ou plusieurs substituants choisis indépendamment dans le groupe constitué par les halogènes, jusqu'à la perhalogénation, et Vₙ,
où n = 0 à 3 et chaque V est choisi indépendamment dans le groupe constitué par les radicaux -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, hétéroaryle en C₃-C₁₃, alkaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkaryle en C₇-C₂₄ substitué et alkylhétéroaryle en C₄-C₂₄ substitué,
lorsque V est un groupe substitué, il est substitué par un ou plusieurs substituants indépendamment choisis dans le groupe constitué par les halogènes jusqu'à la perhalogénation, -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'} et -NO₂ ;
où R⁵ et R^{5'} ont chacun indépendamment la définition donnée ci-dessus.

37. Composé selon la revendication 1, dans lequel R² est un radical phényle ou pyridinyle, éventuellement substitué par -NO₂, -OH, -NH₂, -OCH₃, alkyle en C₁-C₆, alkyle en C₁-C₆ halogéné jusqu'à la perhalogénation,-NHC(O)-(alkyle en C₁-C₆),-SO₂₋(alkyle en C₁-C₆), ou-O-(alkyle en C₁-C₆) éventuellement substitué une ou deux fois par -F, -Cl ou par les deux.

38. Composé selon la revendication 1 choisi parmi :
*N*-(1-phényl-3-*tert*-butyl-5-pyrazolyl)-*N'*-(2,3-dichlorophényl) urée ;
*N*-(1-(4-aminophényl-3-*tert*-butyl-5-pyrazolyl)-*N'*-(2,3-dichlorophényl) urée ;
*N*-(1-(2-méthylphényl-3-*tert*-butyl-5-pyrazolyl)-*N'*-(2,3-dichlorophényl) urée ;
*N*-(1-(2-pyridinyl-3-*tert*-butyl-5-pyrazolyl)-*N'*-(2,3-dichlorophényl) urée ;
*N-*(1-(5-trifluorométhyl-2-pyrimidinyl)-3-*tert*-butyl-5-pyrazolyl)-*N'*-(2,3-dichlorophényl) urée;
*N*-(1-(3-fluorophényl)-3-*tert*-butyl-5-pyrazolyl)-*N'*-(2,3-dichlorophényl) urée ;
*N*-(1-(4-chlorophényl)-3-*tert*-butyl-5-pyrazolyl)-*N'*-(2,3-dichlorophényl) urée ;
*N*-(1-(4-méthariesulfoxyphényl)-3-*tert*-butyl-5-pyrazolyl)-*N'*-(2,3-dichlorophényl) urée ;
*N*-(1-(4-nitrophényl)-3-*tert*-butyl-5-pyrazolyl)-*N'*-(2,3-dichlorophényl) urée ;
*N*--(1-(3-méthoxyphényl)-3-*tert*-butyl-5-pyrazolyl)-*N'*-(2,3-dichlorophényl) urée ;
*N*--(1-(3-trifluorophényl)-3-*tert*-butyl-5-pyrazolyl)-*N'*-(2,3-dichlorophényl) urée ;
*N*--(1-(4-méthoxyphényl)-3-*tert*-butyl-5-pyrazolyl)-*N'*-(2,3-dichlorophényl) urée ;
*N*--(1-(4-isopropylphényl)-3-*tert*-butyl-5-pyrazolyl)-*N'*-(2,3-dichlorophényl) urée ;
*N*--(1-(3-aminophényl)-3-*tert*-butyl-5-pyrazolyl)-*N'*-(2,3-dichlorophényl) urée ;
*N*--(1-(3-nitrophényl)-3-*tert*-butyl-5-pyrazolyl)-*N'*-(2,3-dichlorophényl) urée ;
*N*-(1-phényl)-3-*tert*-butyl-5-pyrazolyl)-*N'*-(3-trifluoro, 4-chlorophényl) urée ;
*N*-(1-phényl)-3-*tert*-butyl-5-pyrazolyl)-*N'*-(4-trifluorophényl) urée ;
*N*-(1-phényl)-3-*tert*-butyl-5-pyrazolyl)-*N'*-(2,4-difluorophényl) urée ;
*N*-(1-phényl)-3-*tert*-butyl-5-pyrazolyl)-*N'*-(3-fluorophényl) urée ;
*N*-(1-phényl)-3-*tert*-butyl-5-pyrazolyl)-*N'*-(3-cyanophényl) urée ;
*N*-(1-phényl)-3-*tert*-butyl-5-pyrazolyl)-*N'*-(4-nitrophényl) urée ;
*N*-(1-(3-acétamidophényl)-3-*tert*-butyl-5-pyrazolyl)-*N'*-(4-phénoxyphényl) urée;
*N*-(1-(3-aminophényl)-3-*tert*-butyl-5-pyrazolyl)-*N'*-(4-phénoxyphényl) urée ;
*N*-(1-(3-nitrophényl)-3-*tert*-butyl-5-pyrazolyl)-*N'*-(4-phénoxyphényl) urée ;
*N*-(1-phényl)-3-*tert*-butyl-5-pyrazolyl)-*N'*-(4-(4-pyridinylméthyl)phényl) urée ;
*N*-(1-(4-pyridinyl)-3-*tert*-butyl-5-pyrazolyl)-*N'*-(4-(4-pyridinylméthyl)phényl) urée ;
*N*-(1-(2,5-dichlorophényl)-3-*tert-*butyl-5-pyrazolyl)-*N'*-(4-(4-pyridinylméthyl)phényl) urée ;
*N*-(1-(4-fluorophényl)-3-*tert*-butyl-5-pyrazolyl)-*N'*-(4-(4-pyridinylméthyl)phényl) urée ;
*N*-(1-(2-méthylphényl)-3-*tert*-butyl-5-pyrazolyl)-*N'*-(4-(4-pyridinylméthyl)phényl) urée ;
*N-*(1-(3-fluorophényl)-3-*tert*-butyl-5-pyrazolyl)-*N'*-(4-(4-pyridinylméthyl)phényl)urée;
*N-*(1-(4-méthanesulfoxyphényl)-3-*tert*-butyl-5-pynizolyl)-*N'*-(4-(4-pyridinylméthyl) phényl) urée ;
*N*-(1-(4-nitrophényl)-3-*tert*-butyl-5-pyrazolyl)-*N'*-(4-(4-pyridinylméthyl)phényl) urée ;
*N*-(1-(3-méthoxyphényl)-3-*tert*-butyl-5-pyrazolyl)-*N'*-(4-(4-pyridinylméthyl)phényl) urée;
*N*-(1-(3-aminophényl)-3-*tert*-butyl-5-pyrazolyl)-*N'*-(4-(4-pyridinylméthyl)phényl) urée ;
*N*-(1-(3-nitrophényl)-3-*tert*-butyl-5-pyrazolyl)-*N'*-(4-(4-pyridinylméthyl)phényl) urée ;
*N*-(1-(3-aminophényl)-3-*tert*-butyl-5-pyrazolyl)-*N'*-(4-(4-pyridinylthio)phényl) urée ;
*N*-(1-(3-nitrophényl)-3-*tert*-butyl-5-pyrazolyl)-*N'*-(4-(4-pyridinylthio)phényl) urée ;
*N*--(1-(3-(2,3-dichlorophénoxyacylamino)phénylméthyl)-3-*tert*-butyl-5-pyrazolyl)-*N'-*(2,3-dichlorophényl) urée ;
*N*--(1-(3-hydroxyphénylméthyl)-3-*tert*-butyl-5-pyrazolyl)-*N'*-(2,3- dichlorophényl) urée ;
*N-*(2-phényl-5-*tert*-butyl-3-thyényl)-*N'*-(2,3-dichlorophényl) urée ;
et
*N*-(2-(4-pyridinyl)-5-*tert*-butyl-3-furyl)-*N'*-(2,3- dichlorophényl) urée ;

39. Composé selon la revendication 1, dans lequel, R¹ est un radical t-butyle et R² est un radical phényle non substitué ou substitué.

40. Composé selon la revendication 3, dans lequel Q est un radical phényle, Q¹ est un radical phényle ou pyrinidile, Y est -O-, -S- ou -CH₂-, et Z est un radical Cl, F, NO₂ ou CF₃.
